# EUROPÄISCHE PATENTANMELDUNG

(11) **EP 4 560 294 A2**
(43) Veröffentlichungstag der Anmeldung: **28.05.2025**
(21) Anmeldenummer: 25168590.5
(22) Anmeldetag: 31.08.2018
(51) Int. Cl.: G01N 21/05

(54) **BEHÄLTER MIT WANDUNGSVORSPRUNG UND SENSORBEREICH**

(30) Priorität: 16.11.2017 DE 102017010629
(62) Teilanmeldung aus: 18773093.2
(71) Anmelder: Sartorius Stedim Biotech GmbH, 37079 Göttingen (DE)
(72) Erfinder: HOEHSE, Marek, 37073 Göttingen (DE); REGEN, Thomas, 37079 Göttingen (DE)
(74) Vertreter: Müller-Boré & Partner Patentanwälte PartG mbB

(57) **Zusammenfassung**

Wandungsvorsprung-Element (20') zur Befestigung an einer Behälterwandung (4) eines Behälters (1), das Wandungsvorsprung-Element (20') umfassend eine sich im befestigten Zustand zur Außenseite (A) des Behälters (1) erstreckende Wandungsausbuchtung (20a) und einen Wandungsvorsprung (20), wobei der Wandungsvorsprung (20) für die Anbringung mindestens eines Sensors (21) von der Außenseite (A) des Behälters zur Erfassung mindestens einer Größe von einem in einem Behälter-Innenraum (22) enthaltenen Medium (8) ausgelegt ist; dazu ausgelegt ist, den Behälter-Innenraum (22) zumindest teilweise zu umgeben; mindestens einen Sensorbereich (23) umfasst, welcher dazu ausgelegt ist, dass die Größe durch den Sensorbereich (23) mittels des Sensors (21) erfasst werden kann; und einen Kanal (K) bzw. ein kanalartiges Volumen (K) umfasst, welcher zumindest teilweise durch ein Leitabschnitt (34) und/oder eine Kanalführung (35) umgeben wird und der Kanal (K) dazu ausgelegt ist, ein sich bewegendes Medium von einem Kanaleingang (KE) zu einem Kanalausgang (KA) in einer Stromrichtung (37) zu führen.

## Beschreibung

Die Erfindung betrifft einen Behälter umfassend einen Wandungsvorsprung, sowie ein Wandungsvorsprung-Element umfassend einen Wandungsvorsprung für eine Anbringung, insbesondere mittels einer Sensor-Anbringungsvorrichtung, eines Sensors oder mehrerer Sensoren, bevorzugt eines optischen Sensors als ein Mittel für die Durchführung einer optischen Methode. Mittels eines oder mehrerer Sensoren kann ein oder können mehrere Größen bzw. Parameter, bevorzugt eine optische Größe erfasst werden. Mit Hilfe einer stichprobenartigen oder dauerhaften Erfassung bzw. Ermittlung von Größen kann eine Überwachung, insbesondere eine dauerhafte Überwachung über einen biologischen und/oder chemischen und/oder biochemischen Prozess in einem Medium oder mehreren Medien erfolgen.

Ferner betrifft die Erfindung eine an einem Behälter anbringbare und von einem Behälter abnehmbare Sensor-Anbringungsvorrichtung als Mittel zur Anbringung bzw. Fixierung bzw. Lagerung bzw. Befestigung eines Sensors, mehrerer Sensoren, eines Sensorelementes oder einer Lichtquelle, insbesondere eines Lichtleiters relativ zu dem Behälter und dem Wandungsabschnitt. Eine Lichtquelle kann einen Laser, eine Diode, einen Globar, eine Nernstlampe, eine Bogenlampe, eine Glühlampe, einen Leuchtstoff, eine Leuchtdiode (LED) und/oder ein anderes Licht emittierendes Mittel umfassen.

Die Erfindung kann insbesondere in einem oder mehreren in den folgenden Bereichen Verwendung finden: Biotechnologie, Lebensmitteltechnologie, Getränketechnologie, chemische Industrie, chemische Forschung, Laborbedarf, Medizintechnik, Prozesschemie, technische Chemie. Folgende ein oder mehrere Prozesse können unter anderem innerhalb eines Behälters stattfinden: chemische, biologische und/oder biochemische Prozesse, insbesondere Fermentation, Vergärung, Destillation, Aufreinigung, Zersetzung, aerobe Prozesse, anaerobe Prozesse.

Bisher werden chemische, biologische und/oder biochemische Prozesse, die beispielsweise innerhalb eines Behälters eines Bioreaktors oder eines Fermenters oder eines Lebensmittebehälters stattfinden, beispielsweise derart überwacht, dass eine Größeund/oder ein Prozess-Stadium basierend auf der regelmäßigen Entnahme einer Probe ermittelt wird. Die Analyse soll dabei möglichst repräsentativ für einen Inhalt eines Behälters sein. Typische Größen, die zur konventionellen Überwachung eines Prozesses ermittelt werden, umfassen beispielsweise die Menge an Trockensubstanz bzw. die organische Trockensubstanz, den pH-Wert, die Konzentration flüchtiger Fettsäuren bzw. das Verhältnis der Konzentration an flüchtigen Fettsäuren zur Pufferkapazität, sowie die Konzentration an Spurenelementen, Ammoniak, Säuren, Basen, Proteinen, Lipiden oder anderer Stoffe.

Die Trockensubstanz bzw. die organische Trockensubstanz stellt beispielsweise eine indirekte Messung anderer Messgrößen bzw. Parameter, wie z.B. eine Stickstoff-, Protein-, und/oder Spurenelement-Konzentration dar. Das Verhältnis aus der Konzentration an flüchtigen Fettsäuren zu der Pufferkapazität gibt Auskunft über das Stadium eines Prozesses beispielsweise während einer Fermentation. Die Konzentration flüchtiger Fettsäuren, wie beispielsweise der Essigsäure oder der Propionsäure gibt ebenso Auskunft über das Stadium eines Prozesses, denn flüchtige Fettsäuren sind oft Zwischenprodukte in Prozessen, wie beispielsweise Biogasprozessen. Bei zu hohen Konzentrationen können flüchtige Fettsäuren beispielsweise hemmend auf die Prozessbiologie wirken. Für die Bestimmung der Anwesenheit und Konzentration von Fettsäuren werden in der Regel unterschiedliche Methoden verwendet. Einzelne Fettsäuren können beispielsweise chromatographisch bestimmt werden. Die Konzentration an Proteinen, Lipiden und/oder anderen Stoffen, wie Ammoniak beispielsweise kann ebenfalls als Indikator für das Stadium eines Prozesses dienen und beispielsweise chromatographisch bestimmt werden.

Die Bestimmung von Größen erfolgt typischerweise per geeigneter Analyse-Methode außerhalb des Behälters, beispielsweise per Auswiegen, chromatographisch, und/oder elektrochemisch. Dazu werden typischerweise Proben aus dem Behälter entnommen und in einem Analyselabor untersucht. Oft werden solche Proben sogar per Post an ein Analyselabor versandt insofern keine Analyse vor Ort vorgenommen werden kann.

Der vorliegenden Erfindung liegt die Aufgabe zugrunde, einen verbesserten Behälter zur Überwachung von Größen des Inhalts bzw. des beinhalteten Mediums bereitzustellen. Diese Aufgabe wird durch die unabhängigen Ansprüche gelöst. Die Gegenstände der abhängigen Ansprüche stellen bevorzugte Ausführungsformen dar.

Die Erfindung betrifft einen Behälter mit mindestens einem Wandungsvorsprung für die Anbringung bzw. Aufnahme bzw. Lagerung bzw. Fixierung mindestens eines Sensors, insbesondere eines optischen Sensors bzw. Detektors von einer Außenseite des Behälters zur Messung bzw. zur Erfassung mindestens einer Messgröße bzw. eines zu messenden bzw. zu detektierenden Parameters, insbesondere einer physikalischen und/oder chemischen und/oder biologischen Größe von einem oder mehreren in einem Behälter-Innenraum enthaltenen Medium bzw. Medien, insbesondere von einem biologischen Medium, wobei der Wandungsvorsprung an einer Behälterwandung des Behälters angeordnet ist und dazu ausgelegt ist, den Behälter-Innenraum und das Medium, insbesondere ein Probenvolumen, vorzugsweise gefüllt mit einem Teil des Mediums, zumindest teilweise zu umgeben und wobei der Wandungsvorsprung mindestens einen Sensorbereich, insbesondere umfassend ein optisches Element, z.B. ein Fenster, ein Prisma eine Lochblende und/oder eine diffus reflektierende und/oder streuende Oberfläche und/oder einen Zugang, welches/welcher/welche dazu ausgelegt ist/sind, dass die Messgröße, beispielsweise die physikalische und/oder chemische und/oder biologische Größe durch den Sensorbereich mittels des Sensors, insbesondere ohne Entnahme eine Probe erfasst werden kann. Bevorzugt erfolgt die Erfassung der Größe derart, dass kein physikalischer bzw. physischer Kontakt bzw. berührungskontakt zwischen dem Sensor und dem Medium besteht. Alternativ kann jedoch auch ein Berührungskontakt zwischen Sensor und Medium bestehen.

Der Begriff einer "Messgröße" bzw. "Größe" hat im Wesentlichen dieselbe Bedeutung, wie der Begriff eines "Parameters", insbesondere eines "zu messenden Parameters". Eine Größe bzw. Messgröße kann eine physikalische und/oder eine chemische und/oder eine biologische Größe umfassen.

Unter physikalischen Größen des Mediums, welche erfassbar bzw. messbar bzw. detektierbar sind, werden im Folgenden insbesondere Konzentration(en) von ein oder mehreren Stoffen, Druck und/oder Partialdruck von Gasen (z.B. Sauerstoff, Kohlendioxid), Konzentration eines in einer Flüssigkeit gelösten Gases, Feuchtigkeit, Anzahl von Partikeln, Trübung, Temperatur, pH-Wert, elektromagnetische Strahlen, Fluoreszenz, elektrische Leitfähigkeit, kapazitive Widerstände und/oder elektrische Widerstände verstanden. Eine physikalische Messgröße kann auch eine Anzahl, Dichte und/oder Größe von ein oder mehreren biologischen Zellen sein bzw. betreffen.

Eine chemische und/oder biologische Messgröße kann beispielsweise die Menge und/oder Konzentration eines Nährstoffs, beispielsweise Glukose; oder eines Titers, beispielsweise Protein; oder eines Metabolits, beispielsweise Laktat, sein bzw. betreffen. Es wird jedoch keine scharfe Abgrenzung zwischen den Definitionen von physikalischen, chemischen und biologischen Größen vorgenommen, weshalb im Zweifel eine biologische Größe auch einer physikalischen und insbesondere einer chemischen Größe entsprechen kann. Besonders bevorzugt können Messgrößen solche Größen umfassen, welche per optischem und/oder elektrischem Verfahren ermittelt werden können.

Der Wandungsvorsprung ist dazu ausgelegt, einen Teil des Behälter-Innenraums des Behälters, insbesondere das Probenvolumen und bevorzugt ein spaltförmiges Probenvolumen teilweise zu umgeben. Das Probenvolumen ist entsprechend ein Teil des Behälter-Innenraums, welcher mit dem restlichen Behälter-Innenraum des Behälters in Kontakt steht. Das Probenvolumen befindet sich zwar auf der Innenseite des Behälters aber im Gegenteil zu dem restlichen Behälter-Innenraum ragt es mit dem Wandungsvorsprung in Richtung der Außenseite. In anderen Worten gibt es eine Verbindung zwischen dem Probenvolumen, welches zumindest teilweise von dem Wandungsvorsprung umgeben ist, und dem restlichen Behälter-Innenraum des Behälters, sodass ein Medium im Behälter-Innenraum des Behälters auch in das Probenvolumen einfließen kann. Insbesondere gibt es eine Fluidverbindung zwischen dem Probenvolumen und dem restlichen Behälter-Innenraum des Behälters. Besonders bevorzugt kann beispielsweise mittels Rühren bzw. Mischen verhindert werden, dass das Medium, wenn es einmal in das Probenvolumen gelangt ist, dort dauerhaft verbleibt bzw. steht. In anderen Worten kann das Medium im Probenvolumen mittels Mischen permanent oder zeitweise oder sporadisch ausgetauscht bzw. ausgewechselt werden durch Medium aus dem restlichen Behälter-Innenraum. Insbesondere wird ein Probenvolumen durch ein spaltartiges Volumen bzw. durch einen Spalt definiert.

Ein Sensor, der an bzw. relativ zu dem Wandungsvorsprung des Behälters angebracht werden kann, kann im Allgemeinen jegliche Sensoren bzw. Detektoren umfassen, welche dazu ausgelegt sind, Messgrößen, beispielsweise physikalische und/oder chemische und/oder biologische Größen eines Mediums innerhalb des Behälters zu erfassen. Der Sensor kann insbesondere ein optischer Sensor sein, wenn ein Sensorbereich einen optischen Zugang bzw. ein optisches Element, insbesondere ein Fenster, ein Prisma eine Lochblende und/oder eine diffus reflektierende und/oder streuende Oberfläche zu dem Behälter-Innenraum von außen darstellt oder umfasst, also der Sensorbereich im Wesentlichen transparent gegenüber Licht zumindest eines spektralen Bereichs bzw. zumindest teilweise lichtdurchlässig ist. Falls der Sensorbereich und/oder der Wandungsvorsprung einen Zugang in Form einer Öffnung bzw. eines Lochs umfasst, so kann ein Sensor auch einen pH-Sensor umfassen, der zur Erfassung eines pH-Wertes mit dem Medium im Behälter-Innenraum in Kontakt stehen muss. Ein solcher Sensor ist insbesondere eine pH-Elektrode. Der zumindest eine pH-Sensor kann im Allgemeinen dauerhaft oder nur sporadisch bzw. zeitweise an dem Wandungsvorsprung angebracht sein.

Insbesondere kann der Behälter dazu ausgelegt sein, ein geschlossenes System zumindest zeitweise zu bilden. Deshalb ist es bevorzugt, dass die Form der Anbringung des Sensors an dem Wandungsvorsprung dazu geeignet ist, einen für das Medium innerhalb des Behälters im Wesentlichen undurchlässigen bzw. impermeablen bzw. dichten Behälter zu ermöglichen. In anderen Worten ist die Kontaktstelle zwischen einer Behälterwandung und einem Wandungsvorsprung bzw. einem Wandungsvorsprung-Element sowie zwischen einem Sensor und einer möglichen Öffnung bevorzugt dicht, derart dass ein Stoffaustausch zwischen der Innen- und der Außenseite im Wesentlichen verhindert werden kann.

Größen zur Überwachung eines Prozesses, die mittels eines Sensors direkt oder indirekt erfasst bzw. aufgenommen werden können, umfassen optische Größen, insbesondere Extinktion, Stärke der Lichtstreuung, Raman-Streuung, Absorption, Fluoreszenz sowie Temperatur, Partikeldichte, pH-Wert, Konzentration flüchtiger Fettsäuren bzw. das Verhältnis der Konzentration an flüchtigen Fettsäuren zur Pufferkapazität, sowie die Konzentration an Spurenelementen, Ammoniak, Säuren, Basen, Proteinen, Lipiden oder an anderen Stoffen beispielsweise gelöste Gase.

Durch den Behälter kann eine Überwachung von Größen stichprobenweise bzw. sporadisch oder dauerhaft von außen erfolgen, ohne, dass die Notwendigkeit für eine Probenentnahme des Behälterinhalts aus dem Behälter besteht. Daher wird die Gefahr einer Kontamination von außen während der Entnahme einer Probe durch eine Entnahmevorrichtung, beispielsweise eine Pipette oder eine Spritze und/oder durch die Person, welche die Probe entnimmt, vermieden oder zumindest verringert.

Besonders vorteilhaft ist, dass in den meisten Fällen der Verwendung des Behälters im Wesentlichen darauf verzichtet werden kann, dass der Behälter für eine Entnahme einer Probe geöffnet wird, wie es für die konventionelle Prozessüberwachung in den meisten Fällen erforderlich ist. Dies mindert die Anfälligkeit für Störungen des Prozesses, da beispielsweise eine Temperatur und/oder ein Druck und/oder eine Gasatmosphäre und/oder ein sensibles und/oder instabiles Material und/oder ein Belichtungszustand durch Öffnen beeinträchtigt werden kann. Beispielsweise kann ein Prozess, der anaerobe Bedingungen erfordert, durch den Kontakt mit Sauerstoff von außen gestört werden. Auch kann ein lichtempfindlicher Prozess durch das Öffnen des Behälters und das damit einfallende Licht gestört werden. Dieser Umstand kann eine sporadische Überwachung eines Prozesses erschweren und eine permanente Überwachung von außen gar unmöglich machen.

Insbesondere erlaubt der Behälter bzw. der Wandungsvorsprung des Behälters und insbesondere eine Sensor-Anbringungsvorrichtung, dass eine exakte Ausrichtung von elektromagnetischer Strahlung bzw. Licht, beispielsweise von Anregungsstrahlung bzw. Probestrahlung, durch ein Medium, sowie eine exakte Ausrichtung eines Detektionskanals gewährleistet sein kann. Dies führt dazu, dass auf zuverlässige Weise reproduzierbare und vergleichbare Ergebnisse erzielt werden können.

Darüber hinaus erlaubt der Behälter, dass eine Überwachung eines Probenvolumens innerhalb des Behälters von außen besonders repräsentativ sein kann. Durch die Vermeidung einer Probenentnahme kann beispielsweise ein Austrocknen, eine Oxidation, ein Denaturieren und/oder ein Degradieren des Probenmaterials vermindert oder gar verhindert werden. Es kann ferner darauf verzichtet werden, eine Probe in ein Analysenlabor zu transportieren oder zu versenden, was damit verbunden sein kann, dass Zeit zwischen der Entnahme und der Analyse gespart werden kann, was insbesondere bei einer augenblicklichen Kontrolle und/oder Regelung und/oder Steuerung des Prozesses vorteilig wäre. Dies ist insbesondere dann vorteilig, wenn der Prozess innerhalb der entnommen Probe durch die Entnahme gehemmt oder gar unterdrückt wäre und/oder die Probe sich gegenüber des Mediums innerhalb des Behälters verändern würde. Um eine Größe "einzufrieren", sodass diese sich in dem Probenmaterial gegenüber dem verbleibenden Medium im Behälter zur Entnahmezeit nicht ändert, wird eine entnommene Probe für die konventionelle Prozessüberwachung oft eingefroren. Dies hat regelmäßig zur Folge, dass sensitive Bestandteile der Probe denaturieren und/oder degradieren. Durch den Behälter kann insbesondere auf das Einfrieren einer Probe und dessen Folgen verzichtet werden.

Dadurch, dass die Entnahme von Probenmaterial bzw. -medium vermieden werden kann, besteht zudem keine Verwechslungsgefahr unter entnommenen Proben bzw. Probenbehältern, die zu unterschiedlichen Zeitpunkten und/oder von unterschiedlichen Behältern entnommen werden.

Die genannten Effekte können besonders vorteilhaft sein, falls sich ein besonders teures und/oder seltenes Edukt und/oder Produkt in dem Behälter befindet. Eine Kontamination und/oder Störung eines Prozesses und/oder fehlerhafte Steuerung und/oder fehlerhafte Regelung kann sich fatal auf die Qualität des Inhalts, insbesondere des Mediums in dem Behälter auswirken. Ferner besteht die Möglichkeit bei einer bevorzugt dauerhaften oder zumindest zeitweisen Überwachung, dass ein Prozess gesteuert und/oder geregelt werden kann. In anderen Worten kann bei der Beobachtung unvorteilhafter Prozess-Vorgänge oder Parameterveränderungen im Wesentlichen sofort bzw. zeitnah eingelenkt werden beispielsweise durch Anpassen von Größen, wodurch sich beispielsweise verhindern lassen kann, dass wertvolle Produkte und/oder Edukte degradieren und/oder denaturieren.

Basierend auf einer Ermittlung von Größen können Prozesse also nicht nur überwacht, sondern auch gesteuert und/oder geregelt werden, indem Stoffe hinzugegeben und/oder entnommen werden und/oder Änderungen von Größen bzw. Parametern, wie beispielsweise der Temperatur und/oder dem Druck vorgenommen werden.

Gemäß einem Aspekt erstreckt sich der Wandungsvorsprung entlang einer Längsachse des Wandungsvorsprungs, welche einen Winkel α von etwa 30° bis etwa 150°, insbesondere von etwa 45° bis etwa 135° mit einer Längsachse des Behälters und/oder einer Konturlinie der Behälterwandung des Behälters einschließt. Besonders bevorzugt schließt die Längsachse mit einer Normalen einer imaginären Konturlinie zur Definition des Probenvolumens einen Winkel β von etwa -45° bis etwa 45° ein. Insbesondere schließt die Längsachse mit einer Normalen einer Längsachse des Behälters einen Winkel β von etwa -45° bis etwa 45° ein. Insbesondere schließt eine Breitenachse des Wandungsvorsprungs mit einer Breitenachse des Behälters einen Winkel γ von etwa -45° bis etwa 45° ein.

In anderen Worten kann die Längsachse des Wandungsvorsprungs gegenüber der Längsachse eines Behälters "nach oben" oder "nach unten" geneigt sein. Diese Neigung kann einen Winkel von etwa -45° bis etwa 45° betragen, wobei der besagte Winkel β zwischen Längsachse des Wandungsvorsprungs und einer Normalen bzw. Senkrechten auf der Längsachse des Behälters liegt. In dem Fall entspricht die Normale bzw. Senkrechte einer imaginären Konturlinie der Normalen bzw. Senkrechten auf der Längsachse des Behälters. Der Winkel β gleicht hier dem Wert, der sich aus 90°- α ergibt. Der Winkel β kann sich auch aus einer Auswölbung der Behälterwandung bzw. Bag-Folie ergeben. Dies kann insbesondere der Fall sein, wenn ein gefüllter single-use Beutel eine bauchige Form aufweist, also in einem unteren Bereich einen größeren Querschnitt hat als in einem oberen Bereich. Dann kann das Medium die Behälterwandung so verformen, dass der Wandungsvorsprung "nach oben" geneigt ist bzw. gedrückt wird. Der Winkel β zwischen der Längsachse des Wandungsvorsprungs und einer Normalen bzw. Senkrechten auf der Längsachse des Behälters wäre dann ungleich 0°, insbesondere größer als 0° und kleiner als 45°, wenn man davon ausgeht, dass sich positive Winkel für eine Neigung des Wandungsvorsprungs "nach oben" und negative Winkel für eine Neigung des Wandungsvorsprungs "nach unten" ergeben.

In anderen Worten kann der Wandungsvorsprung auch gegenüber der Breitenachse eines Behälters geneigt sein. Insbesondere kann eine Breitenachse des Behälters mit einer Breitenachse des Wandungsvorsprungs bzw. des Spalts einen Winkel von etwa -45° bis etwa 45° einschließen.

Ein Winkel γ von etwa -45° bis etwa 45° begünstigt den Materialfluss des Mediums in bzw. durch das Probenvolumen des Wandungsvorsprungs. Eine Neigung des Wandungsvorsprungs gegenüber der Längsachse des Behälters kann hingegen das Anbringen einer Sensorvorrichtung begünstigen. Der Winkel γ von etwa -45° bis etwa 45° erlaubt, dass die Anströmung des Ports optimiert werden kann.

Der Vorteil einer geneigten Anordnung des Wandungsvorsprungs liegt darin, dass eine Anbringungsvorrichtung zur Anbringung optischer Elemente besonders einfach anbringbar und/oder anordenbar ist.

Gemäß einem Aspekt ist der Behälter oder sind zumindest Elemente des Behälters sterilisierbar. Dies hat den Vorteil, dass ein Medium, welches in den Behälter gefüllt werden soll nicht kontaminiert wird, beispielsweise durch unerwünschte oder schädliche Bioorganismen.

Gemäß einem Aspekt umfasst der Behälter ein Rührelement, welches dazu ausgelegt ist, das Medium bzw. Material im Innern des Behälters bzw. auf der Behälter-Innenseite des Behälters im Wesentlichen zu durchmischen.

Eine optionale Durchmischung bzw. ein Verrühren des Behälterinhalts kann vorteilig sein für ein besonders repräsentatives von dem Wandungsvorsprung mindestens teilweise umgebenen Mediums innerhalb eines Probenvolumens. Das Probenvolumen ist dabei das Volumen, welches zumindest teilweise und insbesondere im Wesentlichen durch die Behälter-Innenseitenfläche des Wandungsvorsprungs, also durch die Oberfläche des Wandungsvorsprungs auf der Behälter-Innenseite des Behälters umgeben wird. Das Probenvolumen wird bevorzugt durch einen Spalt definiert. Die Durchmischung kann beispielsweise erlauben, dass ein Prozess innerhalb eines Behälters möglichst homogen abläuft und/oder, dass ein Teil des Mediums beispielsweise vom Boden und/oder aus einem zentralen Abschnitt des Behälters in den Bereich des Probenvolumens gelangt. Bevorzugt ist es möglich, zu vermeiden, dass ein Medium innerhalb des Probenvolumens, insbesondere innerhalb des Spalts steht bzw. im Wesentlichen nicht im Austausch steht mit dem restlichen Medium steht.

Gemäß einem Aspekt ist der Sensorbereich bzw. der Wandungsvorsprung bzw. ein optisches Element, insbesondere ein Fenster dazu ausgelegt, dass die Größe, beispielsweise die physikalische und/oder chemische und/oder biologische Größe mittels einer optischen Methode, insbesondere einer optischen Spektroskopie erfasst werden kann.

Ein optisches Verfahren bzw. eine optische Methode ist eine analytische Methode und kann unter Zuhilfenahme eines optischen Sensors ausgeführt bzw. angewandt werden. Eine optische Methode kann bevorzugt eine optische Spektroskopie umfassen. Insbesondere kann eine optische Methode beispielsweise umfassen: eine optische Bildgebung, eine Mikroskopie, eine Konfokal-Mikroskopie, Raman-Spektroskopie, Infrarot-Spektroskopie, Lichtstreuung, UV/Vis-Spektroskopie, Laserspektroskopie, Fluoreszenzspektroskopie, Terahertz-Spektroskopie, Ellipsometrie, Refraktometrie, Oberflächenplasmonen-Resonanz-Spektroskopie, und/oder allgemein eine Molekülspektroskopie. Es kann auch mittels einer optischen Methode beispielsweise eine Sauerstoffkonzentration oder eine Konzentration anderer (in einer Flüssigkeit gelöster) Gase ermittelt werden. Alternativ oder zusätzlich können auch andere optische Methoden zur Ermittlung von Größen in Betracht kommen.

Eine optische Spektroskopie, beispielsweise eine Molekülspektroskopie, wie die Infrarotspektroskopie kann quantitativ und im Wesentlichen nichtinvasiv einen direkten Aufschluss über eine Wesentliche molekulare Zusammensetzung des Mediums geben oder zumindest einen Hinweis auf die Anwesenheit von Stoffen geben. Eine infrarote Strahlung, also eine elektromagnetische Strahlung in einem spektralen Bereich, welcher zumindest teilweise ein infrarotes Spektrum umfasst, die beispielsweise in oder durch ein Medium eines Probenvolumens gesendet wird, kann darin enthaltenen Moleküle zur Schwingung anregen, sodass bestimmte Wellenlängen von der Probe je nach Zusammensetzung des Mediums zumindest teilweise absorbiert werden. Aufgrund einzelner absorbierter Wellenlängen bzw. einem Muster absorbierter Wellenlängen bzw. Wellenlängenbereiche umfassend mehrere absorbierte Wellenlängen können molekulare Verbindungen qualitativ und insbesondere quantitativ identifiziert werden, ohne dass die betroffenen Moleküle dabei zerstört werden. Derart können Konzentrationen beispielsweise molekularer Bestandteile eines Mediums, direkt bestimmt werden. Es kann deshalb auf indirekte und insbesondere auf fehlerbehaftete Methoden verzichtet werden. Eine optische Methode kann derart beispielsweise im Wesentlichen ein Verfahren ersetzen, in welchem eine Trockensubstanz ausgewogen werden muss, um eine Konzentration eines Stoffes zu ermitteln.

Ferner ist eine optische Methode, insbesondere eine im Wesentlichen nichtinvasive optische Spektroskopie zur permanenten bzw. zur dauerhaften Überwachung von Größen geeignet, da der Zustand des Mediums bzw. des Prozess-Stadiums, in dem sich das Medium befindet, im Wesentlichen nicht durch die Überwachung von außen beeinträchtigt wird. Darüber hinaus kann in der Regel eine Änderung der Größe bzw. des Prozesses bzw. ein Vorgang augenblicklich verfolgt bzw. überwacht werden. Derart kann sofort - als Folge einer beobachteten Veränderung - eine Maßnahme ergriffen werden, um den Prozess zu steuern und/oder zu regeln. Beispielsweise kann eine zunehmende Streuung von Licht darauf hinweisen, dass ein Stoff ungewollt in eine Feststoff-Phase ausfällt und Partikel bildet, woraufhin als Gegenmaßnahme sofort ein Stoff, der eine Partikelbildung verhindern kann, zugesetzt werden kann.

Gemäß einem Aspekt umfasst der Wandungsvorsprung zwei im Wesentlichen zueinander parallele und durch eine Probenschichtdicke voneinander beabstandete Vorsprungs-Wände einer Vorsprungs-Länge und ist die Vorsprungs-Länge mindestens etwa doppelt so groß ist wie die Probenschichtdicke, derart, dass der Wandungsvorsprung im Wesentlichen ein spaltförmiges Volumen umgibt bzw. der Wandungsvorsprung spaltförmig bzw. in Form eines Spaltes ausgebildet ist, wobei mindestens eine der Vorsprungs-Wände bevorzugt jeweils den Sensorbereich und insbesondere ein optisches Element, bevorzugt ein Fenster umfasst. In anderen Worten kann eine Vorsprungs-Wand eines Wandungsvorsprungs oder es können mehrere Vorsprungs-Wände jeweils einen Sensorbereich umfassen oder gar darstellen. Insbesondere kann eine Vorsprungs-Wand eines Wandungsvorsprungs oder es können mehrere Vorsprungs-Wände jeweils ein optisches Element, bevorzugt ein Fenster umfassen oder gar darstellen.

In anderen Worten ausgedrückt kann der Wandungsvorsprung ein spaltartiges Volumen zumindest teilweise umgeben. Zwei im Wesentlichen zueinander parallele und durch eine Probenschichtdicke voneinander beabstandete Vorsprungs-Wände können bei einem konstanten Abstand und insbesondere bei einer stabilen und im Wesentlichen rigiden Ausbildung gewährleisten, dass eine Vielzahl von Messungen unter denselben Bedingungen vorgenommen werden kann, da sich die Probenschichtdicke und damit das untersuchte Volumen im Wesentlichen nicht ändert. Dies ist insbesondere vorteilhaft für Kalibrations-Messungen, da für jede einzelne Messung dieselbe Probenschichtdicke vorliegt und somit Änderungen der Messergebnisse nicht auf Änderungen der Probenschichtdicke zurückgeführt werden müssen. Derart lassen sich besonders zuverlässige und präzise Kalibrations-Messungen vornehmen.

Durch die ausgeprägte Vorsprungs-Länge des Wandungsvorsprungs kann ferner das Anbringen bzw. Befestigen bzw. Fixieren bzw. Lagern von Lichtquellen, Sensoren und/oder einer Sensor-Anbringungsvorrichtung begünstigt werden, da derart ausreichend Raum zur Positionierung bzw. Anordnung der Sensoren an dem Wandungsvorsprung bereitgestellt werden kann. Die Vorsprungs-Wände eines Wandungsvorsprungs können jeweils einen Sensorbereich, insbesondere jeweils ein im Wesentlichen für einen Wellenlängenbereich transparentes optisches Element, insbesondere ein Fenster umfassen. Bevorzugt sind beide Sensorbereiche ebenfalls parallel zueinander ausgerichtet. Insbesondere sind die Sensorbereiche dazu ausgelegt, einen definierten und stabilen Strahlengang in bzw. durch ein Teil des Mediums bzw. des Probenvolumens bereitzustellen bzw. zu begünstigen. Es kann aber auch nur einen Sensorbereich von einem Wandungsvorsprung, insbesondere für eine reflektive Strahlengang-Anordnung umfasst sein. Alternativ können zwei sich im Wesentlichen gegenüberstehende Vorsprungs-Wände auch nicht parallel ausgebildet sein.

Gemäß einem Aspekt umfasst der Wandungsvorsprung eine diffus reflektierende bzw. streuende Fläche, beispielsweise eine weiße Oberfläche, und/oder ein reflektives Element, beispielsweise einen Spiegel. Der mindestens eine Sensorbereich umfasst das optische Element, insbesondere das Fenster und der Wandungsvorsprung ist dazu ausgelegt, dass die Messgröße, insbesondere die physikalische und/oder chemische und/oder biologische Größe mittels einer Sensorvorrichtung, welche vorzugsweise eine optische Faser umfasst, durch eine reflektive StrahlengangAnordnung, beispielsweise eine reflektive Optik-Anordnung, erfasst werden kann. In anderen Worten umfasst der Wandungsvorsprung ein reflektives Element und/oder eine reflektive Optik-Anordnung. Beispielsweise kann ein reflektives Element ein Spiegel sein, an welchem Licht im Wesentlichen in Richtung des optischen Elements, insbesondere des Fensters und des Sensors zurück reflektiert wird. Derart kann beispielsweise eine optische Faser bzw. ein Lichtleiter bzw. eine Lichtquelle ein Licht durch den Sensorbereich, insbesondere das optische Element, bevorzugt das Fenster in einen Behälter-Innenraum und eine reflektive Strahlengang-Anordnung einkoppeln bzw. aussenden, welches dann durch ein reflektives und/oder ein streuendes Element zumindest in Teilen zurück reflektiert und/oder gestreut wird und durch den Sensorbereich aus dem Behälter-Innenraum heraustritt bzw. herauspropagiert und durch denselben Lichtleiter oder einen anderen Lichtleiter aufgefangen bzw. erfasst wird.

Die Kombination aus Transmission durch das Medium und Reflektion an dem Spiegel und/oder einer diffus reflektierenden und/oder streuenden Fläche nennt man Transflexion. In anderen Worten wird ein Teil eines Lichtstrahls zum einen Teil an einer reflektiven und/oder streuenden Fläche reflektiert/gestreut, wobei ein anderer Teil des Lichtstrahls eine transmissive Fläche passiert. Die kann der Fall sein, wenn eine Fläche transmissive und reflektive und/oder streuende Eigenschaften hat. Ein Beispiel für eine solche Fläche ist ein Strahlteiler (engl.: "beam splitter"). Transflexion liegt auch vor, wenn ein Teil eines Strahls auf eine reflektive und/oder streuende Fläche trifft, während ein anderer Teil des Strahls auf eine transmissive Fläche trifft und diese passiert. Eine derartige Kombination einer Transmission und einer Reflektion entspricht einer "transflektiven Strahlenganganordnung".

Es ist auch der Fall umfasst, indem ein Wandungsvorsprung zwei optische Elemente, insbesondere zwei Fenster umfasst, wobei vor oder hinter einem der optischen Elemente eine diffus streuende Fläche und/oder ein Reflektor und/oder ein Spiegel angeordnet ist oder werden kann. In einem anderen Fall kann eines der beiden optischen Elemente, beispielsweise ein Fenster, durch eine diffus streuende Fläche und/oder ein Reflektor und/oder ein Spiegel ersetzt sein. Im Allgemeinen können optische Elemente gleichzeitig als Wände des Wandungsvorsprungs dienen, oder aber in eine Halterung vor oder hinter einem Sensorbereich eingesetzt sein und/oder werden.

Eine diffus reflektierende und/oder streuende Fläche zeichnet sich dadurch aus, dass sie Licht im Wesentlichen diffus reflektiert und/oder streut. Eine diffus reflektierende und/oder streuende Fläche kann beispielsweise eine weiße Oberfläche sein und ein diffus reflektierendes und/oder streuendes Material, beispielsweise eine Keramik und/oder eine Stahlplatte umfassen. Wenn ein Licht auf die diffus reflektierende und/oder streuende Fläche trifft, wird es diffus, also unter verschiedenen Winkeln (zurück)gestreut. Mindestens ein Teil des Lichtes wird unter einem solchen Winkel gestreut, dass es durch das optische Element insbesondere das Fenster wieder nach außen tritt, das optische Element also zweimal passiert.

Gemäß einem Aspekt umfasst der Wandungsvorsprung auf der Behälter-Innenseite einen Spiegel bzw. Reflektor, der dazu ausgelegt ist, dass die Messgröße, insbesondere die physikalische und/oder chemische und/oder biologische Größe mittels einer reflektiven Strahlengang-Anordnung erfasst wird.

Eine reflektive Strahlengang-Anordnung erfordert in der Regel lediglich die Bereitstellung eines Sensorbereichs, bevorzugt umfassend ein optisches Element, insbesondere ein Fenster oder ein optisches Element darstellend, und unter Umständen einen Spiegel bzw. einen Reflektor und/oder ein reflektierendes bzw. streuendes Element bzw. Medium auf der Behälter-Innenseite des Wandungsvorsprungs, welche das einfallende Licht entgegen der Einfallsrichtung zurückreflektieren bzw. zurückstreuen. Derart kann das Licht, welches zuvor eine Wegstrecke auf der Behälter-Innenseite des Wandungsvorsprungs durchlaufen hat, wieder durch den selben Sensorbereich austreten und von einem Sensor bzw. einem Lichtleiter eingefangen bzw. erfasst werden bzw. in einen Sensor eingekoppelt werden.

Beispielsweise kann ein Licht, insbesondere ein Laserlicht durch den Sensorbereich in den Teil des Behälter-Innenraums im Bereich des Wandungsvorsprungs gestrahlt werden, wo es beispielsweise an dem darin enhaltenen Medium und/oder an Partikeln, beispielsweise an mesoskopischen und/oder nanoskopischen Partikeln des Mediums gestreut wird. Die Streuung könnte beispielsweise eine Mie-, eine Raileigh-, eine Raman- oder eine andere Licht-Streuung umfassen. Dies kann insbesondere bei einer Raman-Streuung und/oder bei einer statischen oder dynamischen Lichtstreuung der Fall sein. Das gestreute Licht kann zumindest teilweise durch den einen Sensorbereich oder alternativ durch mehrere Sensorbereiche aus dem Behälter-Innenraum wieder hinausgelangen, wo es beispielsweise von einem Sensor erfasst wird. Ein derartiger Sensor, insbesondere ein optischer Sensor kann beispielsweise ein Spektrometer, insbesondere ein Raman-Spektrometer und/oder eine CCD Kamera und/oder einen Photovervielfacher bzw. Photomultiplier umfassen.

Alternativ oder zusätzlich kann das eingefallene Licht auch durch einen Spiegel oder ein im Wesentlichen reflektives optisches Element auf der entgegengesetzten Behälter-Innenseite des Sensorbereichs zurückgestreut bzw. reflektiert werden, sodass es durch einen oder mehrere Sensorbereiche aus dem Behälter-Innenraum heraustritt und außen von einem Sensor erfasst werden kann. In anderen Worten kann Licht im Wesentlichen aus einer ersten Richtung durch einen Sensorbereich insbesondere durch ein optisches Element, bevorzugt ein Fenster in einen Behälter-Innenraum bzw. in ein Probenvolumen eines Wandungsvorsprungs propagieren, zumindest teilweise auf der Behälter-Innenseite im Wesentlichen entgegen der ersten Richtung bzw. zurück gestreut oder gespiegelt werden, sodass zumindest ein Teil des Lichts aus dem Behälter-Innenraum wieder durch den Sensorbereich austritt, durch welches es eingetreten ist. Nach dem Austreten kann das Licht zumindest teilweise durch einen Lichtleiter erfasst bzw. eingefangen werden.

Die effektive Weglänge einer reflektiven Strahlengang-Anordnung, innerhalb der einfallendes Licht mit dem Medium beispielsweise den im Medium enthaltenen Molekülen wechselwirken kann, entspricht im Wesentlichen der doppelten Probenschichtdicke bzw. im Wesentlichen der zweimaligen Wegstrecke zwischen Sensorbereich und Spiegel bzw. zwischen Sensorbereich und streuendem Medium, wobei ein streuendes Medium beispielsweise ein im Medium enthaltenes Partikel bzw. Nanopartikel oder Tröpfchen einer Emulsion sein kann.

Es kann auch sein, dass lediglich ein Licht, welches aus dem Behälter-Innenraum durch einen Sensorbereich nach außen tritt durch einen Sensor erfasst wird. Beispielsweise kann eine Fluoreszenz erfasst werden. Das bedeutet, dass ein Medium oder Bestandteile eines Mediums im Behälter-Innenraum zumindest teilweise fluoreszieren und zumindest teilweise Licht emittieren, welches den Behälter-Innenraum durch einen Sensorbereich, beispielsweise ein Fenster verlassen. Ein Lichtleiter bzw. ein Sensor bzw. ein Element einer Sensorvorrichtung kann dann die Photonen bzw. das aus dem Behälter-Innenraum herausgetretene Licht einfangen und/oder erfassen und/oder detektieren und/oder aufzeichnen.

Gemäß einem Aspekt umfasst der Wandungsvorsprung einen Shutter bzw. eine Blende, welche dazu ausgelegt ist, zumindest zeitweise ein Eintreten von Licht im Wesentlichen in den Behälter-Innenraum durch einen Sensorbereich, insbesondere ein optisches Element, bevorzugt ein Fenster zu verringern bzw. zu verhindern. Der Shutter bzw. die Blende kann von außen oder aber auf der Behälter-Innenseite relativ zu dem Wandungsvorsprung angebracht sein. Bevorzugt ist der Shutter von außen betätigbar bzw. bedienbar, derart, dass ein Lichteinfall auf die Behälter-Innenseite kontrolliert, geregelt, gesteuert oder verändert werden kann.

Es kann vorteilhaft sein, dass ein Shutter bzw. eine Blende zumindest zeitweise das Eintreten und beispielsweise die Wechselwirkung des Mediums im Behälter-Innenraum mit Licht im Wesentlichen vermindert bzw. verhindert. Die Blende bzw. der Shutter kann im Wesentlichen vollständig den Einfall von Licht aller Wellenlängen blockieren. Beispielsweise kann ein Schritt bzw. eine Stufe eines Prozesses empfindlich gegenüber Licht, beispielsweise UV-Licht und/oder sichtbarem Licht sein, wohingegen eine andere Stufe im Prozess lichtunempfindlich sein kann. In dem Fall kann ein Shutter gegebenenfalls geöffnet oder geschlossen werden bei Bedarf. Die Blende bzw. der Shutter kann auch einen Filter umfassen, sodass lediglich ein spektraler Bereich von einfallendem Licht im Wesentlichen blockiert wird. Die genannten Möglichkeiten bestehen nicht nur für einfallendes Licht von außen, sondern auch für Licht, welches nach außen treten könnte und beispielsweise auf der Behälter-Innenseite erzeugt wird und/oder durch Teile des Behälter-Innenraums propagiert und oder von einem Medium im Behälter-Innenraum emittiert wird.

Je nachdem, ob ein Shutter den Sensorbereich, insbesondere das Fenster im Wesentlichen abschließt bzw. blockiert bzw. abdichtet gegen Einfall des gesamten Lichts oder Teile bzw. Frequenzbereiche des Lichts, oder ob sich ein Shutter zumindest teilweise in einer Offenposition befindet, welche dazu ausgelegt ist, dass zumindest teilweise Licht bzw. zumindest Frequenzbereiche des Lichts durch den Sensorbereich von innen bzw. von der Behälter-Innenseite nach außen und/oder von außen nach innen bzw. auf die Behälter-Innenseite propagieren, kann infolgedessen entschieden werden, ob ein Erfassen einer Messgröße, insbesondere einer physikalischen und/oder chemischen und/oder biologischen Größe von außen mittels eines Sensors durch den Sensorbereich stattfinden soll oder nicht. Beispielsweise kann eine Messung aufgezeichnet werden während sich ein Shutter in einer Offenposition. Befindet sich ein Shutter bzw. eine Blende im Wesentlichen in einer Position, die zumindest in Teilen verhindert, dass zumindest Teile eines Licht von außen nach innen und/oder von innen nach außen treten bzw. propagieren, so kann eine Messung bzw. Erfassung von Größen pausiert bzw. angehalten bzw. gestoppt werden.

Gemäß einem Aspekt umfasst der Wandungsvorsprung mindestens zwei Sensorbereiche, insbesondere zwei optische Elemente und bevorzugt zwei Fenster, besonders bevorzugt zwei zueinander parallel ausgerichtete bzw. angeordnete Fenster, wobei der Wandungsvorsprung dazu ausgelegt ist bzw. die zwei Sensorbereiche dazu ausgelegt sind, dass die Größe mittels einer Sensorvorrichtung, welche vorzugsweise eine optische Faser umfasst, durch eine transmissive Strahlengang-Anordnung erfasst werden kann. Alternativ können zwei optische Elemente, bevorzugt zwei Fenster auch im Wesentlichen nicht parallel zueinander ausgerichtet bzw. angeordnet sein. Es kann sein, dass die Sensorbereiche jeweils ein optisches Element, insbesondere ein Fenster umfassen oder darstellen.

Eine transmissive Strahlengang-Anordnung kann beispielsweise erlauben, dass Licht bzw. elektromagnetische Strahlung einer Lichtquelle, beispielsweise ein Laserstrahl von außen durch einen ersten Sensorbereich, insbesondere durch optisches Element, bevorzugt ein Fenster in den Behälter-Innenraum bzw. auf die Behälter-Innenseite des Wandungsvorsprungs gestrahlt bzw. gesendet wird. Das Licht kann zumindest teilweise das Medium des Probenvolumens bzw. der Probenvolumenschichtdicke passieren und mit dem Medium zumindest teilweise wechselwirken bevor es durch einen zweiten Sensorbereich, insbesondere ein optisches Element, bevorzugt ein Fenster gelangt und von einem Detektor erfasst wird. So kann beispielsweise eine Absorption mittels Infrarotspektroskopie und/oder UV/Vis-Spektroskopie ermittelt bzw. erfasst werden. Die Absorption kann wiederum ein Maß für die Konzentration eines Stoffes sein.

Die effektive Weglänge einer transmissiven Strahlengang-Anordnung, innerhalb der einfallendes Licht zumindest teilweise mit dem Medium beispielsweise mit den im Medium enthaltenen Molekülen wechselwirken kann, entspricht im Wesentlichen der Probenschichtdicke bzw. der Weglänge von dem ersten zu dem zweiten Sensorbereich.

Explizit ist es auch möglich, dass ein Wandungsvorsprung derart ausgestaltet ist, dass wahlweise eine transmissive Strahlengang-Anordnung eine transmissive Strahlengang-Anordnung verwendet werden kann, beispielsweise zur selben Zeit oder aber abwechselnd nacheinander.

Gemäß einem Aspekt umfasst ein Behälter eine Sensor-Anbringungsvorrichtung zur Anbringung des Sensors und/oder einer Lichtquelle relativ zu und/oder an dem Wandungsvorsprung.

Eine Sensor-Anbringungsvorrichtung kann beispielsweise in der Form eines Gestells und vorzugsweise rigide ausgebildet sein. Die Sensor-Anbringungsvorrichtung erlaubt, einen Sensor oder mehrere Sensoren derart relativ zu dem Wandungsvorsprung anzubringen und/oder zu befestigen und/oder zu lagern und/oder zu fixieren, dass der angebrachte Sensor eine Größe bzw. einen Parameter durch einen Sensorbereich insbesondere durch ein optisches Element, bevorzugt ein Fenster erfassen kann.

Die Sensor-Anbringungsvorrichtung kann insbesondere dazu dienen, einen oder mehrere Lichtleiter an dem Wandungsvorsprung anzubringen und/oder zu befestigen und/oder zu lagern und/oder zu fixieren. So kann beispielsweise, insbesondere bei einer transmissiven Strahlengang-Anordnung auch ein Lichtleiter mittels einer Sensor-Anbringungsvorrichtung angebracht werden, wobei der Lichtleiter eine Lichtquelle darstellt und Licht bzw. elektromagnetische Strahlung durch einen ersten Sensorbereich in den Behälter-Innenraum des Behälters insbesondere des Wandungsvorsprungs einstrahlt. Bei einer transmissiven Strahlengang-Anordnung kann mittels Sensor-Anbringungsvorrichtung ferner ein Sensor und/oder ein Lichtleiter eines Sensors an dem Wandungsvorsprung-Element angebracht werden, wobei der Sensor das eingestrahlte Licht der Lichtquelle beispielsweise durch einen zweiten Sensorbereich zumindest teilweise erfasst bzw. einfängt. Bei Kenntnis bzw. Erfassung des Spektrums des eingestrahlten Lichtes und Erfassung des Spektrums des Lichtes, welches das Medium zumindest teilweise passiert, kann beispielsweise eine Absorption ermittelt werden.

Die Sensor-Anbringungsvorrichtung kann ferner auch dazu dienen, andere Elemente als eine Lichtquelle und/oder einen Lichtleiter und/oder einen Sensor, beispielsweise optische Elemente relativ zu dem Wandungsvorsprung und dem Behälter anzubringen. Die Sensor-Anbringungsvorrichtung kann ferner auch dazu dienen bzw. dazu ausgelegt sein, einen oder mehrere pH-Sensoren relativ zu dem Wandungsvorsprung anzubringen und/oder zu befestigen und/oder zu lagern und/oder zu fixieren.

Gemäß einem Aspekt umfasst ein Behälter eine an den Behälter den Wandungsvorsprung anbringbare insbesondere reversibel anbringbare und von dem Behälter bzw. von dem Wandungsvorsprung abnehmbare bzw. entfernbare Sensor-Anbringungsvorrichtung zur Anbringung des Sensors relativ zu dem Wandungsvorsprung. In anderen Worten kann die Sensor-Anbringungsvorrichtung relativ zu dem Wandungsvorsprung je nach Bedarf angebracht und abgenommen werden. Beispielsweise kann eine Anbringung eines Sensors an der Sensor-Anbringungsvorrichtung erfordern, dass die Sensor-Anbringungsvorrichtung von dem Behälter entfernt bzw. abgenommen wird.

Eine von einem Behälter abnehmbare Sensor-Anbringungsvorrichtung kann dazu dienen bzw. dazu ausgelegt sein, an den Wandungsvorsprüngen unterschiedlicher Behälter angebracht und davon abgenommen zu werden. Insbesondere wenn die abnehmbare Sensor-Anbringungsvorrichtung rigide ausgebildet ist und die Sensor-Anbringungsvorrichtung beispielsweise einen optischen Strahlengang vorbestimmt bzw. vorjustiert bzw. vorgibt und beibehält, können Größen für die Medien innerhalb unterschiedlicher Behälter direkt miteinander verglichen werden. Beispielsweise kann anhand einer Transmission und/oder einer Extinktion und/oder einer Absorption festgestellt werden, ob sich die Prozesse, die innerhalb zweier Behälter ablaufen, voneinander unterscheiden und/oder gegeneinander zeitlich versetzt ablaufen.

Eine von einem Behälter abnehmbare Sensor-Anbringungsvorrichtung kann ferner dazu dienen, dass vor dem Anbringen an einem Wandungsvorsprung eines Behälters eine Kalibrations-Messung bzw. eine Hintergrundmessung vorgenommen werden kann. Beispielsweise kann das Licht einer Lichtquelle, welche an der Sensor-Anbringungsvorrichtung angebracht ist, eine Luftschicht passieren, welche im Wesentlichen relativ zu der Sensor-Anbringungsvorrichtung dort vorliegt, wo in einer an den Behälter angebrachten Situation ein Medium relativ zu der Sensor-Anbringungsvorrichtung positioniert wäre und mittels eines Sensors, welcher an der Sensor-Anbringungsvorrichtung angebracht ist, erfasst werden. Im Wesentlichen kann somit das Spektrum eines durch eine Lichtquelle eingestrahlten Lichtes, beispielsweise ein Licht in einem Infrarotspektrum und/oder ein UV/Vis-Spektrum bestimmt bzw. erfasst und/oder ermittelt werden. Nachdem die Sensor-Anbringungsvorrichtung dann an dem Wandungsvorsprung angebracht wurde, bzw. in einer relativ zu dem Wandungsvorsprung angebrachten Situation kann mit derselben optischen Geometrie ein Licht mit einem bestimmten Spektrum, welches das Medium im Behälter-Innenraum des Wandungsvorsprungs, bzw. im Probenvolumen passiert hat, mittels des Detektors bzw. Sensors erfasst werden. Durch eine mathematische Operation beispielsweise durch eine Subtraktion und/oder durch eine Division beider Spektren kann eine Absorption von Licht mit bestimmten Frequenzen bzw. Frequenzbereichen durch das Medium ermittelt werden.

Alternativ kann die Sensor-Anbringungsvorrichtung auch dauerhaft oder zumindest zeitweise an dem Behälter oder an einem Wandungsvorsprung-Element fixiert bzw. angebracht sein. Insbesondere kann eine Sensor-Anbringungsvorrichtung integriert an einem Behälter und/oder einem Wandungsvorsprung-Element vorliegen und/oder einstückig bzw. in einem Stück damit ausgebildet sein.

Gemäß einem Aspekt umfasst deshalb ein Behälter alternativ eine an dem Behälter fixierte Sensor-Anbringungsvorrichtung zur Anbringung des Sensors an dem Wandungsvorsprung.

Eine an dem Behälter fixierte Sensor-Anbringungsvorrichtung kann dazu dienen, dass auf einfache Weise ein optischer Sensor und/oder eine pH-Elektrode relativ zu dem Wandungsvorsprung angebracht werden. Insbesondere kann eine an dem Behälter fixierte Sensor-Anbringungsvorrichtung einstückig mit einem Wandungsvorsprung-Element und/oder mit dem Behälter selbst ausgebildet bzw. ausgeformt sein.

Gemäß einem Aspekt umfasst die Sensor-Anbringungsvorrichtung mindestens eine Aufnahmevorrichtung, welche dazu ausgelegt ist, ein weiteres optisches Element, zusätzlich zu dem optischen Element, welches als Sensorbereich, insbesondere als Fenster dient, vorzugsweise eine Linse und/oder einen Spiegel und/oder ein Prisma und/oder eine Lochblende aufzunehmen und/oder mindestens ein weiteres optisches Element, insbesondere eine Lochblendel"pin hole" und/oder eine Iris und/oder einen Reflektor bzw. einen Spiegel bzw. ein reflektives Element und/oder eine Linse und/oder eine Blende und/oder einen Filter, beispielsweise einen Notch-Filter.

Eine Sensor-Anbringungsvorrichtung mit einen optischen Element kann dazu dienen, dass ein optischer Strahlengang bzw. ein Lichtweg mittels des optischen Elements beeinflusst wird. Beispielsweise kann ein Notch-Filter ein eingestrahltes Licht eines Lasers mit einer bestimmten Wellenlänge bzw. einem bestimmten Wellenlängenbereich im Wesentlichen vollständig oder zumindest teilweise blockieren bzw. herausfiltern, wohingegen Licht einer etwas anderen Wellenlänge den Filter im Wesentlichen passieren kann. Dies kann vorteilhaft sein, wenn Größen mittels Raman-Spektroskopie ermittelt werden. In dem Fall bewirkt eine sogenannte Raman-Verschiebung eine Verschiebung der Wellenlänge eines gestreuten Lichtes, welches gesondert von der eingestrahlten elektromagnetischen Strahlung erfasst werden soll, wohingegen die eingestrahlte elektromagnetische Strahlung mittels eines Notch-Filters im Wesentlichen herausgefiltert bzw. blockiert wird.

Die Sensor-Anbringungsvorrichtung kann beispielsweise auch Aufnahmefächer umfassen, in die optische Elemente, insbesondere modular und/oder austauschbar und/oder reversibel eingesetzt werden kann. Dies erlaubt, einen Strahlengang besonders flexibel und dennoch reversibel an die Bedingungen bzw. an die Methode anzupassen. Alternativ kann die Sensor-Anbringungsvorrichtung auch dauerhaft ein weiteres optisches Element zusätzlich zu dem als Sensorbereich dienendes optisches Element umfassen, welches beispielsweise mit der Sensor-Anbringungsvorrichtung verklebt und/oder verschweißt und/oder verschraubt ist.

Gemäß einem Aspekt umfasst das Wandungsvorsprung-Element, insbesondere der Wandungsvorsprung mindestens ein weiteres optisches Element, insbesondere eine Lochblende und/oder einen Reflektor und/oder einen Filter, beispielsweise einen Notch-Filter.

Gemäß einem Aspekt ist der Behälter dazu ausgelegt, einen Bestandteil eines Einweg-Bioreaktors darzustellen.

Insbesondere stellt gemäß einem Aspekt der Behälter im Wesentlichen einen Einweg-Bioreaktor dar, derart, dass der Wandungsvorsprung an einer Behälterwandung des Behälters, also des Einweg-Bioreaktors angeordnet ist.

Gemäß einem Aspekt stellt der Behälter einen Einweg-Behälter dar.

Ein Einweg-Element, wie ein Einweg-Behälter, insbesondere ein Einweg-Bioreaktor hat allgemein den Vorteil, dass dieser steril bereitgestellt werden kann und nach der Verwendung und Kontamination mit Inhalt nicht wieder gereinigt bzw. autoklaviert werden muss, sondern entsorgt werden kann. Durch die Verwendung kostengünstiger Materialien zur Herstellung von Einweg-Bioreaktoren können Prozesse besonders kostengünstig durchgeführt bzw. umgesetzt werden. Es können alle Bestandteile eines Behälters, insbesondere eines Bioreaktors, sowie sämtliches Zubehör als Einweg-Elemente ausgebildet sein. Alternativ können teilweise Bestandteile eines Behälters, insbesondere eines Bioreaktors, sowie teilweise Bestandteile eines Zubehörs als Einweg-Elemente ausgebildet sein, wohingegen andere Bestandteile Mehrweg-Elemente darstellen.

Insbesondere kann an der Behälterwandung eines Einweg-Bioreaktors ein Wandungsvorsprung-Element mit einem Wandungsvorsprung zumindest teilweise aus einem Kunststoff und/oder einem Metall, insbesondere Stahl angebracht und/oder fixiert und/oder angeklebt und/oder angeschweißt sein. Derart können Wandungsvorsprung-Element und Behälter bzw. Einweg-Bioreaktor mehrstückig, insbesondere zweistückig bzw. in zwei Stücken ausgebildet sein und über ein Verbundstoff verbunden werden. Alternativ können Wandungsvorsprung-Element und Behälter bzw. Einweg-Bioreaktor einstückig ausgebildet sein.

Gemäß einem Aspekt ist der Behälter dazu ausgelegt, einen Bestandteil eines Mehrweg-Bioreaktors darzustellen.

Gemäß einem Aspekt stellt der Behälter einen Mehrweg-Behälter, insbesondere einen Mehrweg-Bioreaktor dar, derart, dass der Wandungsvorsprung an einer Behälterwandung des Behälters, also des Mehrweg-Bioreaktors angeordnet ist.

In Fällen, in denen eine besonders große Menge eines Mediums, beispielsweise mehr als 500 I, insbesondere mehr als 5000 I in einem Behälter prozessiert und/oder gelagert und/oder transportiert werden soll, ist es vorteilhaft einen besonders großen Behälter, beispielsweise einen Stahltank zu verwenden. Solche Behälter erweisen sich als besonders kostengünstig in der Mehrweg-Verwendung, beispielsweise als Mehrweg-Bioreaktoren und/oder Mehrweg-Fermenter und/oder Mehrweg-Mischsysteme und/oder Mehrweg-Braukessel und/oder Mehrweg-Gärsysteme.

Insbesondere kann relativ zu und/oder an der Behälterwandung eines Mehrweg-Behälters bzw. Mehrweg-Bioreaktors ein Wandungsvorsprung-Element mit einem Wandungsvorsprung, zumindest teilweise ausgeformt aus einem Metall, insbesondere aus Stahl, angebracht und/oder verschraubt und/oder fixiert und/oder angeklebt und/oder angeschweißt sein. Derart können Wandungsvorsprung-Element und Behälter bzw. Mehrweg-Bioreaktor mehrstückig, insbesondere zweistückig ausgebildet sein und über ein Verbundstoff und/oder andere Mittel verbunden werden. Alternativ können Wandungsvorsprung-Element und Behälter bzw. Mehrweg-Bioreaktor einstückig ausgebildet sein.

Gemäß einem Aspekt umfasst ein Wandungsvorsprung-Element, das den Wandungsvorsprung und optional eine Wandungsausbuchtung umfasst, insbesondere der Wandungsvorsprung und/oder eine Wandungsausbuchtung mindestens einen Zugang, welcher dazu ausgelegt ist, dass insbesondere ein pH-Wert durch den Zugang mittels einer pH-Elektrode erfasst werden kann. Die pH-Elektrode kann dabei im physikalischen bzw. physischen Kontakt mit dem Probenvolumen stehen und eine Größe bzw. einen Parameter des Probenvolumens ermitteln. Die pH-Elektrode kann in den Behälter-Innenraum, insbesondere in das Probenvolumen ragen und mit einem Medium in physischem Kontakt stehen. In anderen Worten umfasst ein Wandungsvorsprung-Element einen Sensorbereich. Insbesondere umfasst ein Wandungsvorsprung und/oder eine optionale Wandungsausbuchtung einen Sensorbereich. Der Sensorbereich stellt dabei einen Zugang dar, beispielsweise eine Öffnung für das Anbringen bzw. Lagern bzw. Halten von einer pH-Elektrode.

Es kann beispielsweise vorteilhaft sein, eine optische Methode und eine pH-Wert-Messung gleichzeitig zur Überwachung von Prozessen auf komplementäre Weise anzuwenden, um möglichst präzise und insbesondere ergänzende bzw. nichtredundante Größen zu erfassen und Informationen über den Ablauf eines Prozesses zu erhalten.

Gemäß einem Aspekt umfasst ein Behälter ein Wandungsvorsprung-Element, welches den Wandungsvorsprung und optional möglicherweise eine Wandungsausbuchtung umfasst. Es kann demnach sein, dass ein Wandungsvorsprung an einem separaten Wandungsvorsprung-Element angeordnet ist, welches dann wiederum an einem Behälter angebracht bzw. befestigt ist oder angebracht werden kann. Ferner kann das Wandungsvorsprung-Element neben dem Wandungsvorsprung auch eine Wandungsausbuchtung umfassen. Es kann sein, dass ein Wandungsvorsprung-Element nachträglich an einem Behälter angebracht werden kann oder an einem Behälter ausgetauscht werden kann.

Eine Wandungsausbuchtung kann dem Wandungsvorsprung-Element durch seine Form eine erhöhte Stabilität verleihen. Ferner kann eine Wandungsausbuchtung zusätzlichen Raum bieten für einen Zugang und/oder eine Öffnung und/oder einen Sensorbereich durch welchen bzw. durch welche ein Sensor, beispielsweise ein optischer Sensor, insbesondere aber ein pH-Sensor beispielsweise mit dem Behälter-Innenraum bzw. dem im Behälter-Innenraum enthaltenen Medium in Kontakt treten kann. Beispielsweise kann an einer Wandungsausbuchtung eine pH-Elektrode angebracht sein. Im Behälter-Innenraum des Behälters erfährt der Sensor wegen der Wandungsausbuchtung, je nachdem wie lange dieser in den Behälter-Innenraum hineinragt, zumindest teilweise einen Schutz beispielsweise vor herumwirbelnden Teilen oder Rührelementen.

Gemäß einem Aspekt umfasst ein Behälter ein Wandungsvorsprung-Element, welches den Wandungsvorsprung umfasst und einstückig mit dem Behälter ausgebildet ist. In anderen Worten kann ein Behälter mit einem Wandungsvorsprung-Element und einem Wandungsvorsprung in einem Stück ausgebildet sein, beispielsweise mittels einer Gusstechnik oder mittels einer 3D-Printmethode.

Gemäß einem Aspekt umfasst ein Behälter ein Wandungsvorsprung-Element, welches den Wandungsvorsprung umfasst und mehrstückig mit dem Behälter ausgebildet ist. In anderen Worten kann der Behälter ein Wandungsvorsprung-Element und insbesondere einen Wandungsvorsprung umfassen, wobei das Wandungsvorsprung-Element nachträglich an dem Behälter angebracht wurde, beispielsweise mittels Verkleben, Verschweißen, Verschrauben, (Auf- oder An-) Stecken oder Verschmelzen.

Die Erfindung betrifft auch ein Wandungsvorsprung-Element zur Befestigung an einer Behälterwandung eines Behälters umfassend einen Wandungsvorsprung, insbesondere auch eine Wandungsausbuchtung, für die Anbringung des Wandungsvorsprungs an einer Behälterwandung eines Behälters, wobei der Wandungsvorsprung
- für die Anbringung mindestens eines Sensors von der Außenseite des Behälters zur Erfassung mindestens einer Größe bzw. Messgröße bzw. eines Parameters, insbesondere einer physikalischen und/oder chemischen und/oder biologischen Größe von einem in einem Behälter-Innenraum enthaltenen Medium ausgelegt ist;
- den Behälter-Innenraum zumindest teilweise umgebend ausgebildet ist; und
- mindestens einen Sensorbereich umfasst, welcher dazu ausgelegt ist, dass die Größe bzw. der Parameter durch den Sensorbereich mittels des Sensors erfasst werden kann.

Es kann also ferner ein Wandungsvorsprung-Element zur Anbringung eines Wandungsvorsprungs an einen Behälter als einzelnes Element bereitgestellt sein, wobei der Wandungsvorsprung-Element den Wandungsvorsprung für die Anbringung bzw. Aufnahme mindestens eines Sensors bzw. Detektors, insbesondere eines optischen Sensors zur Messung mindestens einer Größe von in einem Behälter-Innenraum enthaltenen Medien umfassen. Dies kann insbesondere vorteilhaft sein, wenn Behälter derart nachgerüstet werden sollen, dass sie mit einem Wandungsvorsprung-Element, sowie einem Wandungsvorsprung versehen werden. In der getrennten Fertigung von Behälterwandung und Wandungsvorsprung-Element können dann in einem (letzten) Schritt beide Elemente vorzugsweise formschlüssig und dicht zusammengefügt bzw. aneinander angebracht werden.

In einem an einen Behälter angebrachten Zustand umgibt der Wandungsvorsprung zumindest teilweise den Behälter-Innenraum, insbesondere zumindest teilweise ein Probenvolumen. Der Wandungsvorsprung umfasst bevorzugt mindestens einen Sensorbereich, insbesondere mindestens ein optisches Element, bevorzugt ein Fenster und/oder mindestens einen Zugang, wobei der Sensorbereich bzw. der Zugang dazu ausgelegt ist, dass die Größe durch den Sensorbereich bzw. den Zugang mittels des Sensors, insbesondere und im Wesentlichen ohne Entnahme eines Probenvolumens erfasst werden kann.

Die Befestigung des Wandungsvorsprung-Elements an der Behälterwandung kann gemäß einem Aspekt reversibel sein, was den Vorteil hat, dass das Wandungsvorsprung-Element für verschiedene Behälter verwendet werden kann.

Die Befestigung des Wandungsvorsprung-Elements an der Behälterwandung kann aber auch gemäß einem Aspekt irreversibel sein, was den Vorteil hat, dass das Wandungsvorsprung-Element mit einem Single-Use-Behälter ausgebildet und einmalig verwendet werden kann.

Gemäß einem Aspekt ist das Wandungsvorsprung-Element sterilisierbar bzw. dazu ausgelegt, sterilisiert bzw. autoklaviert bzw. ultrahoch erhitzt zu werden.

Insbesondere ist das Wandungsvorsprung-Element aus einem oder mehreren Materialien ausgebildet, welche sterilisierbar bzw. autoklavierbar sind. Beispielsweise kann das Wandungsvorsprung-Element aus einem Stahl und/oder Kunststoff, insbesondere aus einem Polymer ausgebildet sein. Dies hat den Vorteil, dass der Behälter mitsamt dem Wandungsvorsprung-Element vor der Verwendung sterilisiert werden kann, sodass ein Medium, welches beispielsweise in den Behälter-Innenraum des Behälters gefüllt wird und teilweise in das von dem Wandungsvorsprung teilweise umgebenen Probenvolumen fließen kann, insbesondere nicht mit mikrobiologischem Material kontaminiert wird. Das Sterilisieren kann vor der ersten und letzten Verwendung eines Einweg-Behälters, insbesondere eines Einweg-Bioreaktors erfolgen oder das Sterilisieren kann vor und nach bzw. zwischen jeder Verwendung eines Mehrweg-Bioreaktors erfolgen.

Die Erfindung betrifft auch eine Sensor-Anbringungsvorrichtung zur Anbringung bzw. Fixierung bzw. Lagerung mindestens eines Sensors bzw. eines Detektors bzw. einer Sensorvorrichtung bzw. einem Element einer Sensorvorrichtung relativ zu einem bzw. an einem Sensorbereich eines Wandungsvorsprungs eines Behälters von einer Außenseite des Behälters, wobei die Sensor-Anbringungsvorrichtung
- eine Aufnahmevorrichtung bzw. Halterung bzw. Fixierung umfasst zur Aufnahme bzw. zum Halten bzw. zum Fixieren des Sensors bzw. des Detektors bzw. der Sensorvorrichtung bzw. dem Element einer Sensorvorrichtung zur Erfassung mindestens einer Größe bzw. eines Parameters von einem in einem Behälter-Innenraum des Behälters enthaltenen Medium; und
- mittels einer Aussparung bzw. Ausbuchtung relativ zu dem bzw. an dem Wandungsvorsprung angebracht werden kann, derart, dass zumindest ein Abschnitt des Wandungsvorsprungs und zumindest ein Teil des Mediums, sowie ein Teil des Probenvolumens für die Erfassung bzw. Messung der Größe durch den Sensorbereich, insbesondere das optische Element, bevorzugt das Fenster mittels des Sensors innerhalb der Aussparung positioniert ist.

Eine Sensor-Anbringungsvorrichtung kann beispielsweise universell zur Anbringung bzw. Fixierung bzw. Lagerung mindestens eines Sensors oder mehrerer Sensoren bzw. eines Detektors bzw. einer Sensorvorrichtung bzw. einem Element einer Sensorvorrichtung an Sensorbereichen von Wandungsvorsprüngen mehrerer Behälter dienen. Beispielsweise kann an der Sensor-Anbringungsvorrichtung ein Sensor dauerhaft oder zumindest über einen Zeitraum angebracht bzw. fixiert sein, wobei die Sensor-Anbringungsvorrichtung zunächst zur Erfassung einer Größe, insbesondere einer physikalischen und/oder chemischen und/oder biologischen Größe an einem Wandungsvorsprung eines ersten Behälters angebracht wird und nach der Erfassung dann zur erneuten Erfassung einer Größe an einem Wandungsvorsprung eines zweiten Behälters angebracht wird. So kann nacheinander mittels einer Sensor-Anbringungsvorrichtung mit einem Sensor und einem Messsystem eine Größe einer Reihe von Behältern bzw. Prozessen bzw. Medien überwacht werden.

Gemäß einem Aspekt ist die Sensor-Anbringungsvorrichtung dazu ausgelegt, zumindest einen optischen Sensor bzw. Detektor und/oder einen Lichtleiter relativ zu einem Sensorbereich anzubringen bzw. zu fixieren bzw. zu befestigen bzw. zu lagern, derart dass die Größe mittels einer optischen Methode, insbesondere einer optischen Spektroskopie erfasst werden kann.

Gemäß einem Aspekt ist die Sensor-Anbringungsvorrichtung dazu ausgelegt, Lichtleiter und/oder Sensoren, beispielsweise zwei Lichtleiter oder einen Lichtleiter und einen Sensor derart relativ zueinander anzuordnen, dass eine transmissive und/oder eine reflektive Strahlengang-Anordnung besteht bzw. entsteht.

Besonders vorteilhaft wirkt sich aus, wenn Lichtleiter und/oder Sensoren an der Sensor-Anbringungsvorrichtung derart fixiert relativ zueinander ausgerichtet sind, dass ein vorjustierter bzw. dauerhaft fixierter Strahlengang bereitgestellt ist und über einen längeren Zeitraum Messungen unter denselben Bedingungen bzw. Justage-Bedingungen vorgenommen werden können. Dies ist insbesondere vorteilhaft für Hintergrundmessungen und Vergleiche von Messdaten und Kalibrierungen. Es kann zusätzlich auch sein, dass ein solcher Strahlengang, insbesondere ein transmissiver und/oder reflektiver, auch feinjustierbar ist, derart, dass sich Strahlengänge im Wesentlichen reversibel anpassen lassen.

Für den Fall, in dem eine reflektive Strahlengang-Anordnung bereitgestellt ist bzw. besteht, kann beispielsweise durch einen einzigen Sensorbereich, insbesondere ein optisches Element, bevorzugt ein Fenster Licht einer Lichtquelle beispielsweise durch einen Lichtleiter in das Medium gesendet bzw. gestrahlt werden. An einem reflektiven und/oder streuenden Element kann das Licht im Behälter-Innenraum des Behälters dann zurückreflektiert und/oder zurückgestreut werden, den Sensorbereich passieren und von einem Detektor und/oder einem Lichtleiter eines Detektors erfasst werden.

Für den Fall, in dem eine transmissive Strahlengang-Anordnung besteht, kann beispielsweise durch einen ersten Sensorbereich, insbesondere ein erstes optisches Element, bevorzugt ein erstes Fenster Licht einer Lichtquelle beispielsweise durch einen Lichtleiter in das Medium gesendet werden bzw. eintreten und nach Passieren eines Teils des Mediums im Behälter-Innenraum des Behälters eine zweiten Sensorbereich, insbesondere ein zweites optisches Element, bevorzugt ein zweites Fenster passieren und von einem Detektor und/oder einem Lichtleiter eines Detektors erfasst werden.

Alternativ kann ein Strahlengang bezüglich eines Teils des Strahls bzw. bezüglich eines Teils der Querschnittsfläche des Lichtstrahls transmissiv und bezüglich des anderen Teils des Strahls reflektiv sein. Beispielsweise kann ein zweiter Sensorbereich für eine transmissive Strahlengang-Anordnung zur Hälfte bzw. zum Teil mit einem Spiegel verdeckt sein, sodass ein Teil des Strahls bzw. bezüglich ein Teil der Querschnittsfläche des Lichtstrahls einer reflektiven Strahlengang-Anordnung folgt.

Insbesondere können Absorptionsmessungen mittels einer transmissiven oder einer reflektiven Strahlengang-Anordnung erfolgen. Die reflektive Strahlengang-Anordnung ermöglicht dabei, dass die optische Wegstrecke durch das Medium im Wesentlichen gegenüber der transmissiven Strahlengang-Anordnung verlängert ist.

Gemäß einem Aspekt ist die Sensor-Anbringungsvorrichtung dazu ausgelegt, relativ zu bzw. an einer Flusszelle bzw. einem Bypass angeordnet bzw. gelagert bzw. fixiert zu werden, derart, dass mittels eines an der Sensor-Anbringungsvorrichtung angebrachten bzw. gelagerten bzw. fixierten Sensors durch einen Sensorbereich, insbesondere durch ein optisches Element, bevorzugt ein Fenster der Flusszelle eine (physikalischer und/oder chemische und/oder biologische) Größen eines darin fließenden bzw. darin gelagerten bzw. darin befindlichen Mediums erfasst bzw. gemessen werden kann.

Insbesondere ist die Sensor-Anbringungsvorrichtung universell und modular einsetzbar, derart, dass die Sensor-Anbringungsvorrichtung dazu ausgelegt ist, relativ zu bzw. an Wandungsvorsprüngen verschiedener Behälter und/oder verschiedener Flusszellen angebracht bzw. gelagert bzw. fixiert zu werden. Insbesondere sind auch mehrere Wandungsvorsprünge sowie Wandungsvorsprung-Elemente einheitlich ausgebildet, derart, dass beispielsweise Wandungsvorsprünge an unterschiedlichen Behältern dieselbe Form aufweisen und/oder dieselbe Form aufweisen, wie ein Abschnitt einer Flusszelle, welche einen Sensorbereich, insbesondere ein optisches Element, bevorzugt ein Fenster aufweist. Derart kann eine universelle Steckverbindung zwischen Sensor-Anbringungsvorrichtung und Wandungsvorsprung und/oder Flusszelle gebildet werden. Dabei kann man annehmen, dass ein Wandungsvorsprung eine Sensor-Anbringungsvorrichtung aufnehmen kann. Man kann alternativ auch annehmen, dass eine Sensor-Anbringungsvorrichtung einen Wandungsvorsprung oder eine Flusszelle aufnehmen kann.

Es kann sein, dass der Behälter mit dem Wandungsvorsprung im Wesentlichen bei dem Schritt der Anbringung der Sensor-Anbringungsvorrichtung relativ zu bzw. an den Wandungsvorsprung nicht bewegt wird, wohingegen die Sensor-Anbringungsvorrichtung beispielsweise auf den Wandungsvorsprung aufgesteckt oder an den Wandungsvorsprung angesteckt wird. Dies ist insbesondere dann der Fall bzw. bevorzugt, wenn der Behälter besonders groß und/oder schwer ist und/oder am Ort fixiert ist. Dann kann der Fall zutreffen, in dem man annimmt, dass ein Wandungsvorsprung eine Sensor-Anbringungsvorrichtung aufnimmt. Alternativ wird eine Sensor-Anbringungsvorrichtung bei dem Schritt der Anbringung relativ zu dem bzw. an den Wandungsvorsprung oder die Flusszelle bzw. den Bypass im Wesentlichen nicht bewegt, wohingegen der Behälter mit dem Wandungsvorsprung oder der Bypass relativ zu der Sensor-Anbringungsvorrichtung bewegt werden muss. Dann kann der Fall zutreffen, in dem eine Sensor-Anbringungsvorrichtung einen Wandungsvorsprung oder einen Bypass aufnimmt.

Es kann sein, dass ein Sensor von einer Sensor-Anbringungsvorrichtung zunächst aufgenommen wird bzw. ein Sensor zunächst an einer Sensor-Anbringungsvorrichtung angebracht bzw. gelagert bzw. fixiert wird und im Anschluss die Sensor-Anbringungsvorrichtung mit dem Sensor relativ zu bzw. an dem Wandungsvorsprung angebracht bzw. gelagert bzw. fixiert wird.

Es kann alternativ sein, die Sensor-Anbringungsvorrichtung zunächst ohne Sensor relativ zu bzw. an dem Wandungsvorsprung angebracht bzw. gelagert bzw. fixiert wird und im Anschluss der Sensor von der Sensor-Anbringungsvorrichtung aufgenommen wird bzw. der Sensor an der Sensor-Anbringungsvorrichtung angebracht bzw. gelagert bzw. fixiert wird. Es kann in diesem Fall auch sein, dass die Sensor-Anbringungsvorrichtung dauerhaft relativ zu bzw. an dem Wandungsvorsprung gelagert wird, um wiederholt unterschiedliche bzw. mehrere Sensoren aufzunehmen bzw. zu lagern bzw. zu fixieren bzw. anzubringen.

Die Erfindung betrifft auch ein Verfahren zur Erfassung bzw. Messung bzw. Detektion mindestens einer Größe, insbesondere einer physikalischen und/oder chemischen und/oder biologischen Größe; bzw. eines Parameters; bzw. eines Zustands von einem in einem Behälter-Innenraum eines Behälters enthaltenen Medium umfassend die Schritte:
- Anordnen bzw. Anbringen bzw. Bereitstellen, insbesondere durch Ankleben bzw. Anschweißen bzw. einstückiges Ausbilden eines Wandungsvorsprungs an einer Behälterwandung des Behälters;
- zumindest teilweises Umgeben bzw. Umschließen des Behälter-Innenraums und des Mediums durch den Wandungsvorsprung;
- Bereitstellen bzw. Anordnen bzw. Anbringen mindestens eines Sensorbereichs, insbesondere mindestens eines optischen Elements, bevorzugt eines Fensters und/oder Zugangs an dem Wandungsvorsprung;
- Anbringen von einer Außenseite des Behälters, insbesondere mittels einer Sensor-Anbringungsvorrichtung , mindestens eines Sensors bzw. eines Detektors bzw. einer Sensorvorrichtung, insbesondere eines optischen Sensors relativ zu mindestens einem Wandungsvorsprung; und
- Erfassen der Größe, insbesondere der physikalischen und/oder chemischen und/oder biologischen Größe des Mediums, insbesondere mittels einer optischen Methode, bevorzugt mittels einer optischen Spektroskopie, durch den Sensorbereich mittels des Sensors.

Unter Behältern werden im Rahmen der vorliegenden Erfindung insbesondere Behälter zum Mischen, Lagern und/oder Transportieren, sowie Bioreaktoren bzw. Behälter als Bestandteil von Bioreaktoren und Fermentern, aber auch Gefäße, Kanister und Behälter zur Lagerung von Pufferlösungen verstanden. Ein Bioreaktor oder Fermenter kann einen Behälter umfassen oder darstellen. Es kann sich ferner bei dem Behälter auch beispielsweise um einen Mischtank bzw. -behälter, einen Lagerbehälter, eine Flasche, einen Kanister oder einen Lebensmitteltank bzw. -fass handeln. Es kann sich bei dem Behälter auch um Behälter handeln, in denen chemisches Material aufbewahrt, transportiert und/oder prozessiert wird, auch kann ein Behälter ein chemisches Laborgerät, beispielsweise ein Säulengefäß für die Säulenchromatographie, ein Gefäß oder ein Element beispielsweise einer Destille darstellen. Ein solcher Behälter kann zur Einweg-Verwendung oder zur Mehrweg-Verwendung ausgelegt sein. Ein Behälter kann insbesondere zumindest teilweise aus Kunststoff ausgebildet sein. Alternativ kann ein Behälter auch zumindest teilweise aus einem Metall insbesondere aus Stahl ausgebildet sein. Ferner kann ein Behälter zumindest teilweise aus Glas ausgebildet sein.

Behälter, wie Bioreaktoren, Mischsysteme und Pellet-Tanks dienen im Wesentlichen zur Aufnahme, zur Lagerung und zum Mischen von biologischen Medien, wie z.B. Fluiden und/oder Feststoffen und/oder Gasen. Biologische Medien können in Behältern wie z.B. Beuteln, insbesondere in Kunststoffbeuteln bereitgestellt werden, die ein Volumen von mehreren hundert Litern umfassen können. Die biologischen Medien können bevorzugt innerhalb eines solchen Beutels in den Bioreaktor eingebracht, in dem sie gelagert, temperiert und/oder durchmischt werden können. In einem solchen Bioreaktor können unterschiedliche Untersuchungen an dem biologischen Medium vorgenommen werden.

Als Medium bzw. Medien werden im Rahmen der vorliegenden Erfindung insbesondere Flüssigkeiten, Gase, Suspensionen, Dispersionen, Puffer und/oder Zellkulturbrühen angesehen. Medien können ferner auch Feststoffe, wie beispielsweise Pulver, gepresste Pellets, Partikel, Körner, sowie Mischungen daraus umfassen. Ein Medium kann entsprechend unterschiedliche Bestandteile mit gleichem oder unterschiedlichem Aggregatszustand, beispielsweise eine Emulsion oder eine Dispersion umfassen.

Ein Wandungsvorsprung-Element kann eine Wandungsausbuchtung umfassen mindestens jedoch einen Wandungsvorsprung. Das Wandungsvorsprung-Element kann an einer Behälterwandung angebracht bzw. befestigt bzw. angeordnet sein oder dazu ausgelegt sein, an einer Behälterwandung angebracht bzw. befestigt bzw. angeordnet zu werden. Das Wandungsvorsprung-Element bildet mit der Behälterwandung eine äußere Hülle, welche das beinhaltete Volumen bzw. das Behälter-Innenvolumen bzw. den Behälter-Innenraum des Behälters, welches mit dem Medium gefüllt sein kann, zumindest teilweise umgibt und insbesondere vollständig umgibt bzw. einschließt. Es kann auch sein, dass der Wandungsvorsprung mit der Behälterwandung eine Hülle bildet. Die Hülle bzw. Haut bzw. Wand, die durch die Behälterwandung und das Wandungsvorsprung-Element bzw. den Wandungsvorsprung gebildet wird trennt bzw. isoliert den Behälter-Innenraum, welcher dem Behälter-Innenvolumen entspricht bzw. die Behälter-Innenseite von einer Außenseite, derart, dass ein Medium bzw. ein Inhalt, welches bzw. welcher sich auf der Behälter-Innenseite bzw. in dem Behälter-Innenraum befindet zumindest teilweise im Wesentlichen von der Außenseite abgeschirmt bzw. isoliert bzw. getrennt wird.

Das im Wesentlichen von dem Wandungsvorsprung-Element umgebene Volumen, insbesondere das Probenvolumen steht im Kontakt bzw. Medium- bzw. Fluidaustausch mit dem im Wesentlichen von der Behälterwandung umgebenen Volumen, beispielsweise mittels einer Öffnung. Beispielsweise öffnet sich ein von dem Wandungsvorsprung teilweise umgebenen Spalt zum Behälter-Innenraum hin. Alternativ kann das im Wesentlichen von dem Wandungsvorsprung-Element umgebene Volumen, insbesondere das Probenvolumen von dem im Wesentlichen von der Behälterwandung umgebenen Volumen zeitweise isoliert werden, beispielsweise mittels eines von außen bedienbaren Shutters bzw. einer Klappe.

Der Wandungsvorsprung kann zwei im Wesentlichen zueinander parallele Vorsprungs-Wände umfassen. Eine Vorsprungs-Wand kann einen Sensorbereich umfassen, was dazu führt, dass sich die Vorsprungs-Wand in einem Sensorbereich-Abschnitt, der im Wesentlichen den Sensorbereich darstellt und einen Wandabschnitt, der im Wesentlichen eine Wand darstellt, welche den Sensorbereich nicht umfasst, unterteilt.

Der Wandungsvorsprung kann beispielsweise im Wesentlichen und zumindest teilweise aus einem Kunststoff, Glas oder Metall ausgebildet sein. Der Wandungsvorsprung umfasst ferner ein oder mehrere Sensorbereiche, wobei ein Sensorbereich einen Zugang, insbesondere einen optischen Zugang für einen optischen Sensor, bevorzugt ein optisches Element, wie z.B. ein Fenster, ein Prisma eine Lochblende und/oder eine diffus reflektierende und/oder streuende Oberfläche darstellt. Anders ausgedrückt kann ein Sensorbereich zunächst allgemein als ein Zugang verstanden werden. Insbesondere stellt ein Sensorbereich einen Zugang für einen optischen Sensor dar. Bevorzugt weist sich ein Sensorbereich, insbesondere ein optisches Element, bevorzugt ein Fenster dadurch aus, dass es im Wesentlichen oder zumindest teilweise transparent für einen spektralen Bereich bzw. Wellenlängenbereich einer elektromagnetischen Strahlung ist. Insbesondere ist ein Sensorbereich dazu ausgelegt, die Behälter-Innenseite bzw. das Behälter-Innenvolumen eines Behälters, was dem Behälter-Innenraum entspricht, von der Außenseite dicht zu trennen. Alternativ kann ein Sensorbereich auch eine Öffnung zwischen Behälter-Innenseite und Außenseite umfassen oder darstellen.

Ein Sensorbereich kann beispielsweise im Wesentlichen oder zumindest teilweise aus einem Kunststoff oder Glas oder einem anderen im Wesentlichen durchsichtigen oder für einen spektralen Bereich transparenten Material gebildet sein. Bevorzugt ist ein Sensorbereich transparent bzw. durchsichtig für ein Licht eines breiten insbesondere zumindest teilweise sichtbaren Wellenlängen- bzw. Frequenzspektrum. Der Sensorbereich kann jedoch alternativ oder zusätzlich auch transparent für Licht mit Wellenlängen und/oder einem Wellenlängenbereich im unsichtbaren Wellenlängenspektrum beispielsweise für infrarotes und/oder ultraviolettes Licht sein. So wäre beispielsweise ein Sensorbereich aus Silizium im Wesentlichen transparent für Licht infraroter Wellenlängen, nicht jedoch für Licht sichtbarer Wellenlängen. Ein Sensorbereich aus Glas wäre beispielsweise transparent für sichtbares Licht, nicht jedoch für Teile des ultravioletten Lichtes. Der Wandungsvorsprung kann deshalb beispielsweise aus einem Kunststoff ausgebildet sein und einen Sensorbereich aus Glas, insbesondere aus Quartz-Glas umfassen. Alternativ können der Wandungsvorsprung und der Sensorbereich jedoch auch vollständig aus Glas ausgebildet sein, sodass sich das Material des Wandungsvorsprungs nicht von dem Material des Sensorbereichs unterscheidet. In anderen Worten kann ein Sensorbereich, insbesondere ein optisches Element, bevorzugt ein Fenster beispielsweise zumindest teilweise transparent für Licht im infraroten, sichtbaren, und/oder ultravioletten spektralen Bereich, insbesondere für Wärmestrahlung sein.

Der Begriff des sichtbaren Wellenlängenbereichs betrifft die Wellenlängen des Lichtes, welche für einen Menschen im Wesentlichen sichtbar sind, insbesondere zwischen etwa 380 nm bis 780 nm. Der Begriff des unsichtbaren Wellenlängenbereichs betrifft die Wellenlängen des Lichtes, welche für einen Menschen im Wesentlichen unsichtbar sind, beispielsweise Wellenlängen, die kürzer als etwa 380 nm oder länger als 780 nm sind. Der hier verwendete Begriff des Lichtes beschränkt sich nicht auf den sichtbaren spektralen Bereich, sondern betrifft vielmehr elektromagnetische Strahlung im Allgemeinen.

Daher umfasst der Wandungsvorsprung gemäß einem Aspekt mindestens einen Sensorbereich, welcher insbesondere dazu ausgelegt ist, an dem Wandungsvorsprung ausgetauscht zu werden.

Dies ist besonders dann vorteilhaft, wenn ein Sensorbereich beschädigt wird oder ist, sodass der beschädigte Sensorbereich durch einen neuen Sensorbereich ausgetauscht werden kann, oder wenn eine Messung gegenüber einer vorherigen anderen Messung eine Transparenz eines Sensorbereichs in einem anderen vorbestimmten Wellenlängenbereich voraussetzen würde.

Gemäß einem Aspekt umfasst ein Wandungsvorsprung Vorsprungs-Wände, welche zumindest abschnittsweise eine Verlängerung aufweisen, die in Richtung der Behälter-Innenseite, das heißt in den Behälter bzw. in das Innere des Behälters hinein ragen. Die Verlängerungen ragen dabei in den Behälter-Innenraum und springen von der Innenseite des Wandungsvorsprungs hervor. Ein Wandungsvorsprung umfasst demnach Vorsprungs-Wände, welche auf die Behälter-Innenseite bzw. in den Behälter-Innenraum ragen. Alternativ kann auch ein Vorsprung von Behälterwandung in Richtung der Innenseite hervorspringen.

Mittels einer Verlängerung einer Vorsprungs-Wand kann ein Materialfluss bzw. ein Fluss des Mediums, insbesondere ausgelöst durch ein Rühren bzw. Verrühren des Mediums innerhalb des Behälters, bevorzugt an der Innenseite der Behälterwandung beeinflusst, insbesondere verlangsamt und/oder abgelenkt werden. Die Strömung in das Probenvolumen des spaltförmigen Wandungsvorsprungs kann damit verringert bzw. beruhigt werden. Insbesondere kann ein Medium innerhalb des Probenvolumens im Wesentlichen zum Stehen kommen, obwohl eine Rührvorrichtung den Großteil des Mediums innerhalb des Behälters verrührt.

Das Material des Wandungsvorsprungs kann derartig gestaltet sein, dass Umgebungslicht abgeschirmt oder abgeschwächt wird. Dies kann beispielsweise für den UV-Vis Bereich durch die Wahl eines dunklen Materials erfolgen. Die Abschirmung kann durch die Erhöhung der Materialwandstärke beeinflusst werden.

Ein Behälter kann ein Bestandteil eines Bioreaktors und/oder eines Fermenters sein. Beispielweise kann ein Behälter auch Bestandteil eines Lebensmitteltanks bzw. Lebensmittelfasses oder eines Silos oder eines Speichers sein. Eine Überwachung kann dazu dienen, die Qualität des von dem Tank beinhalteten Mediums zu überprüfen. Beispielsweise kann sich in einem Tank Kuhmilch befinden, deren Qualität während der Lagerung und/oder des Transports überwacht werden soll. Auch kann der Behälter Bestandteil eines Bier- oder Weinfasses oder einer Wein- bzw. Schaumweinflasche für die Flaschengärung sein. Der Behälter kann dazu ausgelegt sein, den Gärungsprozess des Getränks zu überwachen.

Ein Behälter kann auch Bestandteil eines Laborgeräts, insbesondere eines chemischen Laborgeräts sein. Beispielsweise kann ein Behälter eine Säule zur Säulenchromatographie darstellen.

Gemäß einem Aspekt umfasst der Wandungsvorsprung, insbesondere der Spalt und/oder das Probenvolumen einen Kanal bzw. ein kanalförmiges Volumen, welches zumindest teilweise durch eine Kanalführung und/oder ein Leitblech bzw. Leitabschnitt eingeschlossen bzw. umgeben wird. Der Kanal ist dazu ausgelegt, ein sich bewegendes Medium von einem Kanaleingang zu einem Kanalausgang in einer Stromrichtung zu führen. Das Medium kann dabei durch das Probenvolumen strömen, beispielsweise wenn das Medium verrührt oder gemischt wird.

In anderen Worten umfasst ein Wandungsvorsprung einen in den Behälter- bzw. Bioreaktor-Innenraum hineinragenden Strömungskanal bzw. Kanal zum Auffangen von Medium aus dem Behälter-Innenraum und zum Leiten des Mediums durch den Kanal und im Wesentlichen durch das Probenvolumen. Insbesondere strömt ein Medium durch den Kanal wenn das Medium durch eine Rührvorrichtung im Behälter-Innenraum vermischt bzw. verrührt bzw. bewegt wird.

Der Vorteil eines Kanals besteht darin, dass zumindest ein Teil eines Mediums, welches insbesondere mittels einer Rührvorrichtung durch den Behälter bewegt wird, durch eine Kanalöffnung "eingefangen" und in einer vorbestimmten Strömungsrichtung durch den Kanal geführt werden kann. Auf diese Weise kann das Medium im Probenvolumen effizient ausgetauscht werden, was beispielsweise dann erwünscht wird, wenn ein Prozess innerhalb des Behälters abläuft und eine repräsentative Probe in dem Probenvolumen untersucht werden soll.

Die Erfindung wird nachfolgend anhand von in Figuren gezeigten Ausführungsbeispielen näher erläutert. Einzelne, in den Figuren gezeigte Merkmale können mit anderen Ausführungsbeispielen kombiniert werden, sofern sie sich nicht gegenseitig ausschließen. Gleiche Bezugszeichen bezeichnen dabei gleiche oder ähnliche Bauteile der Ausführungsformen. Es zeigen:
**Fig. 1** eine schematische Seitenansicht eines Bioreaktors mit einem Wandungsvorsprung und optischer Messvorrichtung gemäß einer Ausführungsform;
**Fig. 2a** eine schematische Seitenansicht des Querschnitts eines Bioreaktors mit Wandungsvorsprung und optischer Messvorrichtung gemäß einer weiteren Ausführungsform;
**Fig. 2b** eine schematische vergrößerte Seiten-Detailansicht des Querschnitts des Wandungsvorsprungs an einer Behälterwandung gemäß der **Fig. 2a****;**
**Fig. 3a** eine schematische Detailansicht eines Wandungsvorsprungs mit zwei Sensorbereichen und transmissiver Strahlengang-Anordnung;
**Fig. 3b** eine schematische Detailansicht eines Wandungsvorsprungs mit einem Sensorbereich und reflektiver Strahlengang-Anordnung;
**Fig. 3c** eine schematische Detailansicht eines Wandungsvorsprungs mit einem Sensorbereich, einer reflektiver Strahlengang-Anordnung, einem Zugang und einem pH-Sensor;
**Fig. 4** einen schematischen Querschnitt eines Bioreaktors mit Rührelement, Wandungsvorsprung, Wandungsausbuchtung und optischer Messvorrichtung gemäß einer Ausführungsform;
**Fig. 5** einen schematischen Querschnitt eines Einweg-Beutels bzw. -Bioreaktors mit Wandungsvorsprung, Wandungsausbuchtung und optischer Messvorrichtung gemäß einer Ausführungsform;
**Fig. 6** einen schematischen Querschnitt eines Einweg-Beutels bzw. -Bioreaktors mit Rührelement, Wandungsvorsprung, Wandungsausbuchtung und optischer Messvorrichtung gemäß einer Ausführungsform;
**Fig. 7a** einen Detailausschnitt einer Seitenansicht eines Wandungsvorsprungelementes mit Wandungsvorsprung, Wandungsausbuchtung und Sensoranbringungsvorrichtung gemäß einer Ausführungsform;
**Fig. 7b** einen schematischen Querschnitt einer Seitenansicht eines Wandungsvorsprungelementes mit Wandungsvorsprung, Wandungsausbuchtung und Sensoranbringungsvorrichtung gemäß einer Ausführungsform, sowie eine Verbindungsstelle zwischen einer Behälterwandung und einem Wandungsvorsprung-Element gemäß einer Ausführungsform;
**Fig. 8** eine schematische Frontalansicht eines Bioreaktors mit bezüglich der Breitenachse des Behälters geneigtem Wandungsvorsprung und Wandungsausbuchtung gemäß einer Ausführungsform;
**Fig. 9** eine schematische Detailansicht eines Wandungsvorsprungs mit zwei Sensorbereichen und transmissiver Strahlengang-Anordnung, sowie einer Verlängerung der Vorsprungs-Wände des Wandungsvorsprungs gemäß einer Ausführungsform;
**Fig. 10a** eine perspektivische Ansicht eines Wandungsvorsprung-Elementes mit einem Leitblech gemäß einer Ausführungsform;
**Fig. 10b** eine Ansicht von der Innenseite des Wandungsvorsprung-Elementes der **Fig. 10a** mit einem Leitblech gemäß einer Ausführungsform;
**Fig. 10c** eine Ansicht eines Schnittes entlang der Linie A-A durch das Wandungsvorsprung-Element mit einem Leitblech der **Fig. 10b** gemäß einer Ausführungsform von oben;
**Fig. 11a** eine perspektivische Ansicht eines Wandungsvorsprung-Elementes mit einer Kanalführung gemäß einer Ausführungsform;
**Fig. 11b** eine Ansicht von der Innenseite des Wandungsvorsprung-Elementes der **Fig. 11a** mit einer Kanalführung gemäß einer Ausführungsform;
**Fig. 11c** eine Ansicht eines Schnittes entlang der Linie A-A durch das Wandungsvorsprung-Element mit einer Kanalführung der **Fig. 11b** gemäß einer Ausführungsform von oben.

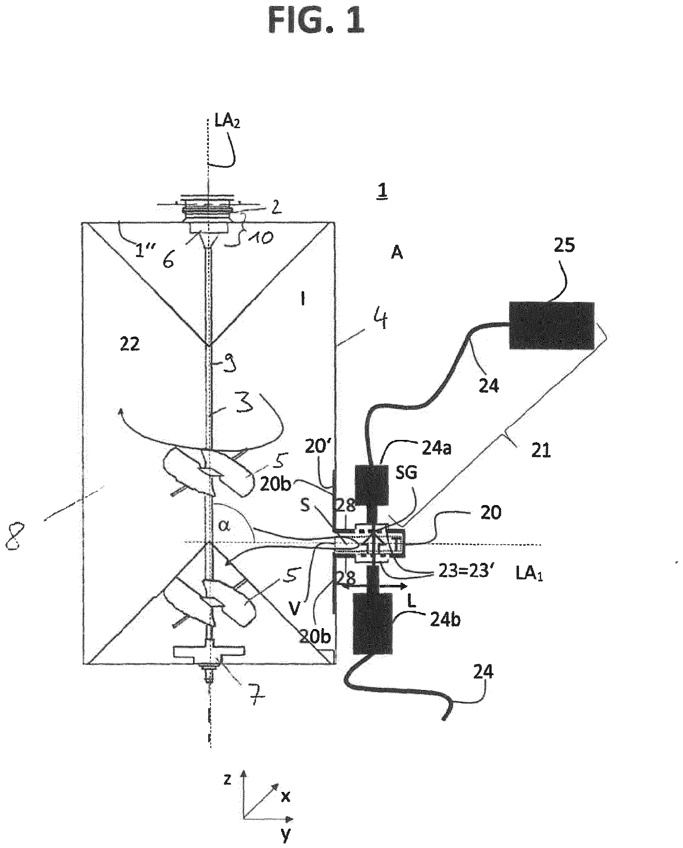

**Fig. 1** ist eine Seitenansicht eines Behälters 1, der Bestandteil eines Bioreaktors ist, gemäß einer Ausführungsform (als eine beispielhafte Ausführungsform eines Behälters mit mindestens einem Wandungsvorsprung für die Anbringung bzw. Aufnahme bzw. Lagerung bzw. Fixierung von zumindest einem Sensor) mit einem Mischsystem bzw. einem Rührelement 3. Bevorzugt ist zumindest der Behälter 1 zur Einweg-Nutzung ausgelegt und insbesondere handelt es sich bei dem Behälter um einen Einweg-Beutel. Alternativ kann der Behälter 1 auch ein Mehrweg-Behälter, beispielsweise ein Stahltank sein. Der Behälter muss zudem nicht zwingend Bestandteil eines Bioreaktors sein.

Der Bioreaktor umfasst neben dem Behälter 1 und dem Rührelement 3, der als Mischsystem oder Rührvorrichtung verstanden werden kann, auch einen Drehstrommotor 10 als Drehstrommaschine für das Rührelement 3. Das Rührelement 3 ist dazu ausgelegt, ein Medium 8 in dem Behälter 1 zu mischen und verrühren. Das Medium 8 kann ein Fluid, insbesondere eine Flüssigkeit und/oder einen Feststoff und/oder ein Gas umfassen und kann insbesondere als ein Fluidgemisch und/oder ein Feststoffgemisch bzw. -gemenge ausgebildet sein, oder auch als ein Gemisch von zumindest einem Fluid und zumindest einem Feststoff.

Der Behälter 1 gemäß der gezeigten Ausführungsform wird von einer Rührwelle 9 des Rührelementes 3 durchdrungen, welche auf der Behälter-Innenseite I des Behälters 1 angeordnet ist und den Behälter 1 vom einen Ende zu einem gegenüber liegenden Ende, also von einer Behälterdecke 1" zu einem Behälterboden 1', entlang der Längsachse LA₂ des Behälters 1 vollständig durchdrungen. Die Längsachse LA₂ des Behälters 1 erstreckt sich im Wesentlichen entlang bzw. parallel zu der Höhe des Behälters vom Behälterboden 1' zur Behälterdecke 1" und parallel zu der z-Achse des gezeigten Koordinatensystems.

Der Bioreaktor weist weiterhin eine Antriebsvorrichtung 2 auf, die außerhalb des Behälters 1 angeordnet ist. Das Rührelement 3 bzw. die Rührwelle 9 ist an die Antriebsvorrichtung 2 gekoppelt. Die Rührwelle 9 des Rührelementes 3 ist im Wesentlichen stabförmig ausgebildet. Die Rührwelle 9 ist im Wesentlichen vollständig im Inneren (auf der Behälter-Innenseite I) des Behälters 1 angeordnet. In der Ausführungsform ist die Rührwelle 9 an einer antriebsseitigen Lagerung 6 und an einer Gegenlagerung 7 gelagert. Die antriebsseitige Lagerung 6 ist unmittelbar benachbart zur Antriebsvorrichtung 2 angeordnet, während die Gegenlagerung 7 an der der Antriebsvorrichtung 2 gegenüberliegenden Seite des Behälters 1 angeordnet ist. An der Rührwelle 9 sind mehrere Rührfortsätze 5 ausgebildet, die dazu ausgelegt sind, sich bei Rotation der Rührwelle 9 um eine Rotationsachse des Rührelements 3 zu bewegen und wenn der Behälter 1 mit einem Medium 8 gefüllt ist, das Medium 8 dabei zu durchmischen. Der Behälter-Innenraum 22 auf der Innenseite I des Behälters 1 kann vollständig oder teilweise mit einem Medium 8 gefüllt sein. Insbesondere kann der Behälter 1 zum Zeitpunkt einer Messung mit einem Medium 8 zumindest teilweise gefüllt sein.

Der Behälter 1 eines Bioreaktor 1 und/oder der Bioreaktor kann alternativ auch ohne Rührelement 3, Rührwelle 9, Antriebsvorrichtung 2, antriebsseitige Lagerung 6 und Gegenlagerung 7, insbesondere ohne jegliches Element, welches zur Durchmischung des Mediums 8 dienen kann, ausgestaltet sein.

An der Behälterwandung 4 des Behälters 1 befindet sich ein Wandungsvorsprung 20, welcher sich über eine Länge L erstreckt. Die Länge L des Wandungsvorsprungs 20 erstreckt sich im Wesentlichen entlang einer Längsachse LA₁ des Wandungsvorsprungs 20, welche in der Ausführungsform im Wesentlichen einen Winkel α von 90° zur Längsachse LA₂ des Behälters 1 aufweist. Die Längsachse LA₁ erstreckt sich auch im Wesentlichen parallel zu der y-Achse des gezeigten Koordinatensystems.

Bevorzugt ist der Behälter 1 mit dem Wandungsvorsprung 20 in zwei kombinierten bzw. zusammengesetzten bzw. -geklebten bzw. -geschweißten Stücken aus Behälter 1 und Wandungsvorsprungs-Element 20' ausgebildet. In dem Fall sind die beiden Stücke umfassend jeweils den Behälter 1 und den Wandungsvorsprung 20 oder das Wandungsvorsprung-Element 20' mit dem Wandungsvorsprung 20 kombinierbar, zusammensetzbar, -klebbar und/oder -schweißbar sofern sie noch nicht zu einem Gegenstand kombiniert wurden. Das Wandungsvorsprungs-Element 20' umfasst den Wandungsvorsprung 20, sowie einen Abschnitt 20b zur Anbringung des Wandungsvorsprung-Elements 20' an den Behälter 1. Das Wandungsvorsprung-Element 20' ist mittels des Abschnitts 20b zur Anbringung des Wandungsvorsprung-Elements 20' an dem Behälter 1 bzw. an der Behälterwandung 4 des Behälters 1 angeordnet bzw. angebracht bzw. anbringbar. Die Behälterwandung 4 hat eine Öffnung bzw. ein Loch, welches durch das Anbringen des Wandungsvorsprung-Elements 20' oder des Wandungsvorsprungs 20 abgedichtet und abgedeckt wird. Die Öffnung in der Behälterwandung 4 erlaubt, dass der gesamte Behälter-Innenraum 22, also das Probenvolumen V mit dem restlichen Behälter-Innenraum 22 verbunden ist und im Kontakt steht. Somit kann ein Materialaustausch bzw. ein Austausch eines Mediums 8 in beiden Teilräumen des Behälter-Innenraums 22 stattfinden.

Der Wandungsvorsprung 20 kann beispielsweise in derselben Stärke bzw. Schichtdicke wie die Behälterwandung 4 ausgebildet sein und zumindest einen Teil des Behälter-Innenraums 22 zumindest teilweise umgeben. Der Wandungsvorsprung 20 kann auch in einer anderen Stärke bzw. Wandungsdicke bzw. Schichtdicke als die restliche Behälterwandung 4 ausgebildet sein. Beispielsweise kann der Wandungsvorsprung 20 im Wesentlichen zumindest teilweise eine dünnere Wandungsdicke bzw. Stärke aufweisen als die restliche Behälterwandung 4, beispielsweise die Hälfte oder ein Drittel weniger. Alternativ kann der Wandungsvorsprung 20 im Wesentlichen auch zumindest teilweise eine dickere Wandungsdicke bzw. Stärke aufweisen als die restliche Behälterwandung 4, beispielsweise die Hälfte oder ein Drittel mehr. Auch das Wandungsvorsprung-Element 20' umfassend den Wandungsvorsprung 20 kann im Wesentlichen eine zumindest teilweise bzw. abschnittsweise verstärkte und/oder dickere Wandung aufweisen als die Behälterwandung 4.

Der Wandungsvorsprung 20 umgibt zumindest teilweise ein Probenvolumen. In der gezeigten Ausführungsform ist das Probenvolumen V als ein Spalt S bzw. ein spaltförmiges Volumen ausgebildet. Der Wandungsvorsprung 20 ragt von der Behälterwandung 4 in Richtung der Außenseite A hin weg. Dabei ragen der Wandungsvorsprung 20 und dessen beide Vorsprungs-Wände 28 im Wesentlichen in einem rechten Winkel zu der Behälterwandung 4 und auch in einem rechten Winkel zu den Wandungen des Abschnitts 20b zur Anbringung des Wandungsvorsprung-Elements 20' an dem Behälter 1 ab. Die zwei Vorsprungs-Wände 28 erstrecken sich dabei parallel zur Längsachse LA₁ des Wandungsvorsprungs 20, wobei sich die Behälterwandung 4 in paralleler Richtung zur Längsachse LA₂ des Behälters 1 erstreckt.

Ein geschwungener Pfeil an dem Wandungsvorsprung 20 auf der Behälter-Innenseite I deutet an, dass ein Medium 8 durch das Probenvolumen V, insbesondere den Spalt S zumindest teilweise fließen bzw. verlaufen kann. In anderen Worten ist durch die konkrete Ausgestaltung sichergestellt, dass ein im Behälter 1 befindliches Medium 8 (insbesondere Flüssigkeit und/oder Gas) in das Probenvolumen V und/oder von diesem heraus fließen bzw. strömen kann.

Der Behälter-Innenraum 22 umfasst das Probenvolumen V und ist mit diesem insbesondere über eine Öffnung verbunden. Der Behälter-Innenraum 22 ohne das Probenvolumen V wird als restlicher Behälter-Innenraum 22 bezeichnet. Wenn der Behälter-Innenraum 22 hinreichend mit dem Medium 8 gefüllt ist, befindet sich das Medium 8 auch in dem Probenvolumen V, insbesondere in dem spaltförmigen Probenvolumen V, derart, dass der Wandungsvorsprung 20 auch einen Teil des Mediums 8 zumindest teilweise umgibt bzw. zumindest teilweise einschließt. In anderen Worten kann das Medium 8 ein Probenvolumen V, insbesondere einen Spalt S ausfüllen bzw. in einen Spalt S hineinfließen. Insbesondere in dem Fall, in dem das Medium 8 beispielsweise mittels des Rührelementes 3 in dem Behälter-Innenraum 22 im Wesentlichen durchmischt wird, kann eine Strömung bzw. ein Strom des Mediums 8 auch durch das Probenvolumen V fließen bzw. verlaufen. Es kann also ein Medium 8 im Probenvolumen V, insbesondere im Spalt S im Wesentlichen zeitweise oder konstant bzw. dauerhaft mit einem Medium 8 aus dem restlichen Behälter-Innenraum 22 ausgetauscht werden. Falls ein Prozess, insbesondere ein chemischer, ein biologischer und/oder biochemischer Prozess innerhalb des Behälters 1 abläuft, kann deshalb gewährleistet werden, dass sich auch innerhalb des Probenvolumens V ein repräsentativer Teil des Mediums 8 aus dem Behälter-Innenraum 22 befindet und ein räumlich inhomogen-ablaufender Prozess im Wesentlichen vermieden werden kann.

Der in **Fig. 1** dargestellte Wandungsvorsprung 20 umfasst zwei Sensorbereiche 23, wobei jeder Sensorbereich 23 an einer der beiden parallelen langen Vorsprungs-Wände 28 angebracht ist, sodass sich auch die Sensorbereiche 23 im Wesentlichen in paralleler Weise gegenüberstehen. Von mindestens einer der beiden Seiten der Vorsprungs-Wände 28 kann somit ein Messzugang (insbesondere optischer Zugang) mittels der Sensorbereiche 23 zu dem Medium 8 im Behälter-Innenraum 22, insbesondere im Probenvolumen V und bevorzugt ein spaltförmiges Probenvolumen V bereitgestellt werden. Der Sensorbereich 23 bzw. Messzugang (z.B. ein optischer Zugang), ist oder umfasst ein optisches Element, bevorzugt ein Fenster 23'. Alternativ könnte im Allgemeinen ein Fenster 23' auch durch ein anderes optisches Element, wie beispielsweise eine Linse, ein Prisma, ein Filter, eine Iris und/oder eine Lochblende ersetzt werden. Das Fenster 23' ist im Wesentlichen durch dessen zumindest teilweise Transparenz für Licht bzw. elektromagnetische Wellen mit einem bestimmten bzw. bestimmbaren Wellenlängenspektrum gegeben und ist im Wesentlichen nicht auf die Transparenz für sichtbares Licht beschränkt. Es ist auch möglich, dass ein Sensorbereich 23 nicht für sichtbares Licht sondern im Wesentlichen für ein Licht einer anderen nicht sichtbaren Wellenlänge (z.B. im Infrarotbereich) transparent ist. Dies kann beispielsweise vorteilhaft sein, wenn der Prozess oder das Medium 8 in dem Behälter 1 empfindlich gegenüber sichtbarem Licht reagiert aber dennoch mittels optischer Messungen Größen, insbesondere physikalische und/oder chemische und/oder biologische Größen des Mediums 8 erfasst werden sollen. Beispielsweise kann in dem Fall ein Sensorbereich 23 zumindest teilweise aus Silizium ausgebildet sein, welches für infrarote Strahlung transparent, für sichtbare Strahlung hingegen im Wesentlichen intransparent ist bzw. eine deutlich reduzierte Transparenz bzw. Durchlässigkeit (z.B. kleiner etwa 10%) aufweist. Es kann ist aber auch möglich, dass der Sensorbereich 23, insbesondere das Fenster 23' zumindest teilweise für Licht eines im Wesentlichen sichtbaren Spektrums und zumindest teilweise für Licht eines im Wesentlichen unsichtbaren Spektrums im Wesentlichen transparent ist. Der Begriff Fenster 23' umfasst entsprechend ein im Wesentlichen lichtdurchlässiges flächiges Element mit einer entsprechend lichtdurchlässigen Fläche. Zwei insbesondere sich gegenüberliegende Fenster 23' können als zwei separate Elemente ausgebildet und/oder gefertigt sein. Alternativ können zwei Fenster 23' auch einstückig ausgebildet, also in einem zusammenhängenden Element gefertigt sein. In anderen Worten müssen zwei oder mehrere Fenster 23' nicht separat voneinander gefertigt sein, sondern können aus einem Stück bestehen. Insbesondere kann der Wandungsvorsprung 20 im Wesentlichen aus einem transparenten Material ausgebildet sein und somit naturgemäß die Eigenschaften der Fenster haben.

Bevorzugt ist, wie zuvor erwähnt, mit dem Begriff "Sensorbereich 23" ein Fenster 23' gemeint, wenn es sich um einen Sensorbereich 23 zur Erfassung optischer Größen mittels einer optischen Sensorvorrichtung 21 handelt. Alternativ oder zusätzlich kann ein Sensorbereich 23 auch einen Zugang und/oder eine Öffnung umfassen oder darstellen.

Relativ zu dem in **Fig. 1** dargestellten Wandungsvorsprung 20 ist von der Außenseite A bzw. von außen ein Sensor bzw. eine Sensorvorrichtung 21 angebracht bzw. anbringbar. In der Ausführungsform umfasst die Sensorvorrichtung 21 einen Lichtleiter 24 bzw. einen Lichtwellenleiter bzw. eine optische Faser, einen Lichtleiter-Einkoppelabschnitt 24a, sowie eine Sensoreinheit, welche in **Fig. 1** durch ein Spektrometer 25 dargestellt wird. Diesbezüglich kann angenommen werden, dass ein Lichtleiter 24 bzw. Lichtwellenleiter im Wesentlichen einer optischen Faser entspricht. Ein optischer Strahlengang bzw. ein Lichtpfad bzw. ein Weg, dem ein Licht folgt durch die Sensorbereiche 23 und das mit zumindest einem Teil des Mediums 8 gefüllten Probenvolumen, ist in der **Fig. 1** als transmissive Strahlengang-Anordnung T dargestellt. Auf einer der Sensorvorrichtung 21 entgegen-gesetzten Seite ist gemäß der Ausführungsform entsprechend der Zeichnung ein Lichtleiter-Auskoppelabschnitt 24b mit einem weiteren Lichtleiter 24, der auch als Lichtwellenleiter bezeichnet wird, dargestellt. Dieser Lichtleiter-Auskoppelabschnitt 24b kann beispielsweise als Lichtquelle dienen, durch die ein Licht eines vorbestimmten bzw. vorbestimmbaren Spektrums ausgekoppelt und in bzw. durch den Sensorbereich 23 und das Probenvolumen gesandt wird. Das Licht kann beispielsweise zumindest teilweise mit dem Medium 8 in dem Probenvolumen V derart wechselwirken, dass dieses zumindest teilweise, insbesondere durch Anregung der Moleküle des Mediums 8 absorbiert wird.

Bevorzugt verläuft ein Licht bzw. ein (bevorzugt kollineares) Lichtbündel im Wesentlichen entlang oder zumindest teilweise parallel zu einer Strahlengangachse SG durch das Probenvolumen V, wie dies in **Fig. 1** angedeutet ist. Eine Strahlengangachse SG kann durch den Verlauf eines Lichtbündels definiert sein, wobei die Strahlengangachse SG im Wesentlichen mittig bzw. zentral innerhalb der Querschnittsfläche des Lichtbündels im Wesentlichen entlang der Propagationsrichtung des Lichtbündels verläuft. Beispielhaft ist eine solche Strahlengangachse SG in der **Fig. 1** abgebildet, um den etwaigen Verlauf bzw. die Propagationsachse bzw. -richtungen eines Lichtbündels anzudeuten. Dazu sind die Lichtleiter-Ein- bzw. Auskoppelabschnitte 24a, 24b derart an dem Wandungsvorsprung 20 angeordnet, dass ein Lichtstrahl, welcher aus dem Lichtleiter-Auskoppelabschnitt 24b austritt, im Wesentlichen entlang oder zumindest parallel zu der Strahlengangachse SG durch das Probenvolumen V bzw. den Spalt S und die Sensorbereiche 23 bzw. Fenster 23' propagiert bzw. verläuft, um dann von dem Lichtleiter-Einkoppelabschnitt 24a zumindest teilweise erfasst bzw. eingefangen bzw. aufgenommen zu werden bzw. in den Lichtleiter-Einkoppelabschnitt 24a eingekoppelt zu werden. Im Wesentlichen verläuft die Strahlengangachse SG und damit die Propagationsrichtung eines Lichtbündels im Wesentlichen parallel auf der Außenseite A zu einer benachbarten Behälterwandung 4 des Behälters 1.

Anstatt eines wie in **Fig. 1** dargestellten Bioreaktors könnte es sich auch um einen Lebensmitteltank, Pellettank, Lagerbehälter, Mischbehälter oder ein anderes Behältnis handeln.

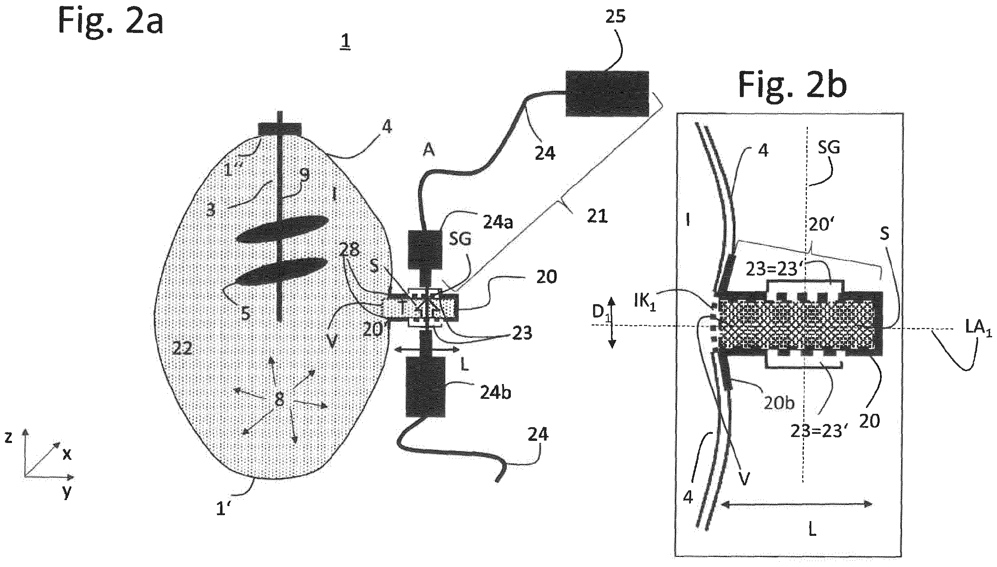

**Fig. 2a** zeigt in einer Seitenansicht eine Ausführungsform eines Bioreaktors mit dessen Behälter 1, wobei der Bioreaktor ein Einweg-Bioreaktor ist und der Behälter 1 ein "single use"- bzw. Einweg-Beutel bzw. Einweg-Behälter ist. Der Bioreaktor umfasst also einen Behälter 1 und ein Mischsystem zur mindestens teilweisen Einweg-Nutzung. Der Bioreaktor umfasst ein Rührelement 3, eine Rührwelle 9 und einen Rührfortsatz 5. Die Rührvorrichtung oder das Rührelement 3 kann in dem Fall beispielsweise zumindest teilweise auch zur Mehrweg-Verwendung geeignet sein, wohingegen der Behälter 1 und im Wesentlichen die Außenhaut bzw. die Behälterwandung 4 zur Einweg-Verwendung geeignet und/oder vorgesehen ist. Es ist auch möglich, dass auch die Rührvorrichtung bzw. das Rührelement 3 vollständig zur Einweg-Verwendung bzw. Nutzung ausgelegt ist. Der Einweg-Behälter 1 kann im Wesentlichen ein Kunststoffbeutel sein, der beispielsweise innerhalb eines anderen starren Behälters, beispielsweise eines Tanks und/oder eines Gerüsts gelagert und/oder aufgehängt werden kann.

In anderen Worten kann zumindest die Außenhaut bzw. die Behälterwandung 4 des Bioreaktors, welche den Behälter-Innenraum 22 bzw. die Behälter-Innenseite I von der Außenseite A zumindest teilweise abschirmt bzw. abgrenzt bzw. trennt, zumindest teilweise einen Kunststoff, insbesondere einen weichen Kunststoff bzw. "Weichplastik" bzw. einen besonders biegsamen Kunststoff umfassen. Denkbar sind insbesondere Weich-PVC, Polyolefin, Polyethylen, Polycarbonat, Zyklo-Olefin Kopolymer, Ko-Polyester und/oder Polystyrol. Ferner kann der Behälter 1 aus einem einlagigen oder mehrlagigen Kunststoff ausgebildet sein, der insbesondere resistent bzw. stabil gegen Beta- bzw. Gamma-Strahlung ist. Im Allgemeinen kann der Behälter-Innenraum 22 eines Behälters 1 ein geschlossenes System darstellen, was insbesondere bei anaeroben Prozessen und/oder unter Ausschluss von Lichteinstrahlung bevorzugt sein kann. Unter Ausschluss von Lichteinstrahlung kann ferner die Behälterwandung 4 teilweise und im Wesentlichen intransparent (z.B. mit weniger als etwa 10% Durchlässigkeit) für Licht in allen spektralen Bereichen oder zumindest für Licht eines bestimmten spektralen Bereichs sein und zumindest einen Teil des Wellenlängenspektrums elektromagnetischer Strahlung, insbesondere des sichtbaren Spektrums herausfiltern bzw. absorbieren.

Ein Einweg-Behälter 1 kann unter Umständen gegenüber mechanischen Einflüssen recht sensibel bzw. empfindlich sein. Beispielsweise kann der Versuch, eine Probe zu entnehmen und/oder einen Prozess mittels einer Messung zu überwachen dazu führen, dass der Einweg-Behälter 1 beschädigt wird, beispielsweise durch eine versehentliche Quetschung und/oder ein Durchstechen. Ein, wie in **Fig. 2a** schematisch dargestellter Wandungsvorsprung 20 zur Anbringung eines Sensors bzw. Detektors, einer Sensorvorrichtung 21 oder mehrerer Sensorvorrichtungen 21 kann besonders vorteilhaft für den Umgang mit einem Einweg-Behälter 1 und dessen im Behälter-Innenraum 22 beinhalteten Medium 8 sein, wennGrößen, insbesondere physikalische und/oder chemische und/oder biologischen Größen des Mediums 8 erfasst werden sollen. Insbesondere die im Wesentlichen nicht-invasive Prozesskontrolle, die dadurch ermöglicht wird, führt dazu, dass das Medium 8 im Behälter-Innenraum 22 im Wesentlichen nicht kontaminiert wird, beispielsweise mit für den Prozessablauf schädlichen Stoffen von außen, insbesondere mikrobiologischen Substanzen und/oder Sauerstoff.

Ein wie in **Fig. 2a** schematisch dargestellter Einweg-Behälter bzw. -Beutel 1 kann derart ausgebildet sein, dass sich die Außenhaut bzw. die Behälterwandung 4 zumindest teilweise nach außen bzw. zur Außenseite A hin wölbt, wenn der Behälter-Innenraum 22 zumindest teilweise mit einem Medium 8 befüllt wird. Zumindest kann sich die Behälterwandung 4 flexibel und/oder dehnbar und/oder sackartig bei einem Befüllen und/oder einem Entleeren des beinhalteten Mediums 8 verhalten. Diese Eigenschaft erschwert insbesondere die Handhabung bei der Überwachung eines im Behälter-Innenraum 22 ablaufenden Prozesses durch Erfassung von Größen des Mediums 8. Aus diesem Grund ist es besonders bevorzugt, dass ein rigider bzw. formstabiler Wandungsvorsprung 20 an der Behälterwandung 4 angebracht bzw. angeordnet bzw. befestigt ist. Der Wandungsvorsprung 20 kann beispielsweise Teil eines Wandungsvorsprung-Elements 20' sein, und/oder an einem Wandungsvorsprung-Element 20' angebracht bzw. angeordnet sein. Das Wandungsvorsprung-Element 20' ist bevorzugt rigide bzw. formstabil ausgebildet. Das Wandungsvorsprung-Element 20', welches den Wandungsvorsprung 20 umfasst, kann deshalb an der Behälterwandung 4 eines Einweg-Behälters angebracht bzw. angeordnet, insbesondere angeklebt und/oder angeschweißt sein. Dazu kann der Wandungsvorsprung-Element 20' einen Abschnitt umfassen, der eine Form der Behälterwandung 4 zumindest teilweise nachahmt oder vorgibt, sodass sich ein bezüglich der Form kontinuierlicher Übergang zwischen Behälterwandung 4 und Wandungsvorsprung-Element 20' ergibt nach der Anbringung. Es ist bevorzugt, dass das Wandungsvorsprung-Element 20' einstückig mit dem Wandungsvorsprung 20 ausgebildet ist, beispielsweise durch Schweißen, Gussverfahren und/oder 3D Drucktechniken. Der Einweg-Behälter 1 kann alternativ auch insgesamt einstückig mit dem Wandungsvorsprung 20 ausgebildet sein.

Das Wandungsvorsprung-Element 20' und insbesondere der Wandungsvorsprung 20 ist bevorzugt zumindest teilweise aus einem sogenannten "Hartplastik" bzw. aus einem steiferen bzw. formstabileren Kunststoff, insbesondere aus einem (schmelzbaren) Thermoplast oder aus einem (nicht schmelzbaren) Duroplast, beispielsweise einem Kunstharz ausgeformt. Insbesondere ist der Kunststoff sterilisierbar z.B. mittels Beta- bzw. Gamma-Strahlung. Allgemein kann das verwendete Material zur Herstellung eines Behälters 1 bzw. zur Herstellung eines Mehrweg- oder Einweg-Bioreaktors mittels thermischer Sterilisation, mittels Dampfsterilisation, mittels Heißluftsterilisation, mittels chemischer und/oder physikalischer Sterilisation (z.B. Beta- oder Gamma-Bestrahlung) sterilisierbar sein.

Durch die formstabile Ausbildung des Wandungsvorsprungs 20 kann gewährleistet werden, dass ein Probenvolumen V, welches mit dem Medium 8 aus dem Behälter-Innenraum 22 gefüllt sein kann, stets einen im Wesentlichen konstanten Wert beibehält. Dies begünstigt den Vergleich von Größen bzw.Parametern die über einen längeren Zeitraum durchgehend oder sporadisch gemessen werden, da keine Korrekturen aufgrund einer (möglicherweise unbekannten) Schichtdickenänderung in Betracht gezogen werden muss. Derart kann insbesondere zu Beginn der Aufzeichnung von Daten bzw. der Erfassung von Größen eine Hintergrundmessung bzw. eine Kalibration erfolgen, die über den gesamten Zeitraum der Datenerfassung als gültig betrachtet werden kann.

Wie in der **Fig. 2a** schematisch gemäß der gezeigten Ausführungsform dargestellt ist, hat der Einweg-Behälter bzw.-Beutel 1 eine nach außen bzw. zur Außenseite A hin gewölbte nicht strikt vordefinierte Form, wohingegen der Wandungsvorsprung 20 eine zumindest teilweise wohldefinierte Kontur bzw. Form aufweist. Es ist leicht vorstellbar, wie umständlich sich eine Handhabung eines derartigen Behälters 1 ohne Wandungsvorsprung 20 bei einer Erfassung von Größen des Inhalts bzw. des Mediums 8 gestalten kann. Insbesondere eine exakte reproduzierbare Ausrichtung von Optikelementen kann umständlich oder gar unmöglich für einen Einweg-Behälter 1 ohne Wandungsvorsprung 20 aber mit einer in einem Einschweißport gelagerten Optik sein, da Gewichtskräfte durch das enthaltene Medium 8 auf einen derartigen Einschweißport wirken würden. Ein solcher Einschweißport könnte beispielsweise eine zur Behälter-Innenseite I hin ausgerichtete Wölbung umfassen, welche eine Öffnung bzw. einen Zugang für einen Sensor oder für eine Probenentnahme umfasst. Dies würde zu einer Auswölbung des Einschweißports führen, was wiederum einen Strahlengang beeinträchtigen kann. Aus diesem Grund hat der Wandungsvorsprung 20 viele Vorteile gegenüber einem Einschweißport mit optischen Elementen. Wie bereits für den Behälter 1 in **Fig. 1** beschrieben wurde und hier nicht weiter ausgeführt, ist an dem Wandungsvorsprung 20 auf der Außenseite A eine identische Sensorvorrichtung 21 wie in **Fig. 1** angebracht, wobei mittels einer transmissiven Strahlengang-Anordnung T durch zwei Sensorbereiche 23 eine Prozessüberwachung mittels einer optischen Methode erfolgen kann. Somit ist die vorgeschlagene Ausführungsform mit einem sich nach außen erstreckenden Wandungsvorsprung 20 vorteilhaft, da diese besonders stabil, insbesondere formstabil gegenüber Gewichtskräften des Mediums 8 innerhalb eines Behälters 1 ausgeformt sein kann. Derart kann im Wesentlichen verhindert werden, dass sich ein zu untersuchendes Volumen bzw. Probenvolumen V insbesondere bezüglich seiner Größe und/oder Form verändert bzw. durch Kräfte wölbt und/oder verformt. Aus diesem Grund kann die beschriebene Ausführungsform im Wesentlichen gewährleisten bzw. ermöglichen, dass ein Probenvolumen V wiederholt unter besonders konstanten Bedingungen insbesondere optisch untersucht werden kann. Dies kann beispielsweise voraussetzen und insbesondere durch die Ausführungsform gewährleistet sein, dass ein optischer Strahlengang bzw. ein optischer Pfad bzw. einen Weg, den ein Licht, beispielsweise ein Laserstrahl insbesondere entlang der Strahlengangachse SG durch das Probenvolumen V nimmt, besonders stabil bzw. konstant ist. Ferner wird durch die nach außen ausgeführte Wölbung insbesondere gewährleistet, dass die (direkte bzw. indirekte) Ankopplung bzw. Anbringung der Sensorvorrichtung 21 einfach gewährleistet wird. Insbesondere durch die von außen bzw. seitlich zugänglichen ein oder mehrere Sensorbereiche 23, welche in der Ausführungsform Fenster 23' sind, ist eine entsprechende Messung besonders einfach gewährleistet.

Das Wandungsvorsprung-Element 20' kann allgemein als ein Port verstanden werden, oder einen Port umfassen, wobei ein Port sich dadurch auszeichnet, dass er Elemente umfasst, welche als Mittel dazu geeignet sind, eine Sensor-Anbringungsvorrichtung 30 relativ zu dem Wandungsvorsprung 20 anzubringen bzw. zu lagern bzw. zu befestigen. Bevorzugt hat ein Wandungsvorsprung 20 in einem angebrachten Zustand ein Lagerspiel.

Der optische Pfad bzw. die optische Strahlengeometrie ist somit im Wesentlichen lediglich durch die Sensor-Anbringungsvorrichtung 30, sowie durch die Geometrie und Zusammensetzung des Wandungsvorsprungs 20 definiert und nahezu unabhängig von auf den Port wirkende Kräfte. Dies kann ein Vorteil für eine bequeme, einfache und sachgerechte Verwendung darstellen, wenn ein Hersteller die Justage bereits vor dem Verkauf einer Sensor-Anbringungsvorrichtung 30 vornehmen kann und der Benutzer die Sensor-Anbringungsvorrichtung 30 lediglich an dem Wandungsvorsprung-Element 20' bzw. relativ zu dem Wandungsvorsprung 20 anbringen muss, um eine Messung durchzuführen bzw. vorzunehmen.

Insbesondere kann sich in dem Fall der Vorgang der Anbringung der Sensor-Anbringungsvorrichtung 30 an dem Wandungsvorsprung-Element 20' als besonders unkompliziert und einfach erweisen. Die Justage der Optik bzw. der Strahlengeometrie an der Sensor-Anbringungsvorrichtung 30 kann vor der Anbringung erfolgen. Insbesondere kann ein Lichtleiter-Ein- bzw. Auskoppelabschnitt 24a, 24b einfach an dem Port bzw. dem Wandungsvorsprung-Element 20' bevorzugt mittels der Sensor-Anbringungsvorrichtung 30 eingeklippt bzw. eingegeklemmt werden. Auch kann die Anbringung der Lichtleiter 24 bzw. der Lichtleiter-Ein- bzw. Auskoppelabschnitte 24a, 24b an der Sensor-Anbringungsvorrichtung 30 vor oder nach der Anbringung der Sensor-Anbringungsvorrichtung 30 an dem Behälter 1 erfolgen.

Auch in **Fig. 2a** wird eine Strahlengangachse SG, entlang der im Wesentlichen ein Lichtbündel propagiert, angedeutet. Bevorzugt verläuft diese Strahlengangachse SG im Wesentlichen parallel auf bzw. zu der Außenseite A zu der benachbarten Behälterwandung 4, allerdings kann es auch sein, dass die Behälterwandung 4, wie in **Fig. 2a** angedeutet, eine Wölbung nach außen aufweist, was dazu führt, dass die Strahlengangachse SG zumindest abschnittsweise nicht parallel zur Behälterwandung 4 verläuft. Dennoch ist es bevorzugt, dass die Strahlengangachse SG und damit die Propagationsrichtung eines Lichtbündels im Wesentlichen senkrecht zu und/oder durch den Spalt S, die Vorsprungs-Wände 28 und die Sensorbereiche 23 bzw. Fenster 23' verläuft. Wie bereits erwähnt, besteht ein Vorteil dieser Ausführungsform deshalb insbesondere darin, dass ein Wandungsvorsprung 20 besonders formstabil ausgebildet ist, wohingegen die Behälterwandung 4 sich verformen und/oder wölben kann. Dies bewirkt, dass eine Strahlengangachse SG stabil und/oder reversibel eingestellt sein kann und besonders stabile Messbedingungen gegeben sein können.

Die **Fig. 2b** ist eine vergrößerte Detailansicht des Wandungsvorsprungs 20. Das Wandungsvorsprung-Element 20' umfasst den Wandungsvorsprung 20 und einen Abschnitt 20b zur Anbringung des Wandungsvorsprung-Elements 20' an den Behälter 1. Der Abschnitt 20b kann sich bevorzugt der Form der Behälterwandung 4 anpassen oder hat eine im Wesentlichen rigide Form, die bereits an die Behälterwandung 4 angepasst ist. In der Ausführungsform der **Figuren 2a** und **2b** umfasst der Wandungsvorsprung 20 zwei Sensorbereiche 23, welche Fenster 23' sind.

Der Wandungsvorsprung 20 mit dem Probenvolumen V liegt im Wesentlichen außerhalb des Krümmungsradius des Einweg-Beutels bzw. des gewölbten EinwegBehälters 1. In anderen Worten ragt das Probenvolumen V bzw. der Spalt S und damit der Wandungsvorsprung 20 von der Behälterwandung 4 nach außen ab. Der Wandungsvorsprung 20 umgibt zumindest teilweise das Probenvolumen V, welches in der Form eines Spaltes S ausgestaltet ist. Das Probenvolumen V ist das Volumen bzw. der Raum des Behälter-Innenraums 22, welches bzw. welcher größtenteils durch den Wandungsvorsprung 20 umgeben ist. Das Probenvolumen V grenzt sich von dem anderen Teil des Behälter-Innenraumes 22, welcher im Wesentlichen durch die Behälterwandung 4 umgeben ist, durch eine imaginäre Konturlinie IK₁ ab (gepunktete Linie in Fig. 2b). Die imaginäre Konturlinie IK₁ ist im Wesentlichen die Verlängerung der Behälterwandungskontur bzw. die Verbindungslinie zwischen den Linien der Behälterwandungskontur, wobei die Behälterwandungskontur die Kontur der Behälterwandung ohne Wandungsvorsprung 20 ist. Die Behälterwandungskontur umfasst also nicht die Kontur des Wandungsvorsprungs 20. Wie zuvor erwähnt, ist damit das Probenvolumen V, welches sich außerhalb der imaginären Konturlinie IK₁ befindet definiert.

Wenn ein Wandungsvorsprungs-Element 20' eine Wandungsausbuchtung 20a umfasst definiert sich die imaginäre Konturlinie IK₁ hingegen durch die Kontur der Wandungsausbuchtung 20a. Die imaginäre Konturlinie IK₁ ist dann im Wesentlichen die Verlängerung der Kontur der Wandungsausbuchtung 20a bzw. die Verbindungslinie zwischen den Konturlinien der Wandungsausbuchtung 20a, wobei die Kontur der Wandungsausbuchtung 20a die Kontur des Wandungsvorsprungs 20 nicht umfasst.

Ein Wandungsvorsprungs-Element 20' umfasst in jedem Fall einen Wandungsvorsprung 20. Wandungsvorsprungs-Element 20' kann ferner eine Wandungsausbuchtung 20a umfassen. Außerdem kann Wandungsvorsprungs-Element 20' einen Abschnitt 20b zur Anbringung des Wandungsvorsprung-Elements 20' an den Behälter 1 umfassen. Insbesondere kann ein Wandungsvorsprungs-Element 20' und bevorzugt ein Abschnitt 20b zur Anbringung auch einen Teil einer Elementverbindung EV umfassen. Beispielsweise kann ein Teil der Elementverbindung EV ein Gewinde sein, welches in eine kompatibles Gewinde an der Behälterwandung 4 geschraubt werden kann.

In anderen Worten ist das Probenvolumen V dasjenige Volumen des Behälter-Innenraumes 22, welches sich von der imaginären Konturlinie IK₁ (gepunktete Linie in Fig. 2b) der Kontur bzw. imaginären Konturlinie der Behälterwandung 4 oder der Kontur bzw. imaginären Konturlinie der Wandungsausbuchtung 20a über eine Länge L entlang der Längsachse LA₁ mit der Probenschichtdicke D₁ des Wandungsvorsprungs 20 erstreckt.

Eine alternative Ausführungsform zu der in den **Figuren 2a** und **2b** gezeigten Ausführungsform, welche hier nicht explizit dargestellt wird, umfasst anstelle von zwei sich gegenüberliegenden Fenstern 23', ein Fenster 23' und gegenüberliegend eine Kombination aus einem Fenster 23' und einem Spiegel bzw. einen Reflektor. Beispielsweise kann die obere Wand des Wandungsvorsprungs 20, insbesondere bezüglich der gezeigten z-Richtung, bzw. die obere Vorsprungs-Wand 28 ein Fenster 23' umfassen und die untere Vorsprungs-Wand 28 kann ein Fenster 23' und einen Spiegel und/oder Reflektor umfassen. Beispielsweise könnte sich die Fläche eines Fenster 23' zumindest teilweise durch die Fläche eines Reflektors überlagern, wobei die reflektive Seite des Reflektors in die Richtung des gegenüberliegenden Fensters 23' zeigt. In diesem Fall können Messungen unter Transmission und Reflexion gleichzeitig getätigt werden. Das heißt, es kann sowohl eine transmissive T als auch eine reflektive Strahlengang-Anordnung. R zur gleichen Zeit verwendet werden. Dies ermöglicht insbesondere, dass mehrere Arten von Spektroskopien mit unterschiedlichen optischen Geometrien verwendet werden können.

Bevorzugt weist die Längsachse LA₁ eines Wandungsvorsprungs 20, wie in **Fig. 2b** angedeutet ist, einen im Wesentlichen rechten Winkel zu der Strahlengangachse SG auf. Dies gilt für eine transmissive T geleichermaßen, wie für eine reflektive Strahlengang-Anordnung R. Bevorzugt weist die Längsachse LA₁ des Wandungsvorsprungs 20, wie in **Fig. 2b** angedeutet ist, auch einen im Wesentlichen rechten Winkel zu der imaginären Konturlinie IK₁ auf. Damit ist die imaginäre Konturlinie IK₁ im Wesentlichen zumindest stellenweise parallel zur Strahlengangachse SG.

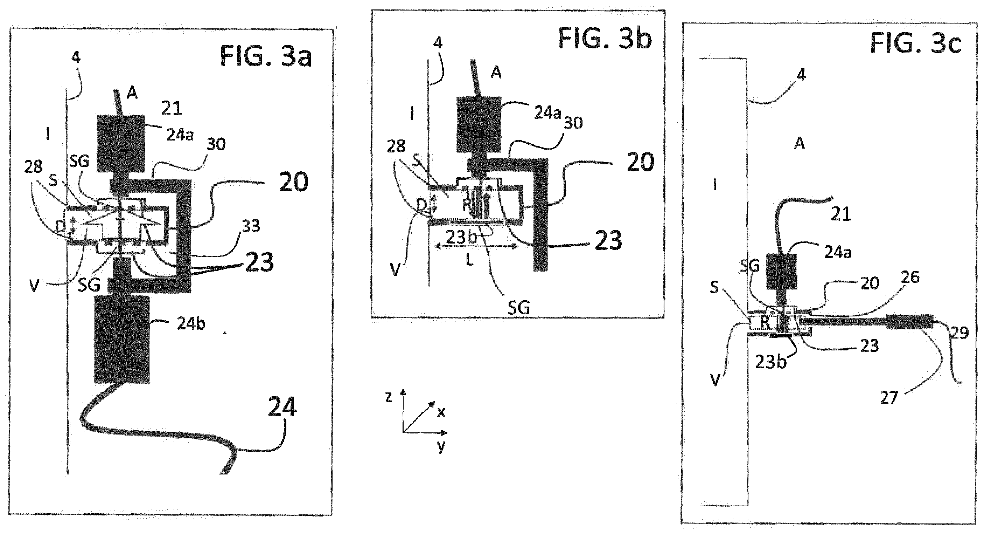

In den **Figuren 3a** bis **3c** sind in Seitenansichten schematisch und detailliert drei verschiedene Ausführungsformen von Wandungsvorsprüngen 20 dargestellt, welche an einer Behälterwandung 4 angebracht sind. Einzelne Merkmale verschiedener Ausführungsformen lassen sich jeweils untereinander kombinieren sofern sie sich nicht ausschließen. Solche Wandungsvorsprünge 20 können an einem beliebigen Behälter 1 angebracht sein, beispielsweise an der Behälterwandung 4 eines Mehrweg-Behälters 1 eines Mehrweg-Bioreaktors oder an der Behälterwandung 4 eines EinwegBehälters 1 eines Einweg-Bioreaktors oder an der Behälterwandung 4 eines Fasses, eines Kanisters, eines Tanks, eines Lebensmitteltanks, eines Transportbehälters und/oder an einem anderen als den bereits genannten Behälter.

Der Wandungsvorsprung 20 aller Ausführungsformen umgibt, wie es bereits für andere Ausführungsformen der Fall ist, zumindest teilweise ein Probenvolumen V, das unter anderem durch eine Probenschichtdicke D₁ und eine Vorsprungs-Länge L definiert wird und mit dem restlichen Behälter-Innenraum 22 in Kontakt bzw. Fluid-Austausch steht bzw. ein Teil dessen ist. Derart kann ein Medium 8 in das Probenvolumen V bei dem Befüllen in den Spalt S hineinfließen und insbesondere mit dem Medium 8 von anderen Positionen des Behälter-Innenraumes 22 ausgetauscht werden. Es kann allerdings auch eine Shutter-Vorrichtung (hier nicht gezeigt) geben, welche von außen bedienbar ist und durch Bedienung das Probenvolumen von dem restlichen Behälter-Innenraum 22 isoliert und/oder trennt. Dies kann insbesondere dann vorteilhaft sein, wenn eine Messung bzw. Erfassung von Größen nicht durch Vorgänge innerhalb des Behälters 1 beispielsweise durch einen Rührvorgang gestört werden soll. Auch kann dadurch verhindert werden, dass ein Prozess auf der Behälter-Innenseite I jenseits des Probenvolumens V durch den Einfall von Licht durch die Fenster 23' gestört wird.

Der in **Fig. 3a** dargestellte Wandungsvorsprung 20 umfasst zwei Sensorbereiche 23, welche ebenfalls als Fenster 23' ausgebildet sind und welche für elektromagnetische Strahlung entsprechend zumindest teilweise durchlässig bzw. transparent sind. In den Beispielen aus **Fig. 3b** und **3c** umfasst jeweils der Wandungsvorsprung 20 nur ein einziges Fenster 23'. Der in **Fig. 3a** dargestellte Wandungsvorsprung 20 umfasst jeweils zwei zu einander im Wesentlichen parallele Vorsprungs-Wände 28, welche im Wesentlichen eine Länge aufweisen, die der Vorsprungs-Länge L entspricht, wenn man die Schichtdicke der Wandung des Wandungsvorsprungs 20 außer Acht lässt.

Die beiden Vorsprungs-Wände 28 und/oder Sensorbereiche 23 und/oder Fenster 23' können alternativ auch in einer nicht zueinander parallel angeordneten Art ausgerichtet sein. Der Vorteil einer im Wesentlichen parallelen Ausrichtung insbesondere der Fenster 23' ist die Vermeidung von Streulicht bzw. die Verringerung von Streulicht, welches bei einem nicht senkrechten Durchtritt von Licht entstehen würde, d.h. wenn die Strahlengangachse SG einen (wesentlich) kleineren Winkel als 90° mit der Fläche mindestens eines Fensters 23b einschließen würde.

**Fig. 3a** stellt in einer schematischen Detail-Seitenansicht eine besondere Ausführungsform eines Wandungsvorsprungs 20 dar, an welchem von der Außenseite A mittels einer Sensor-Anbringungsvorrichtung 30 eine optische Sensorvorrichtung 21, insbesondere ein Lichtleiter-Einkoppelabschnitt 24a und ein Lichtleiter-Auskoppelabschnitt 24b zur Anwendung einer optischen Methode angebracht sind.

Die Sensor-Anbringungsvorrichtung 30 weist bevorzugt ein formstabiles bzw. rigides Gestell bzw. Rahmen bzw. einen Anbringungsvorrichtungskörper auf, welches bzw. welcher insbesondere zumindest teilweise aus einem Metall und/oder einem formstabilen Kunststoff ausgebildet ist. Die Sensor-Anbringungsvorrichtung 30 kann bevorzugt reversibel an den Wandungsvorsprungs 20 angebracht bzw. davon entfernt werden, derart, dass dieselbe Sensor-Anbringungsvorrichtung 30 mehrmals bzw. wiederverwendbar an einem Wandungsvorsprung 20 und/oder an Wandungsvorsprüngen 20 und/oder Behälterwandungen 4 unterschiedlicher Behälter 1 angebracht werden kann.

Alternativ kann die Sensor-Anbringungsvorrichtung 30 dauerhaft an bzw. relativ zu einem Abschnitt des Wandungsvorsprungs 20 und/oder an bzw. relativ zu der Behälterwandung 4 angebracht sein. Insbesondere kann die Sensor-Anbringungsvorrichtung 30 in diesem Fall bevorzugt mittels eines Verbundstoffes und/oder mittels einer Verschraubung und/oder mittels einer Schweißnaht mit der Behälterwandung 4 fixiert bzw. fest verbunden sein.

Die Sensor-Anbringungsvorrichtung 30 umfasst bevorzugt die optischen Elemente der Sensorvorrichtung 21, insbesondere umfassend optische Linsen und/oder Prismen und/oder Spiegel und/oder besonders bevorzugt Lichtleiter bzw. optische Fasern und/oder andere strahlführende Elemente. Falls die Sensor-Anbringungsvorrichtung 30 die genannten optischen Elemente nicht umfasst kann diese zumindest dazu ausgelegt sein, solche optischen Elemente aufzunehmen bzw. zu lagern bzw. zu fixieren. Die Sensor-Anbringungsvorrichtung 30, sowie die optischen Elemente, sofern umfasst oder gelagert, definieren im Wesentlichen die Strahlengeometrie optischen Elemente der Sensorvorrichtung 21. Insbesondere definiert die Sensor-Anbringungsvorrichtung 30, sowie die optischen Elemente, die Strahlengangachse SA bzw. die Geometrie des Lichtstrahls durch das Probenvolumen.

Die Sensor-Anbringungsvorrichtung 30 kann insbesondere einen Sensor bzw. eine Sensorvorrichtung 21, insbesondere eine optische Messvorrichtung derart relativ zu einem Wandungsvorsprung 20 lagern, dass, wie in **Fig. 3a** gezeigt, mittels einer transmissiven Strahlengang-Anordnung T ein oder mehrere Größen erfasst werden können. Beispielsweise kann dazu mittels einem Lichtleiter-Auskoppelabschnitt 24b oder mittels einer anderen Lichtquelle ein Licht bzw. eine elektromagnetische Strahlung, beispielsweise in einem Wellenlängenspektrum, welches Infrarotstrahlung umfasst, von außen durch einen Sensorbereich 23 in den Behälter-Innenraum 22, insbesondere zumindest in einen Abschnitt des Probenvolumens V gesandt bzw. gestrahlt werden.

Die Sensor-Anbringungsvorrichtung 30 weist eine Aussparung 33 auf, welche, wenn die Sensor-Anbringungsvorrichtung 30 an dem Behälter 1 und/oder dem Wandungsvorsprung 20 gelagert ist, zumindest teilweise ausgefüllt ist mit dem Wandungsvorsprung 20 und dem Probenvolumen V. In anderen Worten kann die Sensor-Anbringungsvorrichtung 30 derart relativ zu dem Wandungsvorsprung 20 gelagert bzw. angebracht sein, dass sich zumindest ein Abschnitt des Wandungsvorsprungs 20 und des Probenvolumens V, welches zumindest teilweise mit einem Medium 8 gefüllt sein kann, innerhalb einer Aussparung 33 der Sensor-Anbringungsvorrichtung 30 befinden kann. Anders ausgedrückt kann ein Wandungsvorsprung 20 und ein Probenvolumen V zumindest teilweise von einer Wandung einer Aussparung 33 einer Sensor-Anbringungsvorrichtung 30 umgeben sein.

Wenn das Probenvolumen V mit einem Medium 8 zumindest teilweise befüllt ist, kann die elektromagnetische Strahlung in dem Probenvolumen V mit dem Medium 8, insbesondere den Molekülen und/oder Atomen des Mediums 8, zumindest teilweise im Wesentlichen wechselwirken. Derart kann beispielsweise die elektromagnetische Strahlung (bzw. das eingestrahlte Licht) zumindest teilweise durch das Medium 8 absorbiert und/oder gestreut werden. Mittels eines Lichtleiter-Einkoppelabschnitts 24a kann wiederum das Licht erfasst werden, welches durch das Medium 8 getreten ist bzw. das Medium 8 passiert hat. Derart kann beispielsweise im Fall der Infrarotspektroskopie mittels einer Absorption von Licht bzw. elektromagnetischer Strahlung bestimmter Wellenlängen, insbesondere im infraroten Spektrum, bestimmt werden, wie hoch eine Konzentration einer bestimmten Molekülspezies ist. Dies kann wiederum einen Hinweis auf ein Stadium eines Prozesses geben, in dem sich das Medium 8 zum Zeitpunkt der Erfassung der Daten oder der Größen bzw. der Parameter befindet. Ferner ist es möglich, dass der zumindest eine Sensorbereich 23 zumindest zwei elektrische Elektroden aufweist, die mit dem Medium 8 im Behälter 1 in Kontakt kommen können, so dass eine Widerstandsmessung zwischen diesen Elektroden durchgeführt werden kann, um zumindest eine Eigenschaft des Mediums 8 zu bestimmen.

**Fig. 3b** stellt in einer schematischen Detail-Seitenansicht eine besondere Ausführungsform eines Wandungsvorsprungs 20 mit nur einem einzigen Sensorbereich 23 dar, an welchem von der Außenseite A mittels einer Sensor-Anbringungsvorrichtung 30 eine (insbesondere optische) Sensorvorrichtung 21, insbesondere ein Lichtleiter-Einkoppelabschnitt 24a angebracht werden kann. Es handelt sich hierbei insbesondere um eine transmissive Strahlengang-Anordnung T. Neben dem Lichtleiter-Einkoppelabschnitt 24a kann auch eine Lichtquelle (hier nicht gezeigt) an dem Wandungsvorsprung 20 angebracht sein oder der Lichtleiter-Einkoppelabschnitt 24a kann zur Auskopplung und zur Einkopplung von Licht dienen.

Derart lässt sich elektromagnetische Strahlung durch den Sensorbereich 23 von außen in zumindest einen Abschnitt des Probenvolumens V einstrahlen. Alternativ oder zusätzlich kann auch lediglich ein Sensor einer Sensorvorrichtung 21 bzw. ein Lichtleiter 24 eines Sensors einer Sensorvorrichtung 21 relativ zu dem Sensorbereich 23 angebracht sein, wobei der Sensor einer Sensorvorrichtung 21 beispielsweise dazu ausgelegt ist, eine Fluoreszenz des Mediums 8 zu erfassen. In dem Fall kann es sein, dass das Einstrahlen von Licht im Wesentlichen nicht nötig sein muss, denn eine Fluoreszenz kann beispielsweise durch eine chemische Reaktion in dem Behälter 1 ausgelöst worden sein. Das Licht einer Fluoreszenz-Reaktion kann dann zumindest teilweise durch ein Fenster 23' von dem Behälter-Innenraum 22 auf die Außenseite A gelangen, wo es durch einen Sensor einer Sensorvorrichtung 21 erfasst werden kann.

Durch Reflexion und/oder Streuung an zumindest einem Reflektor 23b und/oder streuenden Elementen und/oder streuenden Partikeln kann Licht, sofern es nicht durch das Medium 8 absorbiert wird, durch den Sensorbereich 23 aus dem Behälter-Innenraum 22 bzw. aus dem Probenvolumen V wieder heraustreten und von dem Lichtleiter-Einkoppelabschnitt 24a erfasst werden. Beispielsweise kann auf der entgegengesetzten Seite des Sensorbereichs 23 an der entgegengesetzten Vorsprungs-Wand 28 ein Spiegel bzw. ein Reflektor 23b bzw. ein streuendes Element auf der Behälter-Innenseite I angebracht sein, welches darauf fallendes Licht zurück reflektieren kann. Mittels eines Lichtleiters 24 kann dann das erfasste Licht beispielsweise an ein Spektrometer 25 weitergeleitet werden, wo es in seine spektralen Anteile zerlegt und von einer Recheneinheit und/oder einem Nutzer beispielsweise analysiert werden kann. Ein streuendes Element kann eine weiße Fläche sein oder ein beugendes Element, wie ein Gitter oder ein anderes Element sein, welches Licht streuen kann.

Auch in der **Fig. 3b** ist eine Strahlengangachse SG angedeutet. Es kann sein, dass ein einfallendes Licht bzw. Lichtbündel im Wesentlichen entlang bzw. parallel zu dieser Strahlengangachse SG verläuft bzw. propagiert. Es kann auch sein, dass insbesondere ein (zurück) reflektiertes Licht bzw. Lichtbündel im Wesentlichen entlang bzw. parallel auf seinem Weg zu einem Sensor einer Sensorvorrichtung 21 zu dieser Strahlengangachse SG verläuft bzw. propagiert. Auch kann zumindest ein Teil eines (zurück) gestreuten Lichtes bzw. Lichtbündels im Wesentlichen entlang bzw. parallel auf seinem Weg zu einem Sensor einer Sensorvorrichtung 21 zu dieser Strahlengangachse SG verlaufen bzw. propagieren.

Ähnlich wie **Fig. 3b** stellt **Fig. 3c** schematisch eine Ausführungsform des Wandungsvorsprungs 20 mit lediglich einem Sensorbereich 23, einem Reflektor 23b und einer reflektiven Strahlengang-Anordnung R dar. Ein Lichtleiter-Einkoppelabschnitt 24a ist gemäß dieser schematischen Darstellung relativ zu dem Wandungsvorsprung 20 und insbesondere dem Sensorbereich 23 angeordnet. In dieser Darstellung bleibt offen, wie die Anbringung erfolgt, da keine Sensor-Anbringungsvorrichtung 30 gezeigt ist, dennoch kann die Anbringung mittels Sensor-Anbringungsvorrichtung 30 erfolgen. Neben dem Sensorbereich 23 umfasst der Wandungsvorsprung 20 einen Zugang 26, welcher dazu ausgelegt ist, dass eine Erfassung eines pH-Wertes des Mediums 8 von außen mittels einer pH-Elektrode erfolgen kann. Insbesondere kann die pH-Elektrode 27 darauf angewiesen sein bzw. dazu ausgelegt sein, dass ein Kontakt zwischen zumindest einem Abschnitt des der pH-Elektrode 27 und dem Medium 8 im Behälter-Innenraum 22 gegeben sein muss. Dies wäre gegeben, wenn zumindest ein Abschnitt der pH-Elektrode 27 durch den Zugang 26, insbesondere in Form einer Öffnung von der Außenseite A auf die Behälter-Innenseite I, insbesondere in das Probenvolumen V gelangt. Derart kann die pH-Elektrode 27 zeitweise oder dauerhaft an dem Wandungsvorsprung 20 zur Erfassung von Größen, insbesondere physikalischer Größen des Mediums 8 angebracht werden. Die pH-Elektrode 27 kann eine Leitung 29 umfassen bzw. mit einer Leitung 29, beispielsweise einer Stromleitung und/oder einer Datenleitung verbunden sein.

Alternativ kann ein Wandungsvorsprung 20 auch mehr als zwei Sensorbereiche 23 und/oder mehr als einen Zugang 26 umfassen. Mittels einer Sensor-Anbringungsvorrichtung 30 kann nur ein oder aber eine Vielzahl von Sensoren 21 und/oder Abschnitte von Sensorvorrichtungen 21 relativ zu dem Wandungsvorsprung 20 angebracht werden.

Eine weitere Ausführungsform, die hier nicht explizit gezeigt ist, umfasst im Wesentlichen eine Kombination der Ausführungsformen, gemäß **Fig. 3a** und **Fig. 3b** oder **Fig. 3a** und **Fig. 3c****.** Gemäß einer Ausführungsform umfasst der Wandungsvorsprung 20 ein reflektives Element und/oder ein Reflektor und/oder ein Spiegel 23b und zwei Sensorbereiche 23, welche jeweils ein Fenster 23' umfassen. Der Wandungsvorsprung 20 ist in dem Fall dazu ausgelegt, dass eine erste Größe bzw. ein erster Parameter mittels einer ersten Sensorvorrichtung, welche vorzugsweise mindestens eine erste optische Faser 24 umfasst, durch eine reflektive Strahlengang-Anordnung R erfasst werden kann und eine zweite Größe bzw. ein zweiter Parameter mittels einer zweiten Sensorvorrichtung, welche vorzugsweise eine zweite optische Faser 24 umfasst, durch eine transmissive Strahlengang-Anordnung T erfasst werden kann. Die erste Größe kann auch die zweite Größe sein oder umfassen. Dies könnte dadurch erreicht werden, dass entweder ein Fenster 23' gegenüber dem anderen Fenster 23' etwas kleiner ist und stattdessen ein Reflektor 23b den Platz, den das Fenster 23' einnehmen würde, einnimmt. Außerdem kann auch die Fläche eines Fensters von außen oder von innen mit der Fläche eines Spiegels und/oder eines Reflektors überlagert werden bzw. räumlich überlappen.

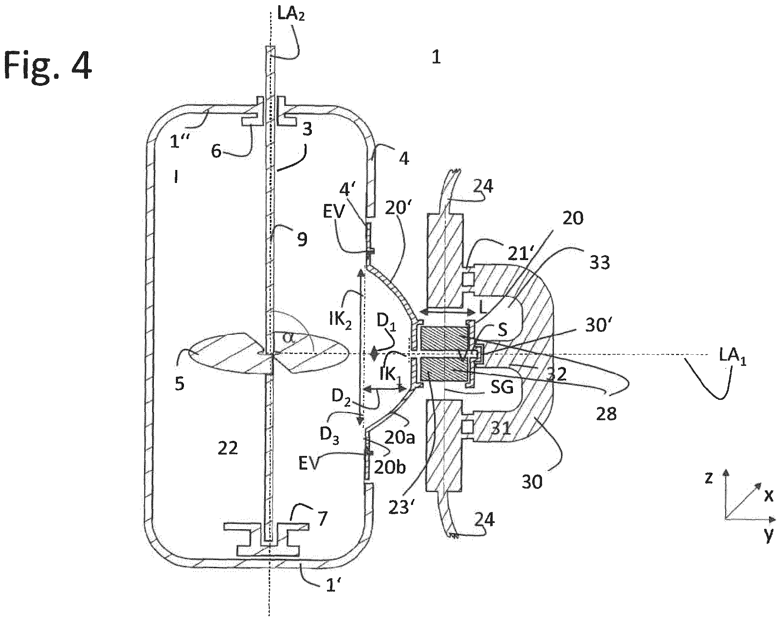

**Fig. 4** ist ein schematischer Querschnitt eines im Wesentlichen formstabil ausgebildeten Behälters 1, beispielsweise eines Bioreaktors. Es handelt sich hierbei um einen Behälter mit Rührwerk und angebrachter bzw. angeordneter Faserhalterung bzw. Sensor-Anbringungsvorrichtung 30, wobei die Fasern bzw. Lichtleiter 24 bezüglich des Wandungsvorsprungs 20 mittels der Sensor-Anbringungsvorrichtung 30 so ausgerichtet sind, dass Größen des Probenvolumens V durch die Sensorvorrichtung 21 aufgenommen werden können. Auch in dieser Ausführungsform kann ein Behälter 1, insbesondere ein Einweg-Behälter, beispielsweise aus einem Material umfassend PVC ausgebildet sein. Der Behälter 1 kann allerdings alternativ auch ein Mehrweg-Behälter, insbesondere eines Fermenters, beispielsweise ausgebildet aus einem Material umfassend Stahl und/oder PVC, sein. Der Bioreaktor umfasst ferner eine Rührvorrichtung bzw. ein Rührelement 3 mit Bestandteilen, die bereits für andere Ausführungsformen näher beschrieben wurden. Eine Rührwelle 9 erstreckt sich von der Behälterdecke 1" bis zum Behälterboden 1' im Wesentlichen entlang der Längsachse LA₂ des Behälters 1.

Der Behälter 1 umfasst ein Wandungsvorsprung-Element 20' welches eine Wandungsausbuchtung 20a mit einer Höhe D₂ und einer Tiefe D₃, sowie einen Wandungsvorsprung 20, welcher sich über eine Länge L nach außen erstreckt, aufweist. Die Höhe D₂ der Wandungsausbuchtung 20a erstreckt sich im Wesentlichen entlang einer imaginären Konturlinie IK₂ als Verlängerung der Behälterwandung 4 und zur Abgrenzung zur Wandungsausbuchtung 20a. Die Tiefe D₃ der Wandungsausbuchtung 20a erstreckt sich hingegen im Wesentlichen von der imaginären Konturlinie IK₂ als Verlängerung der Behälterwandung 4 bis zur imaginären Konturlinie IK₁ zur Definition des Probenvolumens V des Wandungsvorsprungs 20.

Der Wandungsvorsprung 20 umfasst ferner zwei Fenster 23, welche parallel im Wesentlichen mit dem Abstand der spaltartigen Probenschichtdicke D₁ zueinander angeordnet sind.

An dem Wandungsvorsprung 20 ist eine Sensor-Anbringungsvorrichtung 30 mittels eines Anbringungsvorrichtungsstegs 32 und eines Aufnahme-Elements 30' für eine Sensor-Anbringungsvorrichtung 30 gelagert bzw. angebracht. Der Anbringungsvorrichtungssteg 32 umfasst eine Führungsrinne, durch welche eine Schiene, die dem Aufnahme-Element 30' entspricht, des Wandungsvorsprungs 20 geführt werden kann. Wie zuvor genannt kann die Verbindungs- bzw. Lagerungsart jedoch auch eine Klemm-, eine Press- bzw. Druck-, eine Clip- und/oder eine Schraubverbindung umfassen.

In der Ausführungsform schließt die Längsachse LA₁ des Wandungsvorsprungs 20 einen im Wesentlichen rechten Winkel α mit der Längsachse LA₂ des Behälters 1 ein. Der Winkel α kann jedoch alternativ auch ein Winkel sein, der im Wesentlichen von 90° abweicht. Die imaginäre Konturlinie IK₁ zur Definition des Probenvolumens V verläuft im Wesentlichen parallel zu der imaginären Konturlinie IK₂ als Verlängerung der Behälterwandung 4 und als Abgrenzung zur Wandungsausbuchtung 20. Alternativ können die beiden imaginären Konturlinien IK₁, IK₂ auch nicht parallel zueinander verlaufen. Dies wäre beispielsweise der Fall, wenn der Winkel α keine 90° beträgt.

In **Fig. 4** ist ferner angedeutet, dass das Wandungsvorsprung-Element 20' über eine Elementverbindung EV bzw. eine Verbindung zwischen dem Abschnitt 20b zur Anbringung des Wandungsvorsprung-Element 20' an den Behälter 1 und der Behälterwandung 4 verbunden bzw. aneinander angebracht sind.

Das Wandungsvorsprung-Element 20' und insbesondere der Abschnitt 20b zur Anbringung des Wandungsvorsprung-Elements 20' an den Behälter 1 kann einen Teil einer Elementverbindung EV umfassen, wohingegen sich ein anderer dazu kompatibler Teil der Elementverbindung EV an der Behälterwandung 4 befinden kann. Diese Elementverbindung EV kann beispielsweise zwei ineinander schraubbare Gewinde umfassen.

Über die Elementverbindung EV kann das Wandungsvorsprung-Element 20' direkt oder indirekt an der Behälterwandung 4 angebracht sein oder angebracht werden. Die Darstellung der Ausführungsform der **Fig. 4** deutet einen Wandungsabschnitt 4' an, der wiederum auch an der Behälterwandung 4 angebracht ist und an dem die Elementverbindung EV positioniert ist, was einer indirekten Anbringung bzw. Verbindung entspricht. Der Wandungsabschnitt 4' zur Verbindung bzw. Anbringung zwischen Wandungsvorsprung-Element 20' und Behälterwandung 4 kann im Wesentlichen ein verstärkter Abschnitt umfassend einen Kunststoff und/oder ein Metall sein. Beispielsweise kann der Wandungsabschnitt 4' zur Verbindung bzw. Anbringung zwischen Wandungsvorsprung-Element 20' und Behälterwandung 4 an der Behälterwandung dicht angeklebt und/oder angeklemmt und/oder ver-bzw. angeschweißt sein. In diesem Fall kann man das Wandungsvorsprung-Element 20' und den Wandungsabschnitt 4' als zweiteiligen Port verstehen. Beispielsweise kann ein äußerer Ring, der mit der Behälterwandung 4 (auch zu verstehen als Bag-Wand) verschweißt wird, mit einem inneren Kern, der dem Wandungsvorsprung-Element 20' entspricht, dauerhaft und/oder zeitweise kombiniert bzw. verbunden bzw. aneinander angebracht werden. Die Verbindung Wandungsvorsprung-Elements 20' mit dem Ring bzw. dem Wandungsabschnitt 4' zur Verbindung kann einen Bajonettverschluss, Schrauben, Clips, Klemmen, Press- bzw. Druckverbindungen und/oder Klebeverbindungen umfassen. Insbesondere ist das Wandungsvorsprung-Element 20' von der Behälterwandung 4 auf- und abnehmbar.

Alternativ kann das Wandungsvorsprung-Element 20' auch direkt an der Behälterwandung 4 angebracht bzw. angeordnet werden, was einer direkten Verbindung bzw. Anbringung entspricht. In dem Fall handelt es sich entsprechend um einen einteiligen bzw. einstückigen Port.

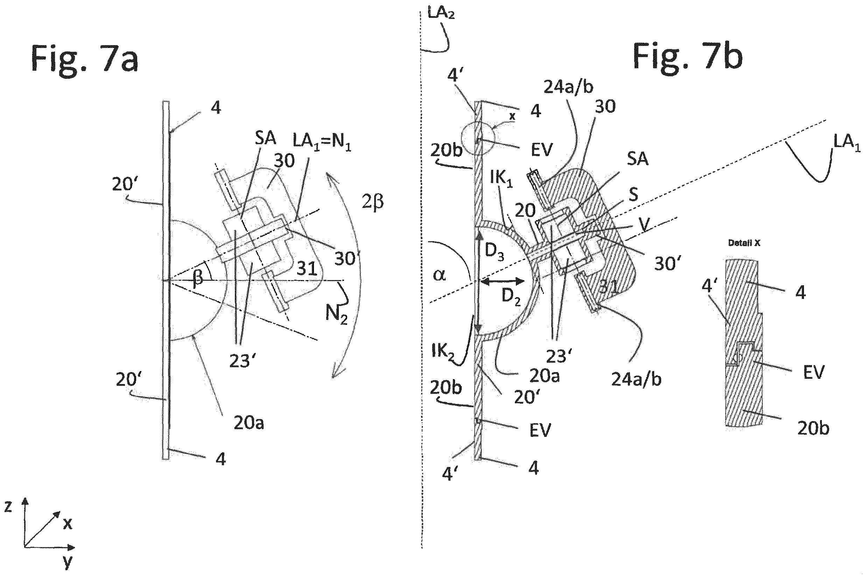

Die Art der Elementverbindung EV zwischen Wandungsvorsprung-Element 20' und Behälterwandung 4 wird nachfolgend in **Fig. 7b** noch detaillierter beschrieben.

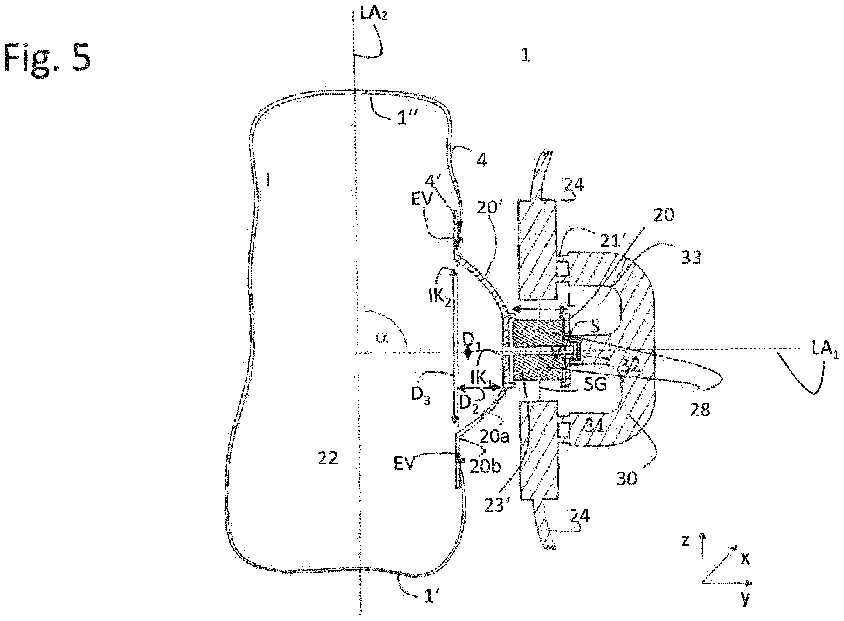

**Fig. 5** ist ein schematischer Querschnitt eines im Wesentlichen nicht formstabil ausgebildeten Behälters 1 beispielsweise eines Bioreaktors und/oder eines Beutels bzw. eines Bags ohne Rührwerk mit Port bzw. Wandungsvorsprung-Element 20'. Diese Ausführungsform betrifft insbesondere einen Einweg-Behälter bzw. einen Einweg-Beutel. Der Bioreaktor umfasst keine Rührvorrichtung bzw. kein Rührelement 3 mit den entsprechenden Bestandteilen. Zwischen der Behälterdecke 1" und dem Behälterboden 1' verläuft im Wesentlichen eine Längsachse LA₂ des Behälters 1. Der Behälter 1 kann insbesondere ein sogenannter "rocking motion bag" sein, welcher auf einem Rüttler bzw. einer Rüttel-Bank bzw. einem Laborschüttler bzw. einer schwankenden und/oder rüttelnden Unterlage angeordnet bzw. aufgelegt bzw. befestigt sein kann.

Andere Merkmale bezüglich des Wandungsvorsprung-Elements 20', sowie der Sensor-Anbringungsvorrichtung 30 entsprechen den entsprechenden Merkmalen der in **Fig. 4** gezeigten Ausführungsform.

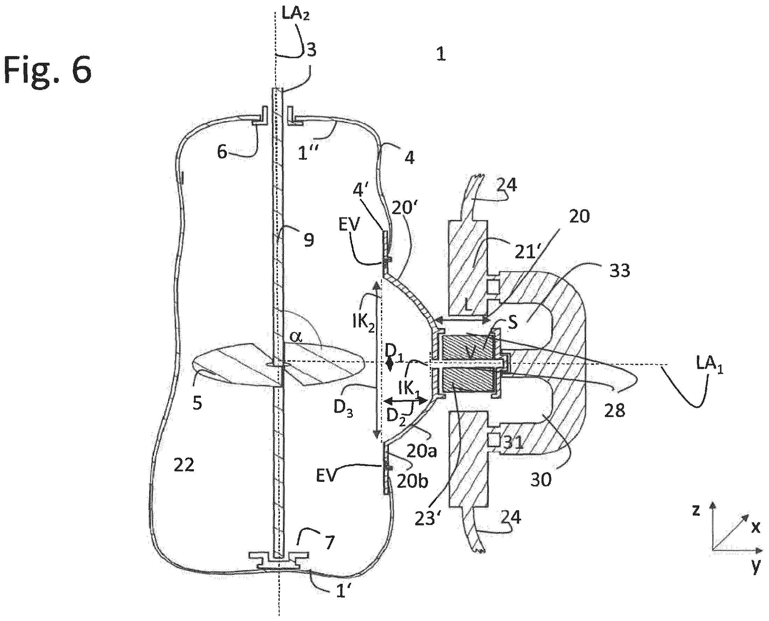

**Fig. 6** ist ein schematischer Querschnitt eines im Wesentlichen nicht formstabil ausgebildeten Behälters 1 eines Bioreaktors. Es handelt sich hierbei um einen Beutel bzw. Bag mit Rührwerk und angebrachter bzw. angeordneter Faserhalterung bzw. Sensor-Anbringungsvorrichtung 30, wobei die Fasern bzw. Lichtleiter 24 bezüglich des Wandungsvorsprungs 20 mittels der Sensor-Anbringungsvorrichtung 30 so ausgerichtet sind, dass Größen des Probenvolumens V durch die Sensorvorrichtung 21 aufgenommen werden können. Diese Ausführungsform betrifft insbesondere einen Einweg-Behälter bzw. einen Einweg-Beutel. Der Bioreaktor umfasst ferner eine Rührvorrichtung bzw. ein Rührelement 3 mit Bestandteilen, die bereits für andere Ausführungsformen näher beschrieben wurden. Eine Rührwelle 9 erstreckt sich von der Behälterdecke 1" bis zum Behälterboden 1' im Wesentlichen entlang der Längsachse LA₂ des Behälters 1.

Andere Merkmale bezüglich des Wandungsvorsprung-Elements 20', sowie der Sensor-Anbringungsvorrichtung 30 entsprechen den entsprechenden Merkmalen der in **Fig. 4** und **Fig. 5** gezeigten Ausführungsformen.

**Fig. 7a** ist eine schematische Seitenansicht (von außen) einer Ausführungsform eines Wandungsvorsprung-Elements 20' an einer Behälterwandung 4 eines hier nur angeschnitten dargestellten Behälters 1. **Fig. 7b** entspricht einem Anschnitt des in **Fig. 7a** gezeigten Gegenstandes. Ferner zeigt **Fig. 7b** eine Detailansicht eines Abschnitts 20b zur Anbringung des Wandungsvorsprung-Elements 20' an den Behälter 1 bzw. an die Behälterwandung 4 und eine Verbindung bzw. Elementverbindung EV zwischen dem Wandungsvorsprung-Element 20' und der Behälterwandung 4.

Die **Fig. 7a** und **7b** sind schematische Darstellungen einer Ausführungsform eines Wandungsvorsprung-Elements 20', welches an einer Behälterwandung 4 eines Behälters 1 angebracht ist. Genauer ist das Wandungsvorsprung-Element 20' mittels einer in der Detailansicht der **Fig. 7b** gezeigten Elementverbindung EV an dem Behälter 1 angebracht. Diese Elementverbindung EV kann dazu ausgelegt sein, das Wandungsvorsprung-Element 20' dauerhaft bzw. über eine längere Zeit mit dem Behälter 1 zu verbinden. Die Elementverbindung EV kann beispielsweise einen Teil oder Abschnitt umfassen, der sich an dem Wandungsvorsprung-Element 20' befindet und einen Teil oder Abschnitt, der sich an der Behälterwandung 4 befindet.

Wie in der Detailansicht des Bereiches x gezeigt ist, kann die Verbindung EV derart ausgestaltet sein, dass die Behälterwandung 4 eine gezahnte bzw. gezackte Struktur bzw. Kontur über die Wandungsdicke bzw. die Dicke der Behälterwandung 4 an einem Abschnitt aufweist, wobei die gezahnte bzw. gezackte Struktur bzw. Kontur insbesondere passgenau in eine entsprechende komplementäre gezahnte bzw. gezackte Struktur bzw. Kontur über die Wandungsdicke des Wandungsvorsprung-Elements 20' greift. In dem Fall können zumindest die aufeinandertreffenden bzw. einander berührenden Flächen bzw. Oberflächen der verzahnten Strukturen des Wandungsvorsprung-Elements 20' und der Behälterwandung 4 verklebt und/oder verschweißt und/oder versiegelt sein. Beispielsweise kann ein Klebstoff und/oder ein Harz und/oder ein zwei Komponenten Polymergemisch oder andere Mittel zur dauerhaften oder zumindest zeitweisen Klebverbindung verwendet werden.

Alternativ kann die Verbindung EV dazu ausgelegt sein, dass das Wandungsvorsprung-Element 20' in unkomplizierter Weise von der Behälterwandung 4 auf- und abgenommen werden kann. In dem Fall kann beispielsweise ein Teflon- oder Silikonfett und/oder ein anderer inerter bzw. reaktionsträger Schmierstoff zur Dichtung verwendet werden. Zusätzlich oder alternativ kann ein Teflonband bzw. eine Teflonfolie zur Abdichtung des Innenraumes 22 des Behälters 1 zwischen die aneinander angrenzenden Flächen der komplementären verzahnten Strukturen beider Elemente geklemmt bzw. positioniert bzw. gelegt werden.

Wie zuvor genannt kann die Verbindungs- bzw. Lagerungsart der Elementverbindung EV jedoch auch eine Klemm- und/oder eine Press- bzw. Druck- und/oder eine Clip- und/oder eine Schraubverbindung umfassen, wobei ein Verbindungsstück an dem Abschnitt 20b zur Anbringung des Wandungsvorsprung-Elements 20' an den Behälter 1 bzw. an die Behälterwandung 4 positioniert ist und ein komplementäres Verbindungsgegenstück an der Behälterwandung 4 positioniert ist. Es kann beispielsweise auch möglich sein, dass das gesamte Wandungsvorsprung-Element 20' mittels eines Gewindes entlang des Umfangs des Wandungsvorsprung-Elements 20' in ein Gewindegegenstück der Behälterwandung 4 eingeschraubt werden kann.

Das Wandungsvorsprung-Element 20' umfasst eine im Wesentlichen kugelförmige Wandungsausbuchtung 20a. Insbesondere hat die Wandungsausbuchtung 20a im Wesentlichen eine Form einer Halbkugel, die durch eine Tiefe D₂, eine Höhe D₃ und eine Breite D₄ (hier nicht gezeigt) charakterisiert ist. Die Tiefe D₂ entspricht insbesondere dem Radius der Kugel und die Höhe D₃ sowie die Breite D₄ entsprechen insbesondere dem Durchmesser der Kugel. Dementsprechend hat das Volumen, welches durch die Wandungsausbuchtung 20a und die beiden imaginären Konturlinien bzw. -Flächen eingegrenzt bzw. definiert bzw. umgeben wird, insbesondere und im Wesentlichen das Volumen einer halben Kugel mit dem Radius, der der Tiefe D₂ entspricht. Die Wandungsausbuchtung 20a kann alternativ auch andere Formen, beispielsweise einen Anschnitt eines Ellipsoids, oder einen Kugelanschnitt, der nicht einer Halbkugel entspricht, umfassen.

Das Wandungsvorsprung-Element 20' umfasst ferner einen Wandungsvorsprung 20, welcher sich entlang der Längsachse LA₁ des Wandungsvorsprungs 20 nach außen hin erstreckt. Anders als in den anderen Ausführungsformen ist dabei die Längsachse LA₁ in eine Richtung "nach oben" zur Behälterwandung 4 geneigt. Dies bedeutet, dass der Winkel α, der von der Längsachse LA₁ bzw. deren linearer Verlängerung und der Längsachse LA₂ des Behälters 1 eingeschlossen wird, im Wesentlichen kleiner als 90° ist. Beispielsweise kann der Winkel α in einem Bereich zwischen etwa 20° und etwa 80°, insbesondere zwischen etwa 30° und etwa 70° und bevorzugt zwischen etwa 40° und etwa 60° liegen. Besonders bevorzugt wäre der Fall, in dem der Winkel α einen Wert von etwa 45° einnimmt. Der Winkel α liegt dabei in der Ebene, welche in dem angedeuteten Koordinatensystem durch die y- und die z-Achse dargestellt ist. Alternativ ist es auch möglich, dass die Längsachse LA₁ in eine Richtung "nach unten" zur Behälterwandung 4 geneigt ist. Dabei würde der Winkel α in einem Bereich zwischen etwa 160° und etwa 100°, insbesondere zwischen etwa 150° und etwa 110° und bevorzugt zwischen etwa 140° und etwa 120° liegen. Besonders bevorzugt wäre der Fall, in dem der Winkel α einen Wert von etwa 135° einnimmt.

In der Ausführungsform gemäß der **Fig. 7a** und **7b** verläuft die Längsachse LA₂ im Wesentlichen parallel zu der Behälterwandung 4 und der imaginären Konturlinie IK₂ (als Verlängerung der Behälterwandung 4 entlang der z-Achse) entlang der Richtung, die in dem angedeuteten Koordinatensystem der z-Achse entspricht.

Die imaginären Konturlinie IK₂ (als Verlängerung der Behälterwandung 4 entlang der z-Achse) weist eine Normale N₂ auf, welche eine Achse ist, die innerhalb der y-z-Ebenen (entsprechend des angedeuteten Koordinatensystems) senkrecht zur imaginären Konturlinie IK₂ ausgerichtet ist. Wenn der Winkel α, der von der Längsachse LA₁ bzw. deren linearer Verlängerung und der Längsachse LA₂ des Behälters 1 eingeschlossen wird, 90° beträgt, dann liegt die Normale N₂ der imaginären Konturlinie IK₂ auf oder mindestens parallel zu einer Normalen N₁ der imaginären Konturlinie IK₁ zur Definition des Probenvolumens V. Die Normale N₁ der imaginären Konturlinie IK₁ zur Definition des Probenvolumens V entspricht in der gezeigten Ausführungsform der Längsachse LA₁ des Wandungsvorsprungs 20. Weicht der Winkel α von 90° ab, so schließen die beiden Normalen N₁ und N₂ den Winkel β ein, der einem Wert von (90°- α) entspricht.

Der Wandungsvorsprung 20 kann sich in einer Richtung, die Senkrecht zu der y-z-Ebene liegt bzw. verläuft, zumindest abschnittsweise entlang des Umfangs der Wandungsausbuchtung 20a erstrecken. Insbesondere kann sich der Wandungsvorsprung in einer Richtung, die Senkrecht zu der y-z-Ebene liegt bzw. verläuft, vollständig entlang des Umfangs bzw. "quer" über den Umfang der Wandungsausbuchtung 20a erstrecken. Die Länge L des Wandungsvorsprungs 20, die sich über die Längsachse LA₁ erstreckt, kann insbesondere konstant sein. Alternativ kann die Länge L des Wandungsvorsprungs 20 an unterschiedlichen Positionen entlang des Umfangs der Wandungsausbuchtung 20a auch variieren.

An dem Wandungsvorsprung 20 ist eine Sensor-Anbringungsvorrichtung 30 derart angeordnet bzw. gelagert bzw. angebracht, dass eine Strahlengangachse SA eines einfallenden Lichtes senkrecht zu der Fensterfläche eines Fensters 23' insbesondere zweier Fenster 23' verläuft. Demnach ist die Sensor-Anbringungsvorrichtung 30 in geneigter Weise an dem Wandungsvorsprung 20 gelagert.

Die Sensor-Anbringungsvorrichtung 30 umfasst in dieser Ausführungsform keinen Anbringungsvorrichtungssteg 32, sondern eine Führungsrinne bzw. eine schmale Rinne bzw. Nut, durch die ein Aufnahme-Element 30' des Wandungsvorsprungs 20, insbesondere ein länglicher Steg oder einen Vorsprung, geführt werden kann. Derart kann die Sensor-Anbringungsvorrichtung 30 im Wesentlichen an dem Wandungsvorsprung 20, insbesondere mit einem kleinen Lagerspiel, gesichert bzw. gelagert bzw. befestigt werden. Die Position der Sensor-Anbringungsvorrichtung 30 gegenüber den Elementen des Wandungsvorsprungs 20 ist dabei reversibel nach jedem Abnehmen und Anbringen einnehmbar. Insbesondere der Strahlengang und die Strahlengangachse beispielsweise bezüglich der Fenster 23' kann reversibel eingenommen werden. Beispielsweise kann eine Position reversibel eingenommen werden, wenn ein magnetisches Ausrichtungssystem bereitgestellt wird. Ferner kann auch eine Präzisionslagerung eine präzise Ausrichtung gewährleisten.

Das Wandungsvorsprung-Element 20', auch Port bezeichnet, kann ein- oder mehrteilig sein. Falls das Wandungsvorsprung-Element 20' einteilig ist, wie in der **Fig. 7b****,** insbesondere in dem Detailausschnitt x angedeutet ist, so ist das Wandungsvorsprung-Element 20' direkt an der Behälterwandung 4 des Behälters 1 angeordnet. Falls das Wandungsvorsprung-Element 20' zweiteilig ist (nicht gezeigt), so ist das Wandungsvorsprung-Element 20' indirekt an der Behälterwandung 4 des Behälters 1 angeordnet und zwar mittels eines Wandungsabschnitts 4' zur Verbindung zwischen Wandungsvorsprung-Element 20' und Behälterwandung 4. Der Wandungsabschnitt 4' zur Verbindung kann bevorzugt als ein Bestandteil des Wandungsvorsprung-Elements 20' betrachtet werden, weshalb das Wandungsvorsprung-Element 20' dann als zweiteilig betrachtet wird. Andererseits kann der Wandungsabschnitt 4' zur Verbindung alternativ auch als ein Bestandteil der Behälterwandung 4 betrachtet werden.

Neben der Längsachse LA₂ des Behälters 1 kann auch die Kontur der Behälterwandung 4, welche unmittelbar an dem Wandungsvorsprung-Element 20' angrenzt, als Referenzlinie für die Neigung der Längsachse LA₁ des Wandungsvorsprungs 20 dienen. Dabei ersetzt die Kontur der Behälterwandung 4 die Längsachse LA₂ des Behälters 1 derart, dass der Winkel α zwischen Konturlinie der Behälterwandung 4 und Längsachse LA₁ des Wandungsvorsprungs 20 eingeschlossen wird.

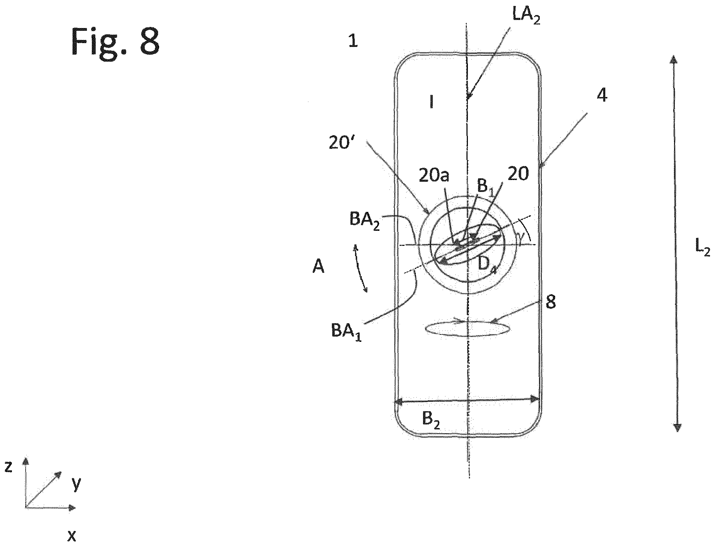

Die **Fig. 8** ist eine schematische Frontalansicht eines Bioreaktors und dessen Behälters 1 mit bezüglich einer Breitenachse BA₂ des Behälters 1 geneigtem Wandungsvorsprung 20 und Wandungsausbuchtung 20a gemäß einer Ausführungsform.

Der Behälter 1 gemäß einer Ausführungsform ist in **Fig. 8** nun in Frontalansicht dargestellt, sodass der Anschnitt entsprechend in der x-z-Ebene des angedeuteten Koordinatensystems liegt. Der Behälter 1 weist eine Breite B₂, sowie eine Länge L₂ auf.

Der Bioreaktor umfasst den Behälter 1 sowie ein Rührelement. Eine Richtung einer möglichen Strömung eines Mediums 8 ist mittels eines Feils in der Darstellung angedeutet.

Ferner weist der Behälter 1 eine Längsachse LA₂ auf sowie eine Breitenachse BA₂, entlang der eine Breite B₂ des Behälters gemessen werden kann, auf. Der Behälter 1 umfasst zudem ein Wandungsvorsprung-Element 20' umfassend einen Wandungsvorsprung 20 und eine Wandungsausbuchtung 20a, welche die Form eines angeschnittenen Ellipsoids aufweist. Der Wandungsvorsprung 20 weist im Wesentlichen eine Breite B₁, sowie eine Breitenachse BA₁ auf, entlang derer sich der Wandungsvorsprung 20 erstreckt. In der Ausführungsform ist der Wandungsvorsprung 20 derart geneigt, dass die Breitenachse BA₁ einen Winkel γ mit der Breitenachse BA₂ des Behälters 1 einschließt, der im Wesentlichen einen Wert ungleich null beträgt.

Das Wandungsvorsprung-Element 20' kann in dieser Ausführungsform ein- oder zweiteilig sein. In der **Fig. 8** deutet ein Pfeil auf der Innenseite I des Behälters 1 eine mögliche Drehrichtung des Rührwerkes, durch die das Medium 8 in eine Drehbewegung versetzt wird. Ausgelöst durch die Drehbewegung kann eine Aufwärtsbewegung des Mediums im Wesentlichen entlang der Wand stattfinden.

Ein Doppelpfeil auf der Außenseite A deutet einen Strömungswinkel an, unter dem das Medium 8 insbesondere ohne große Verluste durch den Schlitz strömt.

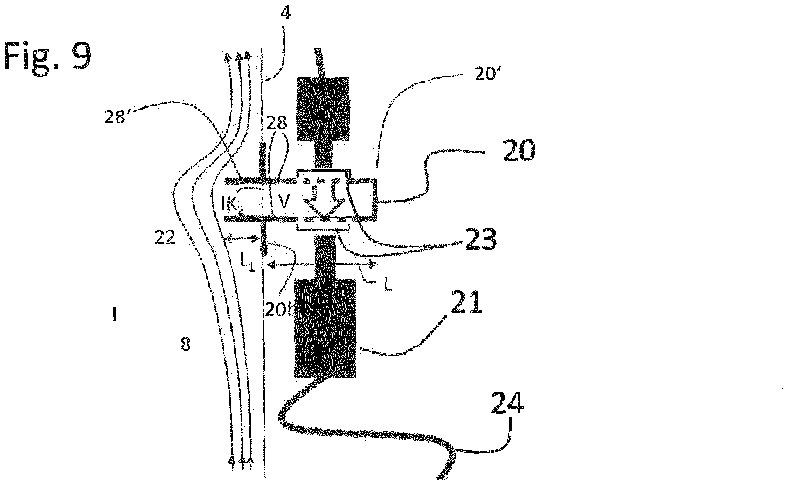

Die **Fig. 9** ist eine schematische Seitenansicht eines Ausschnitts eines Behälters 1 mit Wandungsvorsprung 20, dessen Vorsprungs-Wände 28 jeweils eine Verlängerung 28' umfassen, welche auf der Innenseite des Behälters 1 in das Innenvolumen bzw. den Behälter-Innenraum jenseits der imaginären Konturlinie IK₂ zur Definition des Probenvolumens V hineinragen bzw. in einem befüllten Zustand in das Medium 8 hineinragen. Die Verlängerung 28' weist eine Länge L₁ auf, die variieren kann. Beispielsweise kann die Länge L₁ der Verlängerung 28' etwa 1 cm bis 20 cm betragen, insbesondere kann die Länge L₁ der Verlängerung 28' etwa 2 cm bis 10 cm betragen und bevorzugt kann die Länge L₁ der Verlängerung 28' etwa 3 cm bis 8 cm betragen. Beispielsweise kann die Länge L₁ der Verlängerung 28' etwa 1/2 bis etwa 1/20 der Länge L des Wandungsvorsprungs 20 betragen. Insbesondere kann die Länge L₁ der Verlängerung 28' etwa 1/3 bis etwa 1/10 der Länge L des Wandungsvorsprungs 20 betragen. Bevorzugt kann die Länge L₁ der Verlängerung 28' etwa 1/4 bis etwa 1/8 der Länge L des Wandungsvorsprungs 20 betragen.

Der Wandungsvorsprung 20, dessen Vorsprungs-Wände 28 jeweils eine Verlängerung 28' umfassen, ist Bestandteil eines Wandungsvorsprung-Elementes 20' bzw. eines Ports. Das Wandungsvorsprung-Element 20' umfasst einen Abschnitt 20b zur Anbringung des Wandungsvorsprung-Elements 20' an dem Behälter 1. Durch die Verlängerung 28' der Vorsprungs-Wände 28 lässt sich der Fluss des Mediums 8, insbesondere im Wesentlichen entlang der inneren Seite der Behälterwandung 4 beeinflussen. In anderen Worten kann die Wandströmung durch die jeweilige Verlängerung 28' der Vorsprungs-Wände 28 abgebremst und/oder abgelenkt werden. Dadurch können beispielsweise auch turbulente Strömungen an den Kanten der Verlängerungen 28' entstehen. In der **Fig. 9** ist ein mögliches Strömungsprofil des Mediums 8 durch drei Linienverläufe mit Pfeilen angedeutet. Die Verläufe deuten zunächst eine abgelenkte laminare Strömung des Mediums 8 entlang der Behälterwandung 4 an. Es kann jedoch wie gesagt auch zu turbulenten Strömungen, insbesondere nahe der Verlängerungen 28' kommen.

Insbesondere kann vermieden werden, dass eine zu starke Strömung des Mediums 8 in dem Probenvolumen V bzw. in dem Spalt bzw. spaltförmigen Probenvolumen entsteht. In anderen Worten kann durch die jeweilige Verlängerung 28' ein Volumen des Mediums 8 pro Zeiteinheit, welches durch das Probenvolumen zumindest abschnittsweise fließt, verringert bzw. reduziert werden. Derart kann insbesondere nahezu ein Strömungsstillstand innerhalb des Probenvolumens V erreicht werden. Zumindest kann ein Strom eines Mediums durch das Probenvolumen V stark verlangsamt werden.

Die Ausführungsform des Wandungsvorsprungs 20 umfassend der Verlängerung 28' des Wandungsvorsprungs 20 kann auch so verstanden werden, dass der Wandungsvorsprung an sich in den Innenraum jenseits der imaginären Konturlinie IK₂ zur Definition des Probenvolumens V hineinragt.

Für den Fall, dass das Wandungsvorsprung-Element 20' eine Wandungsausbuchtung 20a umfasst, kann es auch sein, dass insbesondere ein Rand der Wandungsausbuchtung 20a eine Verlängerung 28' umfasst, welche auf der Innenseite des Behälters 1 in den Behälter-Innenraum jenseits der imaginären Konturlinie IK₂ zur Definition des Probenvolumens V hineinragt bzw. in einem befüllten Zustand in das Medium 8 hineinragt.

Die jeweilige Verlängerung 28' der Vorsprungs-Wände 28 kann die Form aufweisen, die durch die Vorsprungs-Wände 28 vorgegeben ist. Alternativ kann die Verlängerung 28' auch von einer Form, welche durch eine Vorsprungs-Wand 28 vorgegeben ist, abweichen. Beispielsweise können die jeweiligen Verlängerung 28' der Vorsprungs-Wände 28 auch zu einander und/oder gegeneinander gerichtet sein, sodass sie gegenüber den Vorsprungs-Wänden 28 abknicken bzw. geneigt sind. Derart kann beispielsweise ein Strom des Mediums 8 besonders gut beeinflusst, beispielsweise in der Nähe des Probenvolumens V abgebremst werden. Der Bereich im Probenvolumen V ist deshalb "beruhigt" gegenüber anderen Bereichen im Behälter-Innenraum 22 des Behälters 1.

Insbesondere lassen sich die Merkmale bezüglich der Orientierung des Wandungsvorsprungs 20 beispielsweise aus den **Figuren 7a, 7b** und **8** kombinieren. Allgemein lassen sich explizit alle Merkmale verschiedener Ausführungsformen kombinieren, sofern sie sich nicht gegenseitig ausschließen.

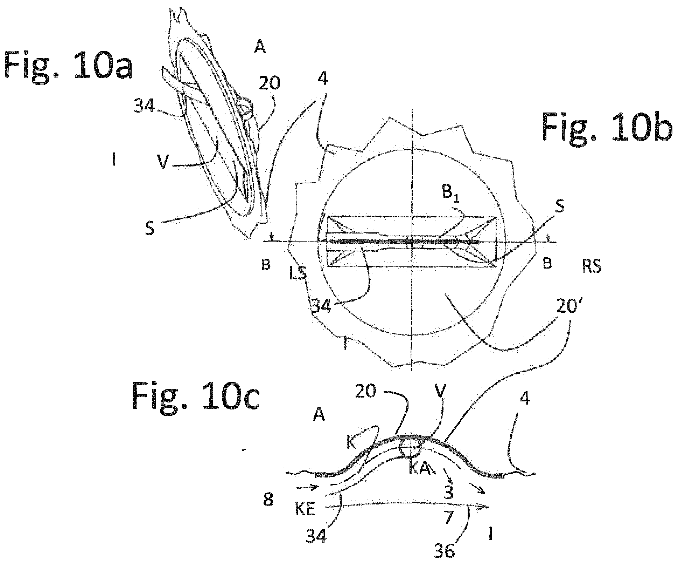

**Fig. 10a** ist eine perspektivische Seitenansicht eines Wandungsvorsprung-Elements 20' gemäß einer besonderen Ausführungsform. Die Seitenansicht betrifft im Wesentlichen eine Ansicht von der Innenseite I des Behälters 1 auf das spaltartige Volumen S der Wandungsausbuchtung 20. An dem Spalt S der Wandungsausbuchtung 20 ist ein Leitblech bzw. ein Leitabschnitt 34 angeordnet, welcher dazu ausgelegt ist, ein Medium, insbesondere eine Flüssigkeit in einen Kanal K bzw. ein kanalartiges Volumen K innerhalb des Spaltes S zu führen. Mit anderen Worten bildet der Leitabschnitt 34 zumindest teilweise einen Kanal, welcher dazu ausgelegt ist, ein Medium im Wesentlichen durch den Spalt S und insbesondere das Probenvolumen V zu führen.

**Fig. 10b** ist die Frontalansicht des Wandungsvorsprung-Elements 20' gemäß der Ausführungsform der **Fig. 10a** von der Innenseite eines Behälters 1. Auf der linken Seite des Spalts S ist das Leitblech bzw. der Leitabschnitt 34 angeordnet. Das Leitblech bzw. der Leitabschnitt 34 schließt einen Teil des spaltförmigen Volumens S im Wesentlichen ein und erstreckt sich entlang der Breitenachse B₁ des Spalts S von der linken Seite LS bis etwa zur Mitte des Spalts S. Alternativ kann sich das Leitblech bzw. der Leitabschnitt 34 auch nicht ganz bis zur Mitte entlang der Breitenachse B₁ des Spalts S von rechts RS und/oder von links LS erstrecken.

**Fig. 10c** ist die Ansicht eines Schnittes durch das Wandungsvorsprung-Element 20' entlang der Schnittlinie B-B aus **Fig. 10b****,** welche im Wesentlichen der Breitenachse B₁ des Spalts S entspricht. Der Leitabschnitt 34 erstreckt sich entlang des Spalts S von der linken Seite LS bis etwa in die Mitte des Spalts S, in Richtung der rechten Seite RS. Ferner ist ein Fluss eines Mediums 8 entlang einer Drehrichtung 36 und entlang einer Flussrichtung 37 durch den Kanal K und aus dem Kanal K heraus mittels Pfeilen angedeutet. Das Medium 8 fließt, beispielsweise angetrieben durch ein Rührelement 3 im Wesentlichen mit dem Uhrzeigersinn durch den Behälter 1. Ein Teil des Mediums 8 wird dabei in einen Kanaleingang KE durch den Leitabschnitt 34 in den Kanal K und in Richtung des Kanalausgangs KA geleitet. Der Kanal K verläuft im Wesentlichen so, dass er das Medium 8 durch das Probenvolumen V und insbesondere durch den Abschnitt zwischen zwei Fenstern 23' des Wandungsvorsprungs 20 führt. Auf diese Weise kann in das Probenvolumen V stets neues Medium 8 gespült werden. Während einer Messung kann die Strömung des Mediums 8 angehalten werden, um eine stabile Messung zu gewährleisten.

In der Ausführungsform der **Fig. 10a** - **10c** endet der Kanal K im Probenvolumen V bzw. in dem Spalt S etwa auf der Mitte der Breitenachse B₁ des Spalts S, sodass das Medium 8, welches durch den Kanal K geführt wird, wieder aus dem Kanal K heraustritt und möglicherweise turbulente Strömungen in dem Spalt S, im Wesentlichen außerhalb des Kanals K, verursacht.

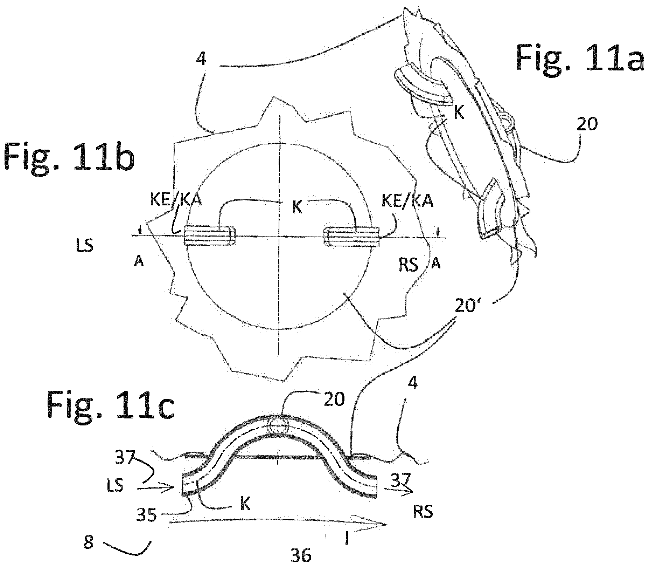

**Fig. 11a** ist auch eine perspektivische Seitenansicht eines Wandungsvorsprung-Elements 20' gemäß einer weiteren besonderen Ausführungsform. Die Seitenansicht betrifft im Wesentlichen eine Ansicht von der Innenseite I des Behälters 1 auf das spaltartige Volumen S der Wandungsausbuchtung 20. An dem Spalt S der Wandungsausbuchtung 20 ist eine Kanalführung 35 angeordnet, welche dazu ausgelegt ist, ein Medium 8, insbesondere eine Flüssigkeit in einen Kanal K bzw. ein kanalartiges Volumen K innerhalb des Spaltes S zu führen. Im Gegensatz zur Ausführungsform der **Fig. 10a - 10c** erstreckt sich der Kanal K gemäß dieser Ausführungsform im Wesentlichen über die gesamte Breite des Wandungsvorsprungs 20 bzw. des Spalts, entlang der Breitenachse B₁ des Spalts S. In anderen Worten umfasst das Probenvolumen V und/oder der Spalt S einen Kanal K, der jeweils im Wesentlichen auf beiden Seiten entlang der Breitenachse B₁ eine Öffnung aufweist.

Der Kanal K ist zwischen den Öffnungen im Wesentlichen durch die Kanalführung 35 eingeschlossen.

**Fig. 11b** ist die Frontalansicht des Wandungsvorsprung-Elements 20' gemäß der Ausführungsform der **Fig. 11a** von der Innenseite eines Behälters 1. Auf beiden Seiten des Wandungsvorsprung-Elements 20' ist ein Kanal-Ein- bzw. -Ausgang KE, KA des Kanals K angeordnet. Die Kanalführung 35 schließt einen Teil des spaltförmigen Volumens S im Wesentlichen ein und erstreckt sich entlang der Breitenachse B₁ des Spalts S von der linken Seite LS bis zur rechten Seite RS des Spalts S.

**Fig. 11c** ist die Ansicht eines Schnittes durch das Wandungsvorsprung-Element 20' entlang der Schnittlinie A-A aus **Fig. 11b**, welche im Wesentlichen der Breitenachse B₁ des Spalts S entspricht bzw. sich entlang der Breitenachse B₁ erstreckt. Die Kanalführung 35 erstreckt sich entlang des Spalts S bzw. entlang der Breitenachse B₁ des Spalts S von der linken Seite LS auf die rechte Seite RS des Spalts S. Ferner ist ein Fluss eines Mediums 8 entlang einer Drehrichtung 36 und entlang einer Flussrichtung 37 durch den Kanal K und aus dem Kanal K heraus mittels Pfeilen angedeutet. Das Medium 8 fließt auch in diesem Fall, beispielsweise angetrieben durch ein Rührelement 3 im Wesentlichen mit dem Uhrzeigersinn durch den Behälter 1. Ein Teil des Mediums 8 wird dabei in den Kanaleingang KE, der hier beispielsweise auf der linken Seite LS liegt durch die Kanalführung 35 in den Kanal K und in Richtung des Kanalausgangs KA, hier auf der rechten Seite RS geleitet. Wird die Drehrichtung 36 umgekehrt, so würde der Kanaleingang KE auf der rechten RS und der Kanalausgang KA auf der linken Seite LS liegen. Der Kanal K verläuft im Wesentlichen so, dass er das Medium 8 durch das Probenvolumen V und insbesondere durch den Abschnitt zwischen zwei Fenstern 23' des Wandungsvorsprungs 20 führt. Auf diese Weise kann in das Probenvolumen V stets neues Medium 8 gespült werden.

Der Kanal K kann im Allgemeinen einen runden oder einen eckigen Querschnitt aufweisen, sich aufweiten oder verengen in eine Richtung.

Nachfolgend werden allgemeine Dimensionierungen genannt, die für verschiedene Ausführungsformen zutreffen können und/oder kombiniert werden können. Die Angaben sind allgemein, beispielhaft und nicht einschränkend.

Die Tiefe D₂ der Wandungsausbuchtung 20a kann im Allgemeinen beispielsweise Werte zwischen etwa 5 mm bis etwa 30 cm, insbesondere zwischen etwa 2 cm und etwa 10 cm und bevorzugt zwischen etwa 3 cm und etwa 5 cm annehmen. Die Höhe D₃ der Wandungsausbuchtung 20a kann im Allgemeinen beispielsweise Werte zwischen etwa 1 cm bis etwa 100 cm, insbesondere zwischen etwa 2 cm und etwa 20 cm und bevorzugt zwischen etwa 3 cm und etwa 10 cm annehmen. Die Breite D₄ der Wandungsausbuchtung 20a kann im Allgemeinen beispielsweise Werte zwischen etwa 1 cm bis etwa 100 cm, insbesondere zwischen etwa 2 cm und etwa 20 cm und bevorzugt zwischen etwa 3 cm und etwa 10 cm annehmen. Die Probenschichtdicke D₁ bzw. der innere Abstand zwischen den zwei im Wesentlichen parallelen Vorsprungs-Wänden 28 kann im Allgemeinen beispielsweise zwischen etwa 20 µm und etwa 10 cm, insbesondere zwischen etwa 500 µm und etwa 2 cm, bevorzugt zwischen etwa 1 mm und etwa 1 cm dick sein. Die Vorsprungs-Länge L kann im Allgemeinen beispielsweise zwischen etwa 5 mm und etwa 20 cm, insbesondere zwischen etwa 1 cm und etwa 10 cm und bevorzugt zwischen etwa 3 cm und etwa 8 cm lang sein. Beispielsweise ist die Vorsprungs-Länge L mindestens etwa doppelt, insbesondere mindestens etwa fünf Mal und bevorzugt mindestens etwa acht Mal so lang bzw. groß wie die Probenschichtdicke D₁.

Insbesondere kann das Verhältnis aus Höhe zu Breite, D₃/D₄ einem Wert von etwa 1 entsprechen. In diesem Fall wäre die Wandungsausbuchtung 20a beispielsweise im Wesentlichen kreisrund von einer Frontalansicht aus betrachtet. Es kann auch sein, dass das Verhältnis aus Höhe zu Breite, *D₃*/*D₄* Werte zwischen etwa 0,2 und etwa 1, insbesondere zwischen etwa 0,33 und etwa 0,8 und bevorzugt zwischen etwa 0,5 und etwa 0,75 annimmt. Ferner kann auch das umgekehrte Verhältnis aus Breite zu Höhe, D₄/D₃ Werte zwischen etwa 0,2 und etwa 1, insbesondere zwischen etwa 0,33 und etwa 0,8 und bevorzugt zwischen etwa 0,5 und etwa 0,75 annehmen. Das Verhältnis aus Tiefe zu Höhe, D₂/D₃ kann beispielsweise einem Wert von etwa 0,5 annehmen. In diesem Fall könnte die Wandungsausbuchtung 20a beispielsweise kreisrund von der Behälter-Innenseite I zur Außenseite A hin hervortreten. Es kann auch sein, dass das Verhältnis aus Tiefe zu Höhe, D₂/D₃ Werte zwischen etwa 0,05 und etwa 0,5, insbesondere zwischen etwa 0,07 und etwa 0,4 und bevorzugt zwischen etwa 0,1 und etwa 0,3 annimmt. Ferner kann auch das Verhältnis aus Tiefe zu Höhe, D₂/D₃ beispielsweise einen Wert annehmen, welcher größer als etwa 0,5 ist. In diesem Fall wäre die Wandungsausbuchtung 20a besonders exponiert und käme der Form eines Spalts nahe. Es kann sein, dass das Verhältnis aus Tiefe zu Vorsprungs-Länge, D₂/L Werte zwischen etwa 0,1 und etwa 1, insbesondere zwischen etwa 0,3 und etwa 0,9 und bevorzugt zwischen etwa 0,33 und etwa 0,75 annimmt. Ferner kann auch das Verhältnis aus Tiefe zu Vorsprungs-Länge, D₂/L beispielsweise einen Wert annehmen, welcher größer als etwa 1 ist und insbesondere zwischen etwa 1,2 und etwa 1,5 liegt.

Das Probenvolumen V welches zumindest teilweise von dem Wandungsvorsprung 20 umgeben bzw. eingeschlossen ist, kann beispielsweise Werte zwischen etwa 100 µl und etwa 500 ml, insbesondere zwischen etwa 200 µl und etwa 200 ml und bevorzugt zwischen etwa 300 µl und etwa 100 ml annehmen. Das gesamte Innenvolumen bzw. der Behälter-Innenraum 22 eines Behälters 1 einschließlich dem Probenvolumen kann beispielsweise Werte zwischen etwa 500 ml und etwa 2000 l, insbesondere zwischen etwa 1 l und etwa 1000 l und bevorzugt zwischen etwa 2 l und etwa 500 l annehmen. Das gesamte Innenvolumen bzw. der Behälter-Innenraum 22 kann beispielsweise etwa 10 bis etwa 25*10⁷, insbesondere etwa 1*10⁶ bis etwa 1,5*10⁷ und bevorzugt etwa 15*10⁶ bis etwa 1*10⁷ Mal so groß sein wie das Probenvolumen V.

Es sei insbesondere angemerkt, dass eine Längsachse LA₂ des Behälters 1 auch durch die Breitenachse BA₂ des Behälters 1 ersetzt werden kann, sodass beispielsweise bei der Definition des Winkels α statt der Längsachse LA₂ des Behälters 1 die Breitenachse BA₂ bzw. eine Breitenachse des Behälters 1 herangezogen wird. Dies ist beispielsweise der Fall, wenn der Behälter 1 ein Beutel ist, der auf einer Fläche aufliegt und dessen Längsachse LA₂ im Wesentlichen parallel zu der Fläche verläuft, auf der der Beutel liegt. Dies wäre ein ähnlicher Fall, wenn der Behälter 1 aus Fig. 8 um 90° gedreht werden würde, wenn man davon ausgeht, dass die z-Achse des angedeuteten Koordinatensystems der entgegengesetzten Richtung der Schwerkraft entspricht. Dann erstreckt sich die Höhe des Beutels 1 entlang der Breitenachse BA₂. Entsprechend ist es auch möglich, dass ein Wandungsvorsprung an der Decke 1" des Behälters 1 oder am Boden 1' des Behälters 1 angeordnet ist. Die Decke 1" des Behälters 1 und der Boden 1' des Behälters 1 definieren sich dabei durch ihre Position gegenüber der Schwerkraft. Das bedeutet, dass im Bezugssystem der Erde eine Behälterdecke 1" "oben" und ein Behälterboden 1' "unten" in einem Behälter 1 vorzufinden sind.

### Bezugszeichenliste

- 1: Behälter insbesondere Einweg-Behälter
- 1': Behälterboden
- 1": Behälterdecke
- 2: Antriebsvorrichtung
- 3: Rührelement
- 4: Behälterwandung
- 4': Wandungsabschnitt zur Verbindung zwischen Wandungsvorsprung-Element und Behälterwandung
- 5: Rührfortsatz
- 6: Antriebsseitige Lagerung
- 7: Gegenlagerung
- 8: Medium, insbesondere Biologisches Medium
- 9: Rührwelle
- 10: Axiale Drehstrommaschine
- 20: Wandungsvorsprung
- 20': Wandungsvorsprung-Element
- 20a: Wandungsausbuchtung
- 20b: Abschnitt zur Anbringung des Wandungsvorsprung-Elements an den Behälter
- 21: Sensor bzw. Sensorvorrichtung bzw. Optische Messvorrichtung
- 22: Behälter-Innenraum bzw. Behälter-Innenvolumen
- 23: Sensorbereich
- 23': Fenster
- 23b: Reflektives Element und/oder diffus reflektierende Fläche und/oder Spiegel
- 24: Lichtleiter bzw. Optische Faser
- 24a: Lichtleiter-Einkoppelabschnitt
- 24b: Lichtleiter-Auskoppelabschnitt
- 25: Spektrometer
- 26: Zugang
- 27: pH-Elektrode bzw. pH-Sensor
- 28: Vorsprungs-Wände
- 28': Verlängerung der Vorsprungs-Wände des Wandungsvorsprungs
- 29: Leitung
- 30: Sensor-Anbringungsvorrichtung
- 30': Aufnahme-Element für eine Sensor-Anbringungsvorrichtung
- 32: Anbringungsvorrichtungssteg
- 33: Aussparung
- 34: Leitblech bzw. Leitabschnitt
- 35: Kanalführung
- 36: Drehrichtung des Mediums
- 37: Flussrichtung des Mediums
- A: Außenseite
- α: Winkel zwischen Längsachse des Wandungsvorsprungs und der Längsachse des Behälters
- β: Winkel zwischen Längsachse des Wandungsvorsprungs und Normale N₂ der imaginären Konturlinie IK₂
- B₁: Breite des Wandungsvorsprungs
- B₂: Breite des Behälters
- γ: Winkel zwischen Breitenachse des Wandungsvorsprungs und Breitenachse des Behälters
- BA₁: Breitenachse des Wandungsvorsprungs
- BA₂: Breitenachse des Behälters
- D₁: Probenschichtdicke
- D₂: Tiefe der Wandungsausbuchtung
- D₃: Höhe der Wandungsausbuchtung
- D₄: Breite der Wandungsausbuchtung
- EV: Verbindung zwischen dem Abschnitt zur Anbringung des Wandungsvorsprung-Elements mit der Behälterwandung
- I: Behälter-Innenseite
- IK₁: Imaginäre Konturlinie zur Definition des Probenvolumens
- IK₂: Imaginäre Konturlinie als Verlängerung der Behälterwandung und Abgrenzung zur Wandungsausbuchtung
- K: Kanal
- KE: Kanaleingang
- KA: Kanalausgang
- L: Länge des Wandungsvorsprungs
- L₁: Länge der Verlängerung der Vorsprungs-Wände des Wandungsvorsprungs
- L₂: Länge des Behälters
- LA₁: Längsachse des Wandungsvorsprungs
- LA₂: Längsachse des Behälters
- LS: Linke Seite
- N₁: Normale der imaginären Konturlinie IK₁
- N₂: Normale der imaginären Konturlinie IK₂
- O: Obere Kante des Behälters
- R: Reflektive Strahlengang-Anordnung
- RS: Rechte Seite
- S: Spalt bzw. Spaltförmiges Volumen
- SG: Strahlengangachse
- T: Transmissive Strahlengang-Anordnung
- V: Probenvolumen
- AV: Volumen der Wandungsausbuchtung

**IN DEN FOLGENDEN PUNKTEN WERDEN WEITERE ASPEKTE, MERKMALE UND AUSFÜHRUNGSFORMEN DER VORLIEGENDEN OFFENBARUNG BESCHRIEBEN:**
1. Behälter (1) mit mindestens einem Wandungsvorsprung (20; 20a) für die Anbringung mindestens eines Sensors (21) von einer Außenseite (A) des Behälters (1) zur Erfassung mindestens einer Größe von einem in einem Behälter-Innenraum (22) enthaltenen Medium (8), wobei der Wandungsvorsprung (20) an einer Behälterwandung (4) angeordnet und dazu ausgelegt ist, den Behälter-Innenraum (22) und das Medium (8) zumindest teilweise zu umgeben und wobei der Wandungsvorsprung (20) mindestens einen Sensorbereich (23) umfasst, welcher dazu ausgelegt ist, dass die Größe durch den Sensorbereich (23) mittels des Sensors (21) erfasst werden kann.
2. Behälter nach Punkt 1, wobei sich der Wandungsvorsprung (20) entlang der Längsachse (LA₁) des Wandungsvorsprungs (20) erstreckt, wobei die Längsachse (LA₁) einen Winkel (b) mit einer Normalen (N₂) einer imaginären Konturlinie (IK₂) zur Definition des Probenvolumens (V) von -45° bis 45° einschließt und/oder eine Breitenachse (B₁) des Wandungsvorsprungs (20) mit einer Breitenachse (BA₂) des Behälters (1) einen Winkel (g) von -45° bis 45° einschließt.
3. Behälter (1) nach Punkt 1 oder 2, wobei der mindestens eine Sensorbereich (23) ein optisches Element, insbesondere ein Fenster (23') umfasst und dazu ausgelegt ist, dass die Größe mittels einer optischen Methode, insbesondere einer optischen Spektroskopie durch das optisches Element, insbesondere das Fenster erfasst werden kann.
4. Behälter (1) nach einem der vorangehenden Punkte, wobei der Wandungsvorsprung (20) zwei zueinander parallele und durch eine Probenschichtdicke (D₁) voneinander beabstandete Vorsprungs-Wände (28) mit einer Vorsprungs-Länge (L) umfasst derart, dass der Wandungsvorsprung (20) ein spaltförmiges Volumen (S) umgibt, wobei mindestens eine der Vorsprungs-Wände (28) bevorzugt jeweils den Sensorbereich (23) und insbesondere ein optisches Element, bevorzugt ein Fenster (23') umfasst.
5. Behälter (1) nach Punkt 3 oder 4, wobei der Wandungsvorsprung (20) eine diffus streuende Fläche umfasst und wobei der mindestens eine Sensorbereich (23) das optische Element, insbesondere das Fenster (23') umfasst und wobei der Wandungsvorsprung (20) dazu ausgelegt ist, dass die Größe mittels einer Sensorvorrichtung, welche vorzugsweise eine optische Faser umfasst, durch Transflexion oder doppelte Transmission durch Reflektion an der diffus streuenden Fläche erfasst werden kann.
6. Behälter (1) nach einem der Punkte 3 bis 5, wobei der Wandungsvorsprung (20) mindestens zwei Sensorbereiche (23) jeweils umfassend ein optisches Element, insbesondere ein Fenster (23') umfasst, welche dazu ausgelegt sind, dass die Größe mittels einer Sensorvorrichtung, welche vorzugsweise eine optische Faser umfasst, durch eine transmissive Strahlengang-Anordnung (T) erfasst werden kann.
7. Behälter (1) nach einem der vorangehenden Punkte, umfassend eine Sensor-Anbringungsvorrichtung (30) zur Anbringung des Sensors (21) relativ zu dem Wandungsvorsprung (20).
8. Behälter (1) nach Punkt 7, wobei die Sensor-Anbringungsvorrichtung (30) mindestens eine Aufnahmevorrichtung umfasst, welche dazu ausgelegt ist, ein weiteres optisches Element, vorzugsweise eine Linse, einen Spiegel, ein Prisma und/oder eine Lochblende aufzunehmen und/oder die Sensor-Anbringungsvorrichtung (30) mindestens ein weiteres optisches Element, insbesondere eine Lochblende umfasst.
9. Behälter (1) nach einem der vorangehenden Punkte, wobei der Behälter (1) dazu ausgelegt ist, einen Bestandteil eines Einweg-Bioreaktors darzustellen.
10. Behälter (1) nach einem der vorangehenden Punkte, wobei ein Wandungsvorsprung-Element (20'), welches den Wandungsvorsprung (20) und optional eine Wandungsausbuchtung (20a) umfasst mindestens einen Zugang (26) umfasst, welcher dazu ausgelegt ist, dass ein pH-Wert durch den Zugang (26) mittels einer pH-Elektrode erfasst werden kann.
11. Behälter (1) nach einem der vorangehenden Punkte, wobei der Wandungsvorsprung (20) Vorsprungs-Wände (28) umfasst, welche zumindest abschnittsweise jeweils eine Verlängerung (28') aufweisen, die in die Behälter-Innenseite (I) ragen oder wobei der Wandungsvorsprung (20) Vorsprungs-Wände (28) umfasst, welche in die Behälter-Innenseite (I) ragen.
12. Behälter (1) nach einem der vorangehenden Punkte, wobei der Wandungsvorsprung (20) einen Kanal (K) umfasst, welcher zumindest teilweise durch eine Kanalführung (35) und/oder ein Leitabschnitt (34) umgeben wird und der Kanal (K) dazu ausgelegt ist, ein sich bewegendes Medium von einem Kanaleingang (KE) zu einem Kanalausgang (KA) in einer Stromrichtung (37) zu führen.
13. Wandungsvorsprung-Element (20') zur Befestigung an einer Behälterwandung (4) eines Behälters (1), das Wandungsvorsprung-Element (20') umfassend einen Wandungsvorsprung (20), wobei der Wandungsvorsprung (20)
   - für die Anbringung mindestens eines Sensors (21) von der Außenseite (A) des Behälters zur Erfassung mindestens einer Größe von einem in einem Behälter-Innenraum (22) enthaltenen Medium (8) ausgelegt ist;
   - dazu ausgelegt ist, den Behälter-Innenraum (22) zumindest teilweise zu umgeben; und
   - mindestens einen Sensorbereich (23) umfasst, welcher dazu ausgelegt ist, dass die Größe durch den Sensorbereich (23) mittels des Sensors (21) erfasst werden kann.
14. Wandungsvorsprung-Element (20') nach Punkt 13, dazu ausgelegt, dass das Wandungsvorsprung-Element (20') an der Behälterwandung (4) reversibel anbringbar ist.
15. Wandungsvorsprung-Element (20') nach Punkt 14, dazu ausgelegt, dass das Wandungsvorsprung-Element (20') an der Behälterwandung (4) irreversibel anbringbar, vorzugsweise anklebbar und/oder anschweißbar ist.
16. Wandungsvorsprung-Element (20') nach einem der Punkte 13 bis 15, wobei sich der Wandungsvorsprung (20) entlang einer Längsachse (LA₁) erstreckt, wobei die Längsachse (LA₁) einen Winkel (b) mit einer Normalen (N₁) einer imaginären Konturlinie (IK₁) zur Definition des Probenvolumens (V) von -45° bis 45° einschließt.
17. Wandungsvorsprung-Element (20') nach einem der Punkte 13 bis 16, wobei das Wandungsvorsprung-Element (20') sterilisierbar ist.
18. Wandungsvorsprung-Element (20') nach einem der Punkte 13 bis 17, wobei ein Wandungsvorsprung (20) Vorsprungs-Wände (28) umfasst, welche zumindest abschnittsweise eine Verlängerung (28') aufweisen, die auf die Behälter-Innenseite (I) ragen oder wobei ein Wandungsvorsprung (20) Vorsprungs-Wände (28) umfasst, welche auf die Behälter-Innenseite (I) ragen.
19. Wandungsvorsprung-Element (20') nach einem der Punkte 13 bis 18, wobei der Wandungsvorsprung (20), einen Kanal (K) umfasst, welcher zumindest teilweise durch eine Kanalführung (35) und/oder ein Leitabschnitt (34) umgeben wird und der Kanal (K) dazu ausgelegt ist, ein sich bewegendes Medium von einem Kanaleingang (KE) zu einem Kanalausgang (KA) in einer Stromrichtung (37) zu führen.
20. Sensor-Anbringungsvorrichtung (30) zur Anbringung mindestens eines Sensors (21) relativ zu einem Sensorbereich (23) eines Wandungsvorsprungs (20) eines Behälters (1) von einer Außenseite (A) des Behälters (1), wobei die Sensor-Anbringungsvorrichtung (30)
   - eine Aufnahmevorrichtung umfasst zur Aufnahme des Sensors (21) zur Erfassung mindestens einer Größe von einem in einem Behälter-Innenraum (22) des Behälters (1) enthaltenen Medium (8); und
   - mittels einer Aussparung (33) relativ zu dem Wandungsvorsprung (20) angebracht werden kann, derart, dass zumindest ein Abschnitt des Wandungsvorsprungs (20) und zumindest ein Teil des Mediums (8) für die Erfassung der Größe durch den Sensorbereich (23) mittels des Sensors (21) innerhalb der Aussparung (33) positioniert ist.
21. Sensor-Anbringungsvorrichtung (30) nach Punkt 20, die dazu ausgelegt ist, zumindest einen optischen Sensor (21) und/oder einen Lichtleiter (24) relativ zu einem Sensorbereich (23) anzubringen, derart dass die Größe mittels einer optischen Methode, insbesondere einer optischen Spektroskopie erfasst werden kann.
22. Sensor-Anbringungsvorrichtung (30) nach Punkt 20 oder 21, wobei die Sensor-Anbringungsvorrichtung (30) mindestens eine Aufnahmevorrichtung umfasst, welche dazu ausgelegt ist, ein weiteres optisches Element, vorzugsweise eine Linse, einen Spiegel, ein Prisma und/oder eine Lochblende aufzunehmen und/oder die Sensor-Anbringungsvorrichtung (30) mindestens ein weiteres optisches Element, insbesondere eine Lochblende umfasst.
23. Sensor-Anbringungsvorrichtung (30) nach Punkt 22, welche eine diffus streuendes Fläche und/oder ein reflektives Element, insbesondere einen Spiegel umfasst und/oder dazu ausgelegt ist, eine diffus streuendes Fläche und/oder ein reflektives Element aufzunehmen und wobei die Sensor-Anbringungsvorrichtung (30) dazu ausgelegt ist, dass die mindestens eine Größe mittels einer Sensorvorrichtung durch eine reflektive Strahlengang-Anordnung (R) erfasst werden kann.
24. Sensor-Anbringungsvorrichtung (30) nach einem der Punkte 20 bis 23, die dazu ausgelegt ist, Lichtleiter (24) und/oder Sensoren (21) derart relativ zueinander anzuordnen, dass eine transmissive (T) und/oder eine reflektive (R) und/oder eine trans-flexive Strahlengang-Anordnung (R) besteht bzw. bestehen.
25. Verfahren zur Bereitstellung einer Erfassung mindestens einer Größe von einem in einem Behälter-Innenraum (22) eines Behälters (1) enthaltenen Medium (8), umfassend die Schritte:
   - Anordnen eines Wandungsvorsprungs (20) an einer Behälterwandung (4) des Behälters (1);
   - zumindest teilweises Umgeben des Behälter-Innenraums (22) und des Mediums (8) durch den Wandungsvorsprung (20);
   - Bereitstellen mindestens eines Sensorbereichs (23) an dem Wandungsvorsprung (20);
   - Anbringen von einer Außenseite (A) des Behälters (1) mindestens eines Sensors (21) relativ zu mindestens einem Wandungsvorsprung (20); und
   - Erfassen der Größe des Mediums (8) durch den Sensorbereich (23) mittels des Sensors (21).

## Patentansprüche

1. Wandungsvorsprung-Element (20') zur Befestigung an einer Behälterwandung (4) eines Behälters (1), das Wandungsvorsprung-Element (20') umfassend eine sich im befestigten Zustand zur Außenseite (A) des Behälters (1) erstreckende Wandungsausbuchtung (20a) und einen Wandungsvorsprung (20), wobei der Wandungsvorsprung (20)
- für die Anbringung mindestens eines Sensors (21) von der Außenseite (A) des Behälters zur Erfassung mindestens einer Größe von einem in einem Behälter-Innenraum (22) enthaltenen Medium (8) ausgelegt ist;
- dazu ausgelegt ist, den Behälter-Innenraum (22) zumindest teilweise zu umgeben;
- mindestens einen Sensorbereich (23) umfasst, welcher dazu ausgelegt ist, dass die Größe durch den Sensorbereich (23) mittels des Sensors (21) erfasst werden kann; und
- einen Kanal (K) bzw. ein kanalartiges Volumen (K) umfasst, welcher zumindest teilweise durch ein Leitabschnitt (34) und/oder eine Kanalführung (35) umgeben wird und der Kanal (K) dazu ausgelegt ist, ein sich bewegendes Medium von einem Kanaleingang (KE) zu einem Kanalausgang (KA) in einer Stromrichtung (37) zu führen.

2. Wandungsvorsprung-Element (20') nach Anspruch 1, wobei der Wandungsvorsprung (20) dazu ausgelegt ist, den Behälter-Innenraum (22) und das Medium (8) zumindest teilweise zu umgeben, und/oder
wobei der Leitabschnitt (34) und/oder die Kanalführung (35) dazu ausgebildet ist, ein Medium, insbesondere eine Flüssigkeit in dem Kanal (K) innerhalb eines Spaltes (S) bzw. eines spaltartigen Volumens (S) zu führen.

3. Wandungsvorsprung-Element (20') nach Anspruch 1 oder 2, wobei ein Fluss eines Mediums (8), vorzugsweise angetrieben durch ein Rührelement (3) im Wesentlichen mit dem Uhrzeigersinn durch den Behälter (1), einen Teil des Mediums (8) in den Kanaleingang (KE) durch den Leitabschnitt (34) und/oder die Kanalführung (35) in den Kanal (K) und in Richtung des Kanalausgangs (KA) leitet

4. Wandungsvorsprung-Element (20') nach Anspruch 1, wobei der Kanal (K) im Wesentlichen so verläuft, dass er das Medium (8) durch das Probenvolumen (V) in dem Spalt (S) und insbesondere durch einen Abschnitt zwischen zwei optischen Elementen (23'), insbesondere zwei Fenster (23'), des Wandungsvorsprungs (20) führt,
wobei, vorzugsweise, der Wandungsvorsprung (20) mindestens zwei Sensorbereiche (23) jeweils umfassend ein optisches Element (23'), insbesondere ein Fenster (23'), umfasst, welche dazu ausgelegt sind, dass die Größe mittels einer Sensorvorrichtung, welche vorzugsweise eine optische Faser umfasst, durch eine transmissive Strahlengang-Anordnung (T) erfasst werden kann.

5. Wandungsvorsprung-Element (20') nach einem der vorherigen Ansprüche, wobei der Leitabschnitt (34) als ein Leitblech ausgebildet ist

6. Wandungsvorsprung-Element (20') nach einem der vorherigen Ansprüche, wobei der Leitabschnitt (34) auf einer linken Seite (LS) des Spalts S angeordnet ist,
wobei der Leitabschnitt (34) im Wesentlichen einen Teil des Spalts (S) einschließt, und
wobei sich der Leitabschnitt (34) entlang einer Breitenachse (B₁) des Spalts (S) von der linken Seite (LS) in Richtung einer rechten Seite (RS) bis etwa zur Mitte des Spalts (S) erstreckt.

7. Wandungsvorsprung-Element (20') nach einem der vorherigen Ansprüche 1 bis 5, wobei sich der Leitabschnitt (34) nicht ganz bis zur Mitte des Spalts (S) entlang einer Breitenachse (B₁) des Spalts (S) von einer linken Seite (LS) des Spalts und/oder von einer rechten Seite (RS) des Spalts erstreckt.

8. Wandungsvorsprung-Element (20') nach einem der Ansprüche 1 bis 5, wobei sich der Kanal (K) im Wesentlichen über die gesamte Breite des Wandungsvorsprungs (20) bzw. des Spalts (S), entlang einer Breitenachse (B₁) des Spalts (S) erstreckt

9. Wandungsvorsprung-Element (20') nach Anspruch 8, wobei der Kanal (K) jeweils auf beiden Seiten entlang der Breitenachse (B₁) eine Öffnung aufweist, und wobei der Kanal (K) zwischen den Öffnungen im Wesentlichen durch die Kanalführung (35) eingeschlossen ist.

10. Wandungsvorsprung-Element (20') nach Anspruch 8 oder 9, wobei der Kanaleingang (KE) und der Kanalausgang (KA) auf beiden Seiten des Wandungsvorsprung-Elements (20') angeordnet sind.

11. Wandungsvorsprung-Element (20') nach einem der Ansprüche 8 bis 10, wobei die Kanalführung (35) im Wesentlichen einen Teil des Spaltes (S) bzw. des spaltartigen Volumens (S) einschließt.

12. Wandungsvorsprung-Element (20') nach Anspruch 11, wobei sich die Kanalführung (35) entlang der Breitenachse (B₁) des Spalts (S) von einer linken Seite (LS) auf eine rechte Seite (RS) des Spalts (S) erstreckt.

13. Wandungsvorsprung-Element (20') nach einem der vorherigen Ansprüche, wobei der Kanal (K) im Allgemeinen einen runden oder einen eckigen Querschnitt aufweist.

14. Wandungsvorsprung-Element (20') nach einem der vorherigen Ansprüche, wobei sich der Kanal (K) in eine Richtung aufweitet oder verengt, und/oder
wobei der Sensor (21) ein optischer Sensor, vorzugsweise ein Spektrometer, ist, und
wobei der Sensor (21) vorzugsweise ein Raman-Spektrometer und/oder eine CCD Kamera und/oder einen Photovervielfacher bzw. Photomultiplier umfasst.

15. Behälter (1) mit mindestens einem Wandungsvorsprung-Element (20') nach einem der vorherigen Ansprüche.
